# EUROPÄISCHE PATENTSCHRIFT

(11) **EP 0 686 152 B1**
(45) Veröffentlichungstag und Bekanntmachung des Hinweises auf die Patenterteilung: **02.05.1997**
(21) Anmeldenummer: 94907561.8
(22) Anmeldetag: 12.02.1994
(51) Int. Cl.: C07D 261/12, C07D 277/34, C07D 231/18, C07D 231/22, A01N 43/78, A01N 43/50, A01N 43/80, C07D 271/10, C07D 285/08, C07D 249/12, C07D 233/70

(54) **ORTHO-SUBSTITUIERTE 2-METHOXYIMINOPHENYLESSIGSÄUREMETHYLAMIDE**
ORTHO-SUBSTITUTED 2-METHOXYIMINOPHENYLACETIC ACID METHYLAMIDES
METHYLAMIDES DE L'ACIDE 2-METHOXYIMINOPHENYLACETIQUE ORTHO-SUBSTITUES

(30) Priorität: 23.02.1993 DE 4305502
(43) Veröffentlichungstag der Anmeldung: 13.12.1995
(60) Teilanmeldung: 96115410.1
(73) Patentinhaber: BASF Aktiengesellschaft, 67063 Ludwigshafen (DE)
(72) Erfinder: KIRSTGEN, Reinhard, D-67434 Neustadt (DE); GRAMMENOS, Wassilios, D-67063 Ludwigshafen (DE); BAYER, Herbert, D-68159 Mannheim (DE); DOETZER, Reinhard, D-69469 Weinheim (DE); KOENIG, Hartmann, D-67117 Limburgerhof (DE); OBERDORF, Klaus, D-69117 Heidelberg (DE); SAUTER, Hubert, D-68167 Mannheim (DE); WINGERT, Horst, D-68159 Mannheim (DE); LORENZ, Gisela, D-67434 Neustadt (DE); AMMERMANN, Eberhard, D-64646 Heppenheim (DE); HARRIES, Volker, D-67227 Frankenthal (DE)
(86) Internationale Anmeldenummer: EP9400408
(87) Internationale Veröffentlichungsnummer: WO9419331

(56) Entgegenhaltungen:
- EP-A- 0 363 818
- EP-A- 0 398 692
- EP-A- 0 477 631
- EP-A- 0 513 580

## Beschreibung

Die vorliegende Erfindung betrifft Verbindungen der allgemeinen Formel I, 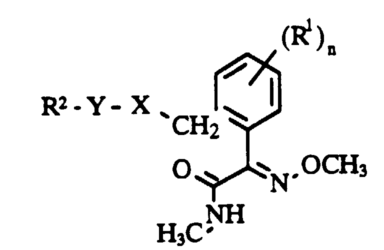 in der der Index und die Substituenten die folgende Bedeutung haben:
- n: 0, 1, 2, 3 oder 4, wobei die Reste R¹ verschieden sein können, wenn n größer als 1 ist;
- X: O oder S;
- Y: ein fünfringheteroaromatischer Ring, der neben R² noch ein oder zwei Reste aus der Gruppe Cl, CH₃, CF₃ oder OCH₃ tragen kann;
- R¹: Nitro; Cyano; Halogen;
C₁-C₄-Alkyl; C₁-C₄-Halogenalkyl; C₁-C₄-Alkoxy; C₁-C₄-Halogenalkoxy; C₁-C₄-Alkylthio;
Phenyl oder Phenoxy, wobei die aromatischen Ringe ein bis fünf Halogenatome oder ein bis drei der folgenden Reste tragen können: Halogen, C₁-C₄-Alkyl, C₁-C₄-Halogenalkyl, C₁-C₄-Alkoxy, C₁-C₄-Halogenalkoxy und C₁-C₄-Alkylthio;
oder, sofern n größer als 1 ist, eine an zwei benachbarte C-Atome des Phenylrestes gebundene 1,3-Butadien-1,4-diylgruppe, welche ihrerseits ein bis vier Halogenatome oder ein oder zwei der folgenden Reste tragen kann: Nitro, Cyano, Halogen, C₁-C₄-Alkyl, C₁-C₄-Halogenalkyl, C₁-C₄-Alkoxy, C₁-C₄-Halogenalkoxy und C₁-C₄-Alkylthio;
- R²: Wasserstoff;
ggf. subst. Alkyl, Alkenyl oder Alkinyl;
ein ggf. subst. gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff;
oder ein ggf. subst. ein- oder zweikerniger aromatischer Ring, welcher neben Kohlenstoffatomen ein bis vier Stickstoffatome oder ein oder zwei Stickstoffatome und ein Sauerstoff- oder Schwefelatom oder ein Sauerstoff-oder Schwefelatom als Ringglieder enthalten kann;

Weiter betrifft die Erfindung Verfahren zur Herstellung dieser Verbindungen, sie enthaltende Mittel und deren Verwendung zur Bekämpfung von Pilzen und Schadlingen.

Substituierte 2-Alkoxyiminophenylessigsäureamide sind bekannt (EP-A 398 692, EP-A 477 631, JP-A 4 182 461, WO 92/13 830 und GB 22 53 624). Die Verbindungen haben fungizide und z. T. auch insektizide Wirkung (EP-A 477 631). Ihre Wirkung ist jedoch unbefriedigend.

Überraschend wurde gefunden, daß die erfindungsgemäßen Verbindungen der allgemeinen Formel I eine stark verbesserte Wirksamkeit und Pflanzenvertraglichkeit besitzen.

Desweiteren wurden Verfahren zur Herstellung dieser Verbindungen, sie enthaltende Mittel und deren Verwendung zur Bekämpfung von Pilzen und Schädlingen gefunden.

Die Herstellung der Verbindungen der Formel I erfolgt in Analogie zu verschiedenen aus der Literatur an sich bekannten Methoden. Bei der Synthese der Verbindungen I ist die Reihenfolge, in der die Gruppierungen R²-Y-XCH₂- bzw. Gruppierung -C(=NOCH₃)-CO-NHCH₃ aus den entsprechenden Vorstufen aufgebaut werden, im allgemeinen unerheblich.

Besonders bevorzugt erhalt man diese Gruppierungen nach den nachstehend beschriebenen Verfahren, wobei zur besseren Übersichtlichkeit in den folgenden Reaktionsschemata die jeweils nicht an der Umsetzung beteiligte Gruppe vereinfacht dargestellt ist:
die Gruppierung R²-Y-XCH₂- bzw. deren Vorstufe als R* und
die Gruppierung -C(=NOCH₃)-CO-NHCH₃ bzw. deren Vorstufe als R^{#}.

### A: Verfahren zur Synthese der Gruppierung R²-Y-XCH₂-

Man erhält die Verbindungen der allgemeinen Formeln I bzw. II durch Umsetzung von IA bzw. IIA mit Hydroxy- bzw. Mercapto-substituterten fünfringheteroaromatischen Systemen III in inerten Lösungsmitteln in Gegenwart einer Base. 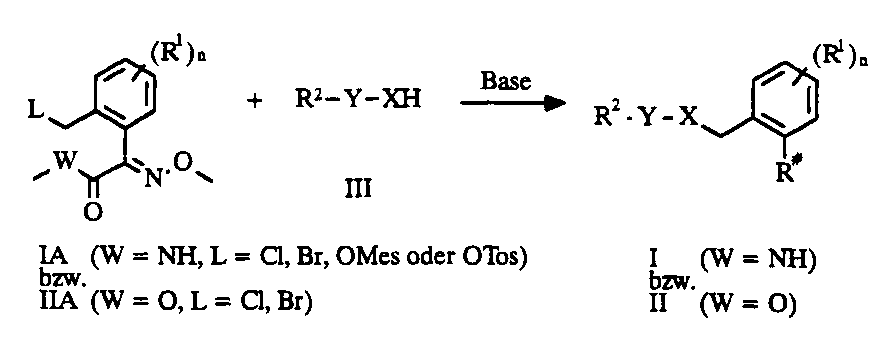
- Mes =: H₃CSO₂-
- Tos =: 4-CH₃-Phenyl-SO₂-

Diese Umsetzung wird Üblicherweise bei Temperaturen von 0°C bis 80°C, vorzugsweise 20°C bis 60°C durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, Ketone wie Aceton und Methylethylketon sowie Dimethylsulfoxid, Dimethylformamid, Dimethylacetatamid, 1,3-Dimethylimidazolidin-2-on und 1,3-Dimethyltetrahydro-2(1H)-pyrimidinin besonders, bevorzugt Methylenchlorid, Aceton und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Kaliumcarbonate und Calziumcarbonat sowie Alkalimetallhydrogencarbonate wie Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkymagnesiumhalogenide wie Methylmagnesiumchlorid sowie Alkalimetall- und Erdalkalimetallalkoholate wie Natriummethanolat, Natriummethanolat, Kaliumethanolat, Kalium-tert.-butanolat und Dimethoxymagnesium außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Natriumhydrid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers wie z.B. 18-Krone-6 oder 15-Krone-5 zuzusetzen.

Die Umsetzung kann auch in Zweiphasensystemen bestehen aus einer Lösung von Alkali- oder Erdalkalihydroxiden oder Alkali- oder Erdalkalicarbonaten in Wasser und einer organischen Phase wie z.B. halogenierten Kohlenwasserstoffen durchgeführt werden. Als Phasentransferkatalysatoren können Ammoniumhalogenide und -tetrafluoroborate wie z.B. Benzyltriethylamnoniumchlorid, Benzyltributylammoniumbromid, Tetrabutylammoniumchlorid, Hexadecyltrimethylammoniumbromid oder Tetrabutylammoniumtetrafluoroborat sowie Phosphoniumhalogenide wie Tetrabutylphosphoniumchlorid oder Tetraphenylphosphoniumbromid eingesetzt werden.

Es kann für die Umsetzung vorteilhaft sein, zunächst die Verbindungen III mit Base zu behandeln und das resultierende Salz mit den Verbindungen IA bzw. IIA umzusetzen.

Die Herstellung der Ausgangsverbindungen IA.1 (L = C1) und IA.2 (L = Br), vgl. EP477631, Tab. 1, Nr. 332 und 333, aus entsprechenden Alkoxy- bzw. Aryloxyverbindungen IB gelingt durch Spaltung 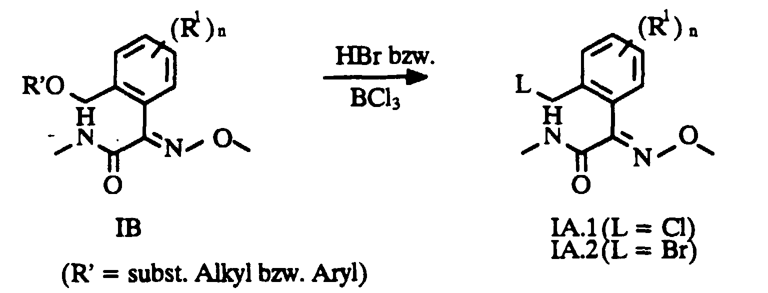 mit z. B. Bortrichlorid (für IA.1) bzw. mit Bromwasserstoff (für IA.2) in inerten Lösungsmitteln wie halogenierten Kohlenwasserstoffen bei Temperaturen von -30°C bis +40°C. Eine vorteilhafte Darstellung aus R' = 2-Tolyl (s. EP-A477631, Tab. 1, Nr. 94) ist in Beispielen 1 bis 3 beschrieben.

Die Darstellung der Ausgangsverbindungen IIA ist in der Literatur mehrfach beschrieben, vgl. EP-A 363 818.

Die Darstellung der Hydroxy- bzw. Mercapto-Fünfringheterocyclen III ist in der Literatur beschrieben, bzw. kann ananlog den dort gezeigten Synthesewegen durchgeführt werden, vgl. J. Heterocycl. Chem. 19 (1982), 541 ff; Acta Chem. Scand. 7 (1953), 374 ff; Chem. Pharm. Bull. 33 (1985), 3479 ff; Acta Chem. Scand. 17 (1963), 144 ff; Canadian Patent 1 177 081; Can. J. Chem. 57 (1979), 904 ff; Ann. Chem. 338 (1905), 273 ff; DOS1029827; Chem. Pharm. Bull. 15 (1967), 1025 ff; Agric. Biol. Chem. 50 (1986), 1831 ff; J. Org. Chem. 23 (1958), 1021 ff; Chem. Ber. 22 (1889), 2433 ff; Chem. Ber. 24 (1891), 369 ff; J. Med. Chem. 15 (1971), 39 ff; J. Am. Chem. Soc. 76 (1954), 4450 ff.

### B: Verfahren zur Synthese der Gruppierung -C(=NOCH₃)-CO-NHCH₃

Man erhält die Verbindungen der allgemeinen Formel I durch Aminolyse der entsprechenden 2-Methoxyimino-phenylessigsäureester II (vgl. Houben-Weyl Bd. E 5, S. 983 ff).

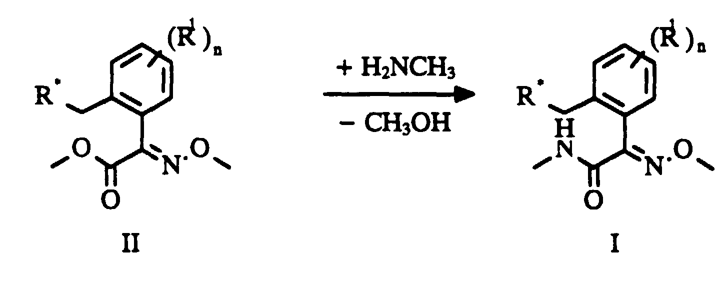

Diese Umsetzung wird üblicherweise bei Temperaturen von 0°C bis 60°C, vorzugsweise 10°C bis 30°C durchgeführt.

Methylamin kann durch gasförmiges Einleiten oder als wäßrige Lösung zu einer Lösung von II zudosiert werden.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol, besonders bevorzugt Methanol, Toluol und Tetrahydrofuran.

Es können auch Gemische der genannten Lösungsmittel verwendet werden.

Im Hinblick auf die biologische Wirkung gegen Schädlinge wie insbesondere Schadpilze, Insekten, Nematoden und Spinnentiere kommen insbesondere solche Verbindungen I in Betracht, in denen der Index und die Substituenten die folgende Bedeutung haben:
- n: 0, 1, 2, 3 oder 4, wobei die Reste R¹ verschieden sein können, wenn n größer als 1 ist, insbesondere 0 oder 1;
- X: O oder S;
- Y: ein fünfringheteroaromatisches System wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,2,4-Triazol-5-yl, 1,3,4-Oxadiazol-2-yl, 1,3,4-Thiadiazol-2-yl, 1,2,3-Oxadiazol-4-yl, 1,2,3-Oxadiazol-5-yl, 1,2,3-Triazol-5-yl und 1,2,3-Triazol-4-yl, insbesondere 2-Furyl, 2-Thienyl, 3-Pyrrolyl, 3-Isoxazolyl, 5-Isoxazolyl, 3-Isothiazolyl, 3-Pyrazolyl, 4-Pyrazolyl, 5-Pyrazolyl, 2-Oxazolyl, 4-Oxazolyl, 2-Thiazolyl, 4-Thiazolyl, 1,2,4-Oxadiazol-5-yl, 1,2,4-Thiadiazol-5-yl, 1,2,4-Triazol-3-yl, 1,3,4-Oxadiazol-2-yl und 1,3,4-Thiadiazol-2-yl.
- R¹: Nitro;
Cyano;
Halogen wie Fluor, Chlor, Brom und Jod, insbesondere Fluor und Chlor;
C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl und 1-Methylethyl, insbesondere Methyl; C₁-C₄-Halogenalkyl, besonders C₁-C₂-Halogenalkyl wie Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Difluormethyl und Trifluormethyl, insbesondere Trifluormethyl;
C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy und Ethoxy, 1-Methylethoxy, insbesondere Methoxy;
C₁-C₄-Halogenalkoxy, besonders C₁-C₂-Halogenalkoxy wie Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Difluormethyloxy und Chlordifluormethyloxy, insbesondere Difluormethoxyloxy;
C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Methylthio, Ethylthio und 1-Methylethylthio, insbesondere Methylthio;
Phenyl oder Phenoxy, wobei die aromatischen Ringe ein bis fünf Halogenatome wie Fluor, Chlor, Brom und Jod, insbesondere Fluor und Chlor oder ein bis drei der folgenden Reste tragen können:
   - Halogen wie vorstehend genannt;
   - C₁-C₄-Alkyl wie vorstehend genannt, insbesondere Methyl;
   - C₁-C₄-Halogenalkyl, besonders C₁-C₂-Halogenalkyl wie vorstehend genannt, insbesondere Trifluormethyl;
   - C₁-C₄-Alkoxy wie vorstehend genannt, insbesondere Methoxy;
   oder, sofern n > 1 ist, eine an zwei benachbarte C-Atome des Grundkörpers gebundene 1,3-Butadien-1,4-diylgruppe, welche ihrerseits ein bis vier Halogenatome wie Fluor, Chlor, Brom und Jod, insbesondere Fluor und Chlor, oder ein oder zwei der folgenden Reste tragen kann:
   - Halogen wie vorstehend genannt,
   - Nitro,
   - Cyano,
   - C₁-C₄-Alkyl wie vorstehend genannt, insbesondere Methyl;
   - C₁-C₄-Halogenalkyl, besonders C₁-C₂-Halogenalkyl wie vorstehend genannt, insbesondere Trifluormethyl;
   - C₁-C₄-Alkoxy wie vorstehend genannt, insbesondere Methyloxy;
- R²: ggf. subst. Alkyl, besonders C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, 1-Methylethyl, 1-Methylpropyl, 1,1-Dimethylethyl, 1,1-Dimethylpropyl und 2,3-Dimethylbutyl, insbesondere Methyl, 1-Methylethyl und 1,1-Dimethylethyl;
ggf. subst. Alkenyl, besonders C₂-C₆-Alkenyl wie Ethenyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl,
1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise 1-Propenyl, 1-Methyl-2-propenyl, 1, 1-Dimethyl-2-propenyl, 1,1-Dimethyl-2-butenyl, insbesondere 2-Propenyl und 1,1-Dimethyl-2-propenyl;
oder ggf. subst. Alkinyl, besonders C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 1-Methyl-2-propinyl, 1-Methyl-2-butinyl, 1,1-Dimethyl-2-propinyl und 1,1-Dimethyl-2-butinyl, insbesondere 2-Propinyl, 1-Methyl-2-propinyl und 1,1-Dimethyl-2-propinyl;
ein ggf. subst. gesättigter oder ein- oder zweifach ungesattigter Ring, welcher neben Kohlenstoffatomen ein bis drei der folgenden Heteroatome als Ringglieder enthalten kann: Sauerstoff, Schwefel und Stickstoff, beispielsweise Carbocyclen wie Cyclopropyl, Cyclopentyl, Cyclohexyl, Cyclopent-2-enyl, Cyclohex-2-enyl, 5- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, vorzugsweise 2-Tetrahydrofuranyl, 2-Tetrahydrothienyl, 2-Pyrrolidinyl, 3-Isoxazolidinyl, 3-Isothiazolidinyl, 1,3,4-Oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl;
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Aryireste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
sechsring Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Die bei den Resten genannten ein- oder zweikernigen aromatischen oder heteroaromatischen Systeme können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Diese ein- oder zweikernigen aromatischen oder heteroaromatischen Systeme können neben den bezeichneten Halogenatome zusätzlich ein bis drei der folgenden Substituenten tragen:
Nitro;
Cyano, Thiocyanato;
Alkyl, besonders C₁-C₆-Alkyl wie vorstehend genannt, vorzugsweise Methyl, Ethyl, 1-Methylethyl, 1,1-Dimethylethyl, Butyl, Hexyl, insbesondere Methyl und 1-Methylethyl;
Alkenyl, besonders bevorzugt C₂-C₆-Alkenyl wie vorstehend genannt, vorzugsweise Ethenyl, 1-Propenyl, 2-Propenyl, 1-Methyl-2-propenyl, 2-Butenyl, 3-Butenyl, 3-Hexenyl, insbesondere Ethenyl, 2-Propenyl und 2-Butenyl;
Alkinyl, besonders C₂-C₆-Alkinyl wie vorstehend genannt, vorzugsweise Ethinyl, 2-Propinyl, 1-Methyl-2-butinyl, 1,1-Dimethyl-2-butinyl insbesondere Ethinyl und 1,1-Dimethyl-2-butinyl;
C₁-C₄-Halogenalkyl, wie vorstehend genannt, vorzugsweise Trichlormethyl, Difluormethyl, Trifluormethyl, 2,2-Difluorethyl, 2,2,2-Trifluorethyl und Pentafluorethyl;
C₁-C₄-Alkoxy, vorzugsweise Methoxy, Ethoxy, 1-Methylethoxy und 1,1-Dimethylethoxy, insbesondere Methoxy;
C₁-C₄-Halogenalkoxy, besonders C₁-C₂-Halogenalkoxy, vorzugsweise Difluormethyloxy, Trifluormethyloxy und 2,2,2-Trifluorethyloxy, insbesondere Difluormethyloxy;
C₁-C₄-Alkylthio, vorzugsweise Methylthio und 1-Methylethylthio, insbesondere Methylthio;
C₁-C₄-Alkylamino wie Methylamino, Ethylamino, Propylamino, 1-Methylethylamino, Butylamino, 1-Methylpropylamino, 2-Methylpropylamino und 1,1-Dimethylethylamino, vorzugsweise Methylamino und 1,1-Dimethylethylamino, insbesondere Methylamino,
Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di- (1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl) amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl) amino, N-Ethyl-N-(2-methylpropyl) amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)-amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl) amino, N-(1-Methylpropyl)-N-(2-methylpropyl) amino, N-(1,1-Dimethylethyl)-N-(1-methylpropyl)amino und N-(1,1-Dimethylethyl)-N-(2-methylpropyl)amino, vorzugsweise N,N-Dimethylamino und N,N-Diethylamino, insbesondere N,N-Dimethylamino;
C₃-C₆-Alkenyloxy wie 2-Propenyloxy, 2-Butenyloxy, 3-Butenyloxy,1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 3,3-Dimethyl-2-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, vorzugsweise 2-Propenyloxy und 3-Methyl-2-butenyloxy, insbesondere 2-Propenyloxy;
C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy,2-Ethyl-3-butinyloxy und 1-Ethyl-1-methyl-2-propinyloxy, vorzugsweise 2-Propinyloxy und 2-Butinyloxy, insbesondere 2-Propinyloxy;
C₁-C₆-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentyl) carbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Methylcarbonyl, Ethylcarbonyl und 1,1-Dimethylcarbonyl, insbesondere Ethylcarbonyl;
C₁-C₆-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise Methoxycarbonyl, Ethoxycarbonyl und 1,1-Dimethylethoxycarbonyl, insbesondere Ethoxycarbonyl;
C₁-C₆-Alkylthiocarbonyl wie Methylthiocarbonyl, Ethylthiocarbonyl, Propylthiocarbonyl, 1-Methyl-ethylthiocarbonyl, Butylthiocarbonyl, 1-Methylpropylthiocarbonyl, 2-Methylpropylthiocarbonyl, 1,1-Dimethylethylthiocarbonyl, Pentylthiocarbonyl, 1-Methylbutylthiocarbonyl, 2-Methylbutylthiocarbonyl, 3-Methylbutylthiocarbonyl, 2,2-Dimethylpropylthiocarbonyl, 1-Ethylpropylthiocarbonyl, Hexylthiocarbonyl, 1,1-Dimethylpropthiocarbonyl, 1,2-Dimethylpropylthiocarbonyl, 1-Methylpentylthiocarbonyl, 2-Methylpentylthiocarbonyl, 3-Methylpentylthiocarbonyl, 4-Methylpentylthiocarbonyl, 1,1-Dimethylbutylthiocarbonyl, 1,2-Dimethylbutylthiocarbonyl, 1,3-Dimethylbutylthiocarbonyl, 2,2-Dimethylbutylthiocarbonyl, 2,3-Dimethylbutylthiocarbonyl, 3,3-Dimethylbutylthiocarbonyl, 1-Ethylbutylthiocarbonyl, 2-Ethylbutylthiocarbonyl, 1,1,2-Trimethylpropylthiocarbonyl, 1,2,2-Trimethylpropylthiocarbonyl, 1-Ethyl-1-methylpropylthiocarbonyl und 1-Ethyl-2-methylpropylthiocarbonyl, vorzugsweise Methylthiocarbonyl und 1-Methylethylthiocarbonyl, insbesondere Methylthiocarbonyl;
C₁-C₆-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl und 1-Ethyl-2-methylpropylaminocarbonyl, vorzugsweise Methylamincarbonyl und Ethylamincarbonyl, insbesondere Methylaminocarbonyl;
Di-C₁-C₆-alkylaminocarbonyl, besonders Di-C₁-C₄-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl) aminocarbonyl, N-Methyl-N-(2-methylpropyl) aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl) aminocarbonyl, N-Ethyl-N-(1,1-dimethylethyl) aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl) aminocarbonyl, N-(1-Methylethyl)-N-(1-methylpropyl)aminocarbonyl, N-(1-Methylethyl)-N-(2-methylpropyl) aminocarbonyl, N-(1,1-Dimethylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl) aminocarbonyl, N-(1-Methylpropyl)-N-(2-methyl-propyl) aminocarbonyl, N-(1,1-Dimethylethyl)-N-(1-methylpropyl) aminocarbonyl und N-(1,1-Dimethylethyl)-N-(2-methylpropyl) aminocarbonyl, vorzugsweise N,N-Dimethylaminocarbonyl und N,N-Diethylamincarbonyl, insbesondere N,N-Dimethylaminocarbonyl;
C₁-C₆-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl, 1-Methylbutylcarboxyl, 2-Methylbutylcarboxyl, 3-Methylbutylcarboxyl, 1,1-Dimethylpropylcarboxyl, 1,2-Dimethylpropylcarboxyl, 2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl, 1-Ethyl-1-1-methylpropylcarboxyl und 1-Ethyl-2-methylpropylcarboxyl, vorzugsweise Methylcarboxyl, Ethylcarboxyl und 1,1-Dimethylethylcarbonyl, insbesondere Methylcarboxyl und 1,1-Dimethylethylcarboxyl;
C₁-C₆-Alkylcarbonylamino wie Methylcarbonylamino, Ethylcarbonylamino, Propylcarbonylamino, 1-Methylethylcarbonylamino, Butylcarbonylamino, 1-Methylpropylcarbonylamino, 2-Methylpropylcarbonylamino, 1,1-Dimethylethylcarbonylamino, Pentylcarbonylamino, 1-Methylbutylcarbonylamino, 2-Methylbutylcarbonylamino, 3-Methylbutylcarbonylamino, 2,2-Dimethylpropylcarbonylamino, 1-Ethylpropylcarbonylamino, Hexylcarbonylamino, 1,1-Dimethylpropylcarbonylamino, 1,2-Dimethylpropylcarbonylamino, 1-Methylpentylcarbonylamino, 2-Methylpentylcarbonylamino,3-Methylpentylcarbonylamino, 4-Methylpentylcarbonylamino, 1,1-Dimethylbutylcarbonylamino, 1,2-Dimethylbutylcarbonylamino, 1,3-Dimethylbutylcarbonylamino, 2,2-Dimethylbutylcarbonylamino, 2,3-Dimethylbutylcarbonylamino, 3,3-Dimethylbutylcarbonylamino, 1-Ethylbutylcarbonylamino, 2-Ethylbutylcarbonylamino, 1,1,2-Trimethylpropylcarbonylamino, 1,2,2-Trimethylpropylcarbonylamino, 1-Ethyl-1-methylpropylcarbonylamino und 1-Ethyl-2-methylpropylcarbonylamino, vorzugsweise Methylcarbonylamino und Ethylcarbonylamino, insbesondere Ethylcarbonylamino;
C₃-C₇-Cycloalkyl wie Cyclopropyl, Cyclobutyl, Cyclopentyl, Cyclohexyl und Cycloheptyl, vorzugsweise Cyclopropyl, Cyclopentyl und Cyclohexyl, insbesondere Cyclopropyl;
C₃-C₇-Cycloalkoxy wie Cyclopropyloxy, Cyclobutyloxy, Cyclopentyloxy, Cyclohexyloxy und Cycloheptyloxy, vorzugsweise Cyclopentyloxy und Cyclohexyloxy, insbesondere Cyclohexyloxy;
C₃-C₇-Cycloalkylthio wie Cyclopropylthio, Cyclobutylthio, Cyclopentylthio, Cyclohexylthio und Cycloheptylthio, vorzugsweise Cyclohexylthio;
C₃-C₇-Cycloalkylamino wie Cyclopropylamino, Cyclobutylamino, Cyclopentylamino, Cyclohexylamino und Cycloheptylamino, vorzugsweise Cyclopropylamino und Cyclohexylamino, insbesondere Cyclopropylamino;
C₅-C₇-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl und Cyclohept-4-enyl, vorzugsweise Cyclopent-1-enyl, Cyclopent-3-enyl und Cyclohex-2-enyl, insbesondere Cyclopent-1-enyl;
5- bis 6-gliedrige, gesättigte oder ungesättigte Heterocyclen, enthaltend ein bis drei Stickstoffatome und/oder ein Sauerstoff-oder Schwefelatom wie vorstehend genannt, vorzugsweise Tetrahydropyrazin-1-yl und 2-Tetrahydrofuranyl, Tetrahydropyran-4-yl, 1,3-Dioxan-2-yl,
Phenyl,
5-Ring Heteroaromaten enthaltend ein bis drei Stickstoffatome und/oder ein Sauerstoff- oder Schwefelatom wie vorstehend genannt, vorzugsweise 3-Furyl, 3-Thienyl, 5-Isoxazolyl, 3-Isoxazolyl, 4-Oxazolyl, 1,3,4-Thiadiazol-3-yl und 2-Thienyl,
wobei an die vorstehend genannten 5-gliedrigen Heteroaromaten ein Benzolring anneliert sein kann.

6-Ring Heteroaromaten enthaltend ein bis drei Stickstoffatome als Heteroatome, vorzugsweise 5-Pyrimidyl und 3-Pyridinyl, wobei die vorstehend genannten Aryl- und Heteroarylringe ein bis drei der folgenden Gruppen tragen können: Fluor, Chlor, Cyano, Methyl, Methoxy, Trifluormethyl und Trifluormethoxy;

Zwei benachbarte Reste R² können die Bedeutung einer, gegebenenfalls mit Flur substituierte Oxy-C₁-C₂-alkylidenoxy-Kette, wie z.B. -O-CH₂-O, -O-CF₂-O-, -O-CH₂CH₂-O- oder -O-CF₂CF₂-O-, oder einer C₃-C₄-Alkylidenkette, wie z.B. Propyliden oder Butyliden, haben.

Die bei dem Rest R² genannten Alkyl-, Alkenyl- und Alkinylgruppen können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Die genannten Alkyl-, Alkenyl- und Alkinylgruppen können neben den bezeichneten Halogenatomen zusätzlich ein bis drei der folgenden Substituenten tragen:
Nitro;
Cyano; Thicyanato;
C₁-C₄-Alkoxy, vorzugsweise Methoxy, Ethoxy und 1-Methylethoxy, insbesondere Methoxy;
C₁-C₄-Halogenalkoxy, besonders C₁-C₂-Halogenalkoxy, insbesondere Difluormethyloxy,
C₁-C₄-Alkylthio, vorzugsweise Methylthio und 1,1-Dimethylethylthio, insbesondere Methylthio;
C₁-C₄-Alkylamino;
Di-C₁-C₄-alkylamino;
C₃-C₆-Alkenyloxy, insbesondere 2-Propenyloxy;
C₃-C₆-Alkinyloxy, insbesondere 2-Propinyloxy;
C₁-C₆-Alkylcarbonyl;
C₁-C₆-Alkoxyimino (Alkyl-O-N=) wie Methoxyimino, Ethoxyimino, Propyloxyimino, 1-Methyl-ethoxyimino, Butyloxyimino, 1-Methylpropyloxyimino, 2-Methylpropyloxyimino, 1,1-Dimethylethoxyimino, Pentyloxyimino, 1-Methylbutyloxyimino, 2-Methylbutyloxyimino, 3-Methylbutyloxyimino, 2,2-Dimethylpropyloxyimino, 1-Ethylpropyloxyimino, Hexyloxyimino, 1,1-Dimethylpropoxyimino, 1,2-Dimethylpropyloxyimino, 1-Methylpentyloxyimino, 2-Methylpentyloxyimino, 3-Methylpentyloxyimino, 4-Methylpentyloxyimino, 1,1-Dimethylbutyloxyimino, 1,2-Dimethylbutyloxyimino, 1,3-Dimethylbutyloxyimino, 2,2-Dimethylbutyloxyimino, 2,3-Dimethylbutyloxyimino, 3,3-Dimethylbutyloxyimino, 1-Ethylbutyloxyimino, 2-Ethylbutyloxyimino, 1,1,2-Trimethylpropyloxyimino, 1,2,2-Trimethylpropyloxyimino, 1-Ethyl-1-methylpropyloxyimino und 1-Ethyl-2-methylpropyloxyimino, vorzugsweise Methoxyimino, Ethoxyimino, Propyloximino, 1, 1-Dimethylethyloximino und 1-Methylethyloximino, insbesondere Methyloximino und Ethyloximino;
C₃-C₆-Alkenyloxyimino, d.h. C₃-C₆-Alkenyloxy wie vorstehend genannt, welches über -N= (Imino) an das Gerüst gebunden ist;
C₃-C₆-Alkinyloxyimino, d.h. C₃-C₆-Alkinyloxy wie vorstehend genannt, welches über -N= (Imino) an das Gerüst gebunden ist;
C₁-C₆-Alkoxycarbonyl, insbesondere Methoxycarbonyl, Ethoxycarbonyl und 1,1-Dimethylethoxycarbonyl, insbesondere Methoxycarbonyl und 1,1-Dimethylethoxycarbonyl;
C₁-C₆-Alkylthiocarbonyl, insbesondere Methylthiocarbonyl;
C₁-C₆-Alkylaminocarbonyl, insbesondere Methyliminocarbonyl;
Di-C₁-C₆-alkylaminocarbonyl, insbesondere N,N-Dimethylaminocarbonyl;
C₁-C₆-Alkylcarboxyl, vorzugsweise Methylcarboxyl und 1,1-Dimethylethylcarboxyl, insbesondere Methylcarboxyl;
C₁-C₆-Alkylcarbonylamino, vorzugsweise Methylcarbonylamino und 1,1-Dimethylethylcarbonylamino, insbesondere Methylcarbonylamino;
C₃-C₇-Cycloalkyl, vorzugsweise Cyclopropyl, Cyclopentyl und Cyclohexyl, insbesondere Cyclopropyl;
C₃-C₇-Cycloalkoxy, insbesondere Cyclohexyloxy;
C₃-C₇-Cycloalkylthio, insbesondere Cyclohexylthio;
C₃-C₇-Cycloalkylamino;
C₅-C₇-Cycloalkenyl, vorzugsweise Cyclopent-1-enyl, Cyclopent-2-enyl und Cyclohex-2-enyl, insbesondere Cyclopent-1-entyl;
5- bis 6-gliedrige, gesättigte oder ungesättigte Heterocyclen, enthaltend ein bis drei Stickstoffatome und/oder ein Sauerstoff-oder Schwefelatom, wie vorstehend genannt, vorzugsweise Tetrahydropyran-4-yl, 2-Tetrahydrofuranyl und 1,3-Dioxan-2-yl;
aromatische Systeme wie Phenyl, 1-Naphthyl und 2-Naphthyl;

5-Ring Heteroaromaten enthaltend ein bis drei Stickstoffatome und/oder ein Sauerstoff- oder Schwefelatom, wie vorstehend genannt, vorzugsweise 2-Furyl, 3-Furyl, 2-Thienyl, 3-Thienyl, 5-Isoxazolyl und 4-Oxazolyl, insbesondere 2-Furyl und 2-Thienyl, wobei an die vorstehend genannten 5-gliedrigen Heteroaromaten ein Benzoring anneliert sein kann.

6-Ring Heteroaromaten enthaltend ein bis drei Stickstoffatome als Heteroatome wie vorzugsweise 2-Pyrimidinyl, 5-Pyrimidinyl und 3-Pyridyl, wobei an die vorstehend genannten 6-gliedrigen Heteroaromaten ein Benzoring anneliert sein kann.

Die bei dem Rest R² genannten gesättigten oder ein- oder zweifach ungesättigten alicyclischen oder heterocyclischen Systeme, können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Diese ein- oder zweifach ungesättigten alicyclischen oder heterocyclischen Systeme können neben den bezeichneten Halogenatomen zusätzlich ein bis drei der folgenden Substituenten tragen:
Nitro;
Cyano;
C₁-C₆-Alkyl, vorzugsweise Methyl und Ethyl, insbesondere Methyl;
C₁-C₄-Halogenalkyl, besonders C₁-C₂-Halogenalkyl, insbesondere Trifluormethyl;
C₁-C₄-Alkoxy, insbesondere Methoxy;
C₁-C₄-Alkylthio;
Di-C₁-C₄-alkylamino;
C₂-C₆-Alkenyl, vorzugsweise Ethenyl, 1-Propenyl, 2-Propenyl und 1-Methylethinyl, insbesondere Ethenyl und 1-Methylethenyl,
C₂-C₆-Alkinyl, vorzugsweise Ethinyl, 2-Propinyl, 1-Butinyl, insbesondere Ethinyl;
C₁-C₆-Alkoxycarbonyl, vorzugsweise Methoxycarbonyl, Ethoxycarbonyl, 1-Methylethoxycarbonyl und 1,1-Dimethylethoxycarbonyl, insbesondere Ethoxycarbonyl;
C₁-C₆-Alkylaminocarbonyl;
Di-C₁-C₆-alkylaminocarbonyl;
C₁-C₆-Alkylcarboxyl, insbesondere Methylcarboxyl;
C₁-C₆-Alkylcarbonylamino, insbesondere Methylcarbonylamino und 1,1-Dimethylcarbonylamino;
C₃-C₇-Cycloalkyl, vorzugsweise Cyclopropyl und Cyclohexyl, insbesondere Cyclopropyl;
aromatische Systeme wie insbesondere Phenyl;

Daneben sind Verbindungen I bevorzugt, in denen R¹ für Halogen, C₁-C₄-Alkyl, C₁-C₂-Halogenalkyl, C₁-C₄-Alkoxy, C₁-C₂-Halogenalkoxy oder Phenyl steht.

Zusätzlich sind solche Verbindungen I bevorzugt, in denen X für Sauerstoff steht.

Von besonderem Interesse sind solche Verbindungen I, in denen Y für 3-Isoxazolyl, 1-Pyrazolyl, 3-Pyrazolyl, 4-Pyrazolyl, 2-Oxazolyl, 2-Thiazolyl, 4-Thiazolyl, 1,2,4-Triazol-3-yl, 1,3,4-Oxadiazol-2-yl, 1,2,4-Oxadiazol-5-yl und 1,2,4-Thiadiazol-5-yl, insbesondere 3-Pyrazolyl, 4-Thiazolyl und 1,2,4-Triazol-3-yl steht.

Insbesondere sind solche Verbindungen I bevorzugt, in denen R² für ggf. substituiertes Phenyl steht. Als Substituneten des Phenylrestes kommen bevorzugt Halogen, Cyano, C₁-C₄-Alkyl, C₁-C₄-Alkoxy, C₁-C₂-Halogenalkyl, C₁-C₂-Halogenalkoxy, Phenyl und Oxy-C₁-C₂-alkylidenoxy in Betracht.

Ebenfalls bevorzugt sind solche Verbindungen I, in denen R² für ggf. substituierten Fünfringheteroaromaten wie z.B. Thiazolyl, Isoxazolyl, Oxazolyl oder 1,2,4-Oxadiazolyl, steht. Als Substituenten des Fünfringheteroaromaten kommen bevorzugt Halogen, Cyano, C₁-C₆-Alkyl, C₁-C₆-Alkoxy, C₁-C₄-Halogenalkyl, C₁-C₂-Halogenalkoxy und Phenyl in Betracht.

Ebenfalls bevorzugt sind solche Verbindungen I, in denen R² für gegebenenfalls substituierte Sechsringheteroaromaten, wie z.B. Pyridyl, Pyrimidyl, Pyridazinyl oder Pyrazinyl steht. Als Substituenten des Sechsringheteroaromaten kommen bevorzugt Halogen, Cyano, C₁-C₄-Alkyl, C₁-C₄-Alkoxy, C₁-C₂-Halogenalkyl, C₁-C₂-Halogenalkoxy und Phenyl in Betracht.

Ebenfalls bevorzugt sind solche Verbindungen I, in denen R² für C₁-C₆-Alkoxyimino-, C₃-C₆-Alkenyloxyimino- oder C₃-C₆-Alkinyloxyiminosubstitutiertes Alkyl, wie z.B. Methoxyimino-eth-1-yl, Ethoxyimino-eth-1-yl, Propoxyimino-eth-1-yl, i-Propoxyimino-eth-1-yl, (2-Propen)oxyimino-eth-1-yl, (2-Buten)oxyimino-eth-1-yl, (2-Propin)oxyimino-eth-1-yl, Methoxyimino-prop-1-yl, Ethoxyiminoprop-1-yl, Propoxyimino-prop-1-yl, (2-Propen)oxyimino-prop-1-yl oder (2-Propin)oxyimino-prop-1-yl steht.

Außerdem werden Verbindungen der allgemeinen Formel I bevorzugt, in denen
- Y: für ein fünfringheteroaromatisches System, das neben R² noch ein oder zwei Reste aus der Gruppe Cl, CH₃, CF₃ oder OCH₃ tragen kann, steht;
- R¹: für Halogen, C₁-C₄-Alkyl, C₁-C₂-Halogenalkyl, C₁-C₂-Alkoxy, C₁-C₂-Halogenalkoxy, C₁-C₂-Alkylthio oder Phenyl steht;
- n: für 0 oder 1 steht;
- X: für O oder S steht;
- R²: für ggf. substituiertes C₁-C₄-Alkyl oder ggf. substituiertes C₃-C₆-Cycloalkyl steht.

Daneben werden Verbindungen der allgemeinen Formel I bevorzugt, in denen R²-Y- für eines der folgenden fünfringheteroaromatischen Systeme steht

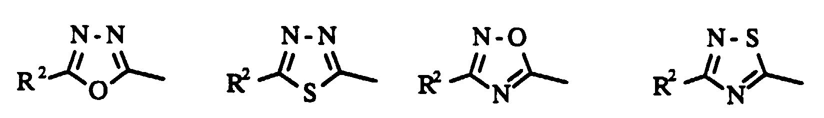

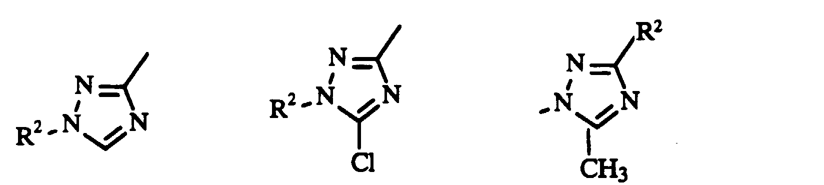

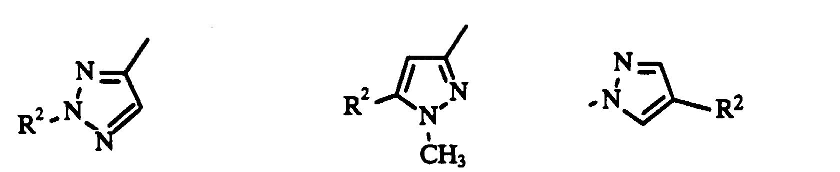

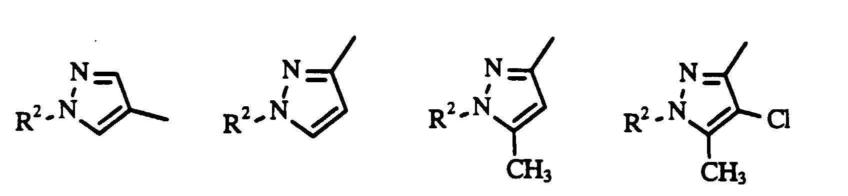

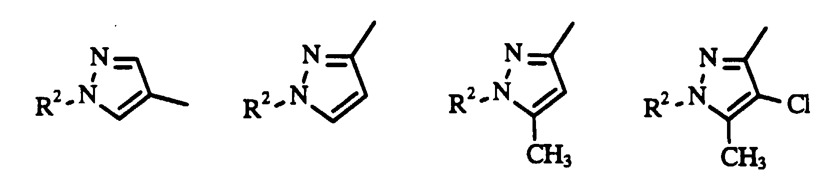

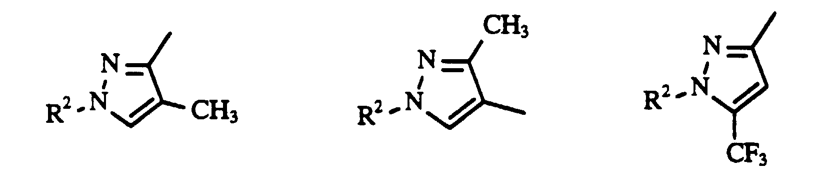

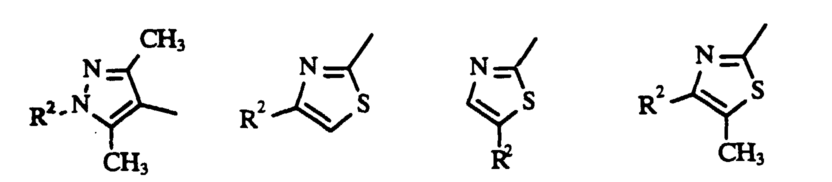

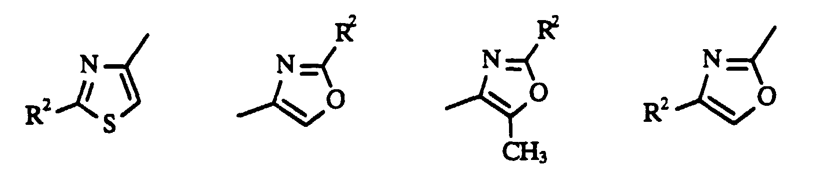

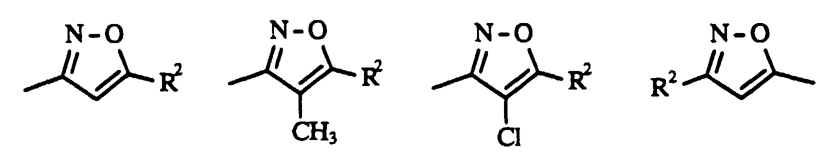

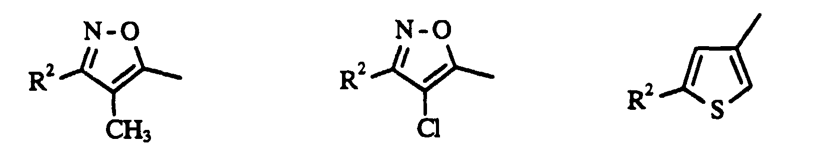

Des weiteren werden Verbindungen der allgemeinen Formel I bevorzugt, in denen
- n: für 0 oder 1 steht;
- X: für O oder S steht;
- Y: für ein fünfringheteroaromatisches System, das neben R² noch ein oder zwei Reste aus der Gruppe Cl, CH₃, CF₃ oder OCH₃ tragen kann, steht;
- R¹: für Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl steht;
- R²: für ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff-oder Schwefelatom als Ringglieder enthalten kann, steht.

Außerdem werden Verbindungen der allgemeinen Formel I bevorzugt, in denen
- n: für 0 steht;
- X: für O steht;
- Y: für ein fünfringheteroaromatisches System, das neben R² noch ein oder zwei Reste aus der Gruppe Cl, CH₃, CF₃ oder OCH₃ tragen kann, steht;
- R²: für ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff-oder Schwefelatom als Ringglieder enthalten kann, steht.

Daneben werden Verbindungen der allgemeinen Formel I bevorzugt, in denen
- n: für 0 oder 1 steht;
- x: für O oder S steht;
- Y: für ein fünfringheteroaromatisches System, das neben R² noch ein oder zwei Reste aus der Gruppe Cl, CH₃, CF₃ oder OCH₃ tragen kann, steht;
- R¹: für Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl steht;
- R²: für ggf. substituiertes Phenyl steht.

Des weiteren werden Verbindungen der allgemeinen Formel I bevorzugt, in denen
- n: für 0 steht;
- X: für O steht;
- Y: für ein fünfringheteroaromatisches System, das neben R² noch ein oder zwei Reste aus der Gruppe Cl, CH₃, CF₃ oder OCH₃ tragen kann, steht;
- R²: für ggf. substituiertes Phenyl steht.

Weiter werden Verbindungen der allgemeinen Formel I bevorzugt, in denen
- n: für 0 oder 1 steht;
- X: für O oder S steht;
- Y: für ein fünfringheteroaromatisches System, das neben R² noch ein oder zwei Reste aus der Gruppe Cl, CH₃, CF₃ oder OCH₃ tragen kann, steht;
- R¹: für Halogen, C₁-C₄-Alkyl, C₁-C₂-Halogenalkyl, C₁-C₄-Alkoxy, C₁-C₂-Halogenalkoxy, C₁-C₂-Alkylthio oder Phenyl steht;
- R²: für ggf. substituierten Fünfringheteroaromaten steht.

Außerdem werden Verbindungen der allgemeinen Formel I bevorzugt, in denen
- n: für 0 oder 1 steht;
- X: für O oder S steht;
- Y: für ein sechsringheteroaromatisches System, das neben R² noch ein oder zwei Reste aus der Gruppe Cl, CH₃, CF₃ oder OCH₃ tragen kann, steht;
- R²: für ggf. substituierten Sechsringheteroaromaten steht.

Insbesondere bevorzugte Verbindungen der Formel I.1, I.2, I.3, I.4, I.5, I.6, I.7, I.8, I.9, I.10, I.11 und I.12 sind in den nachstehenden Tabellen A.1, A.2, A.3, A.4, A.5, A.6, A.7, A.8, A.9, A.10, A.11 und A.12 zusammengefaßt.

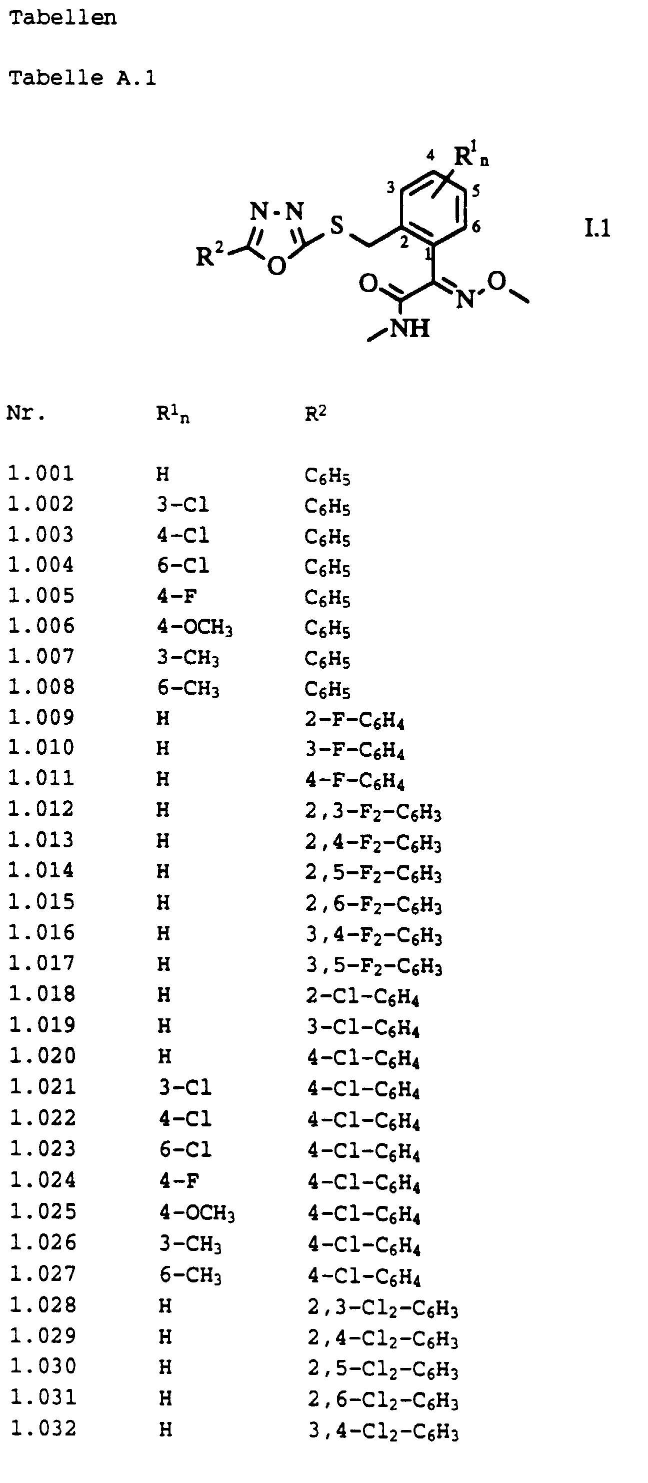

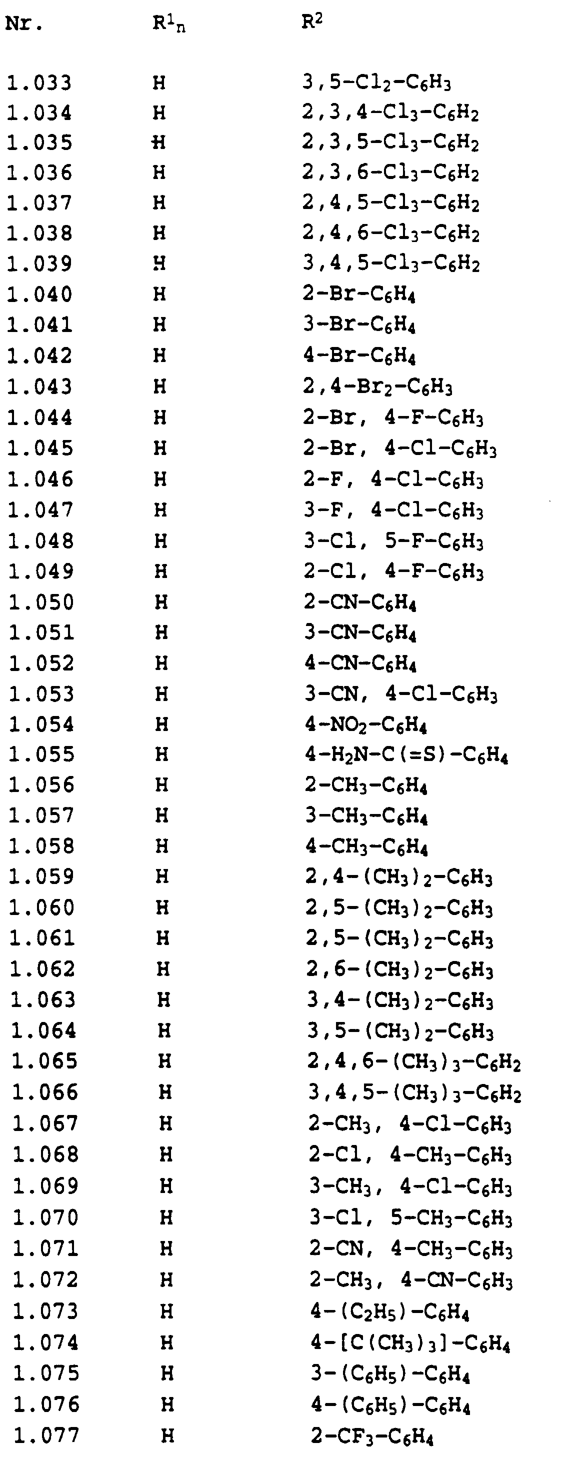

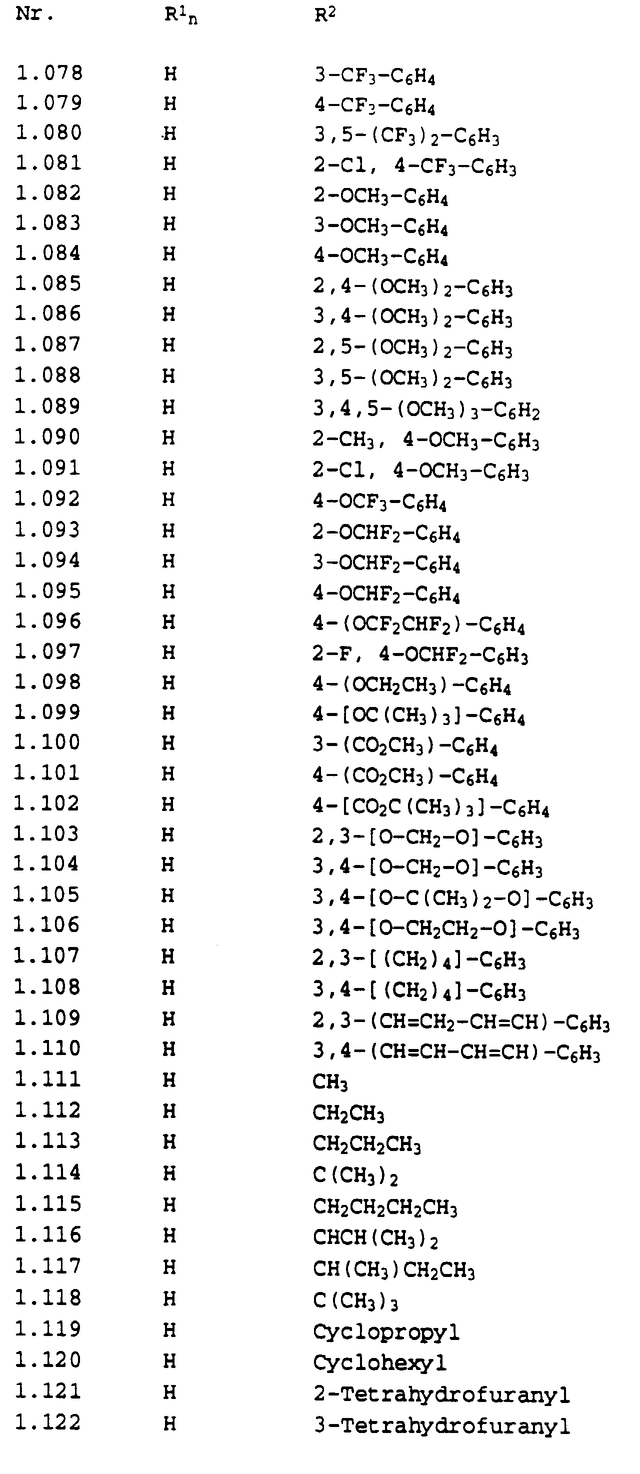

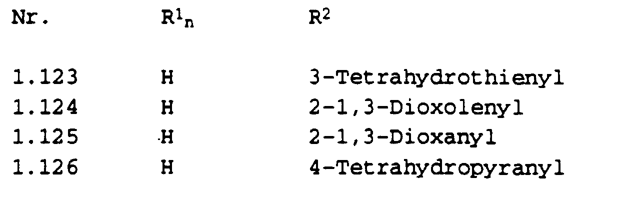

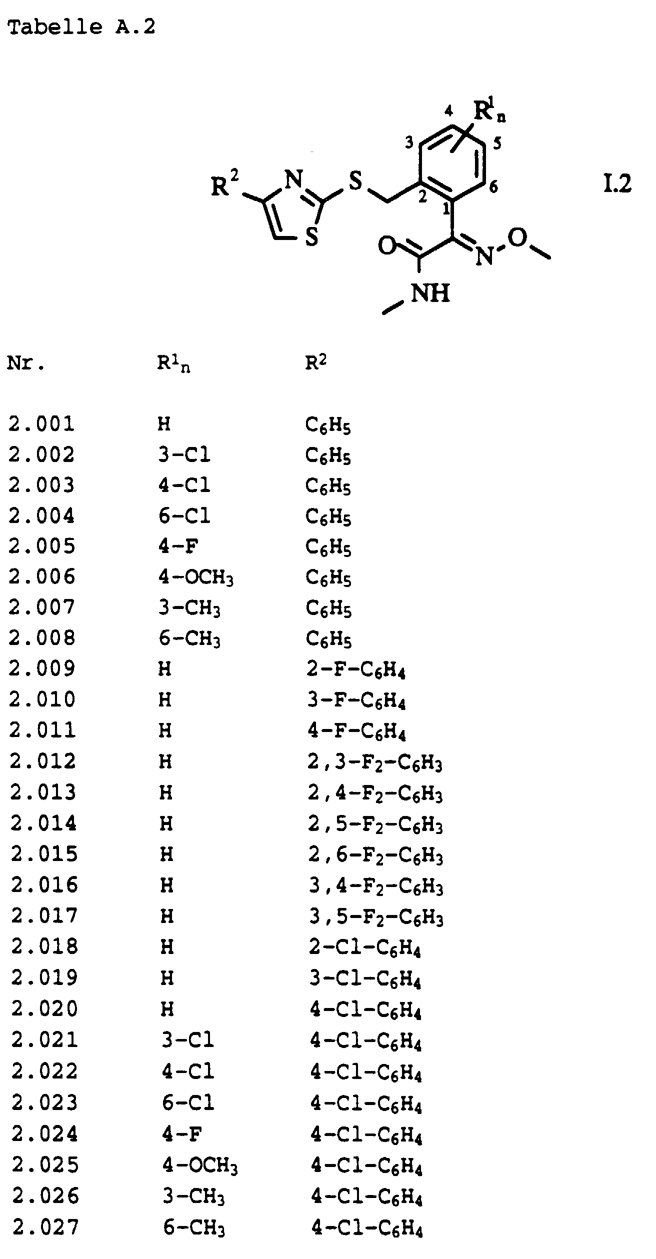

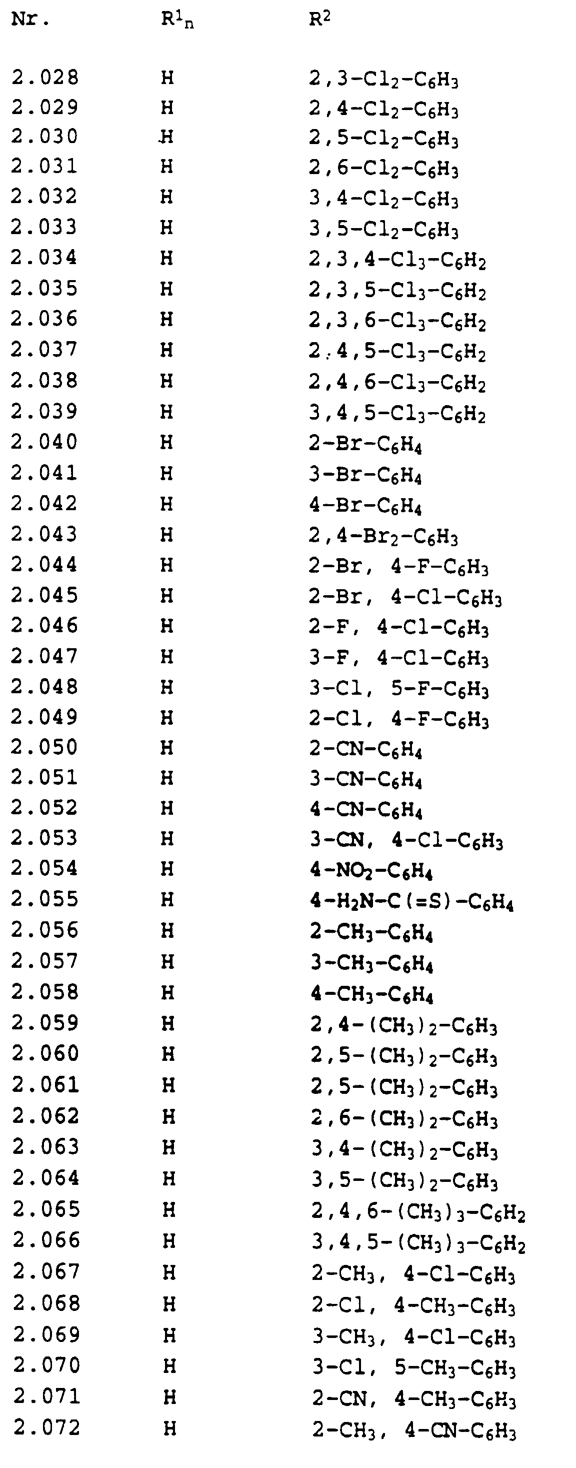

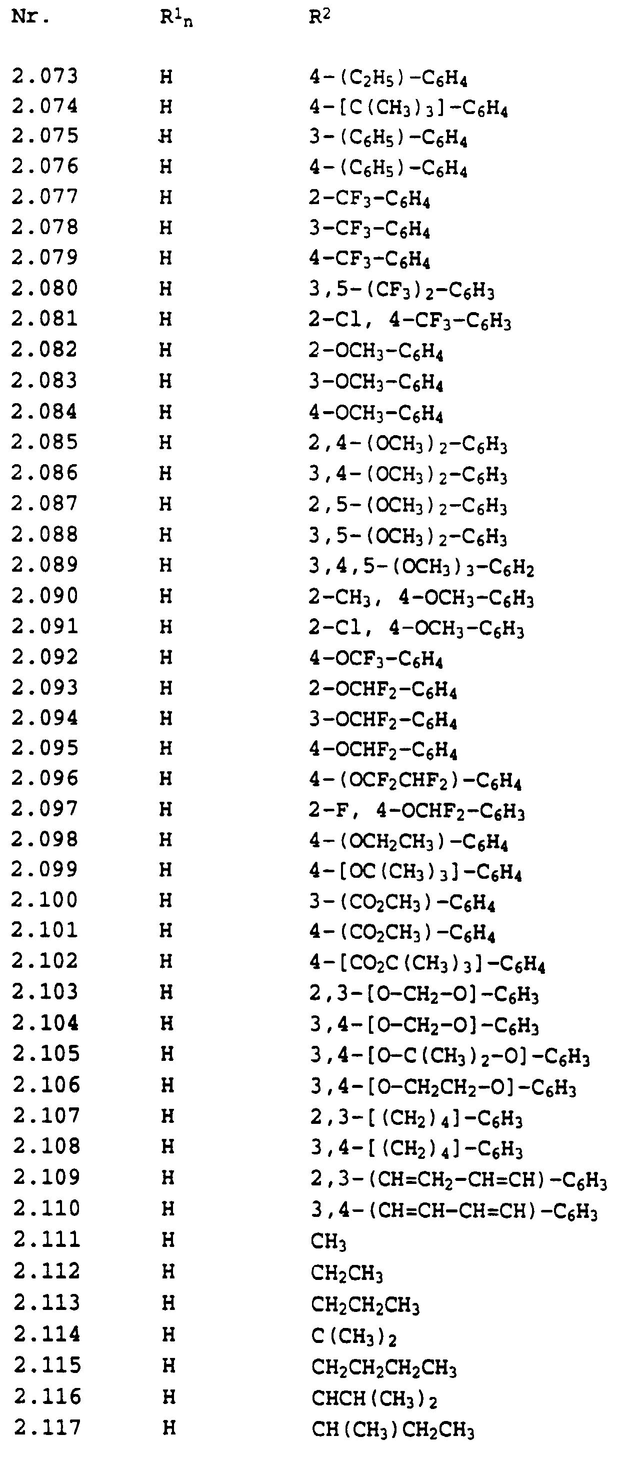

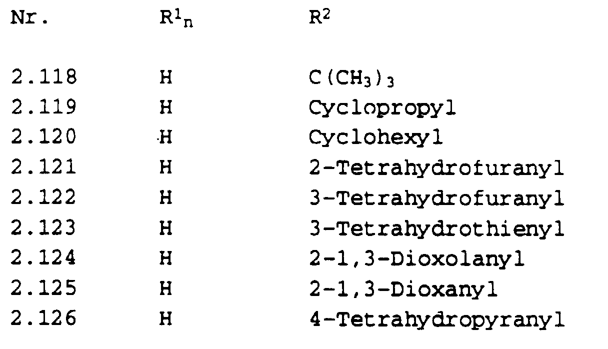

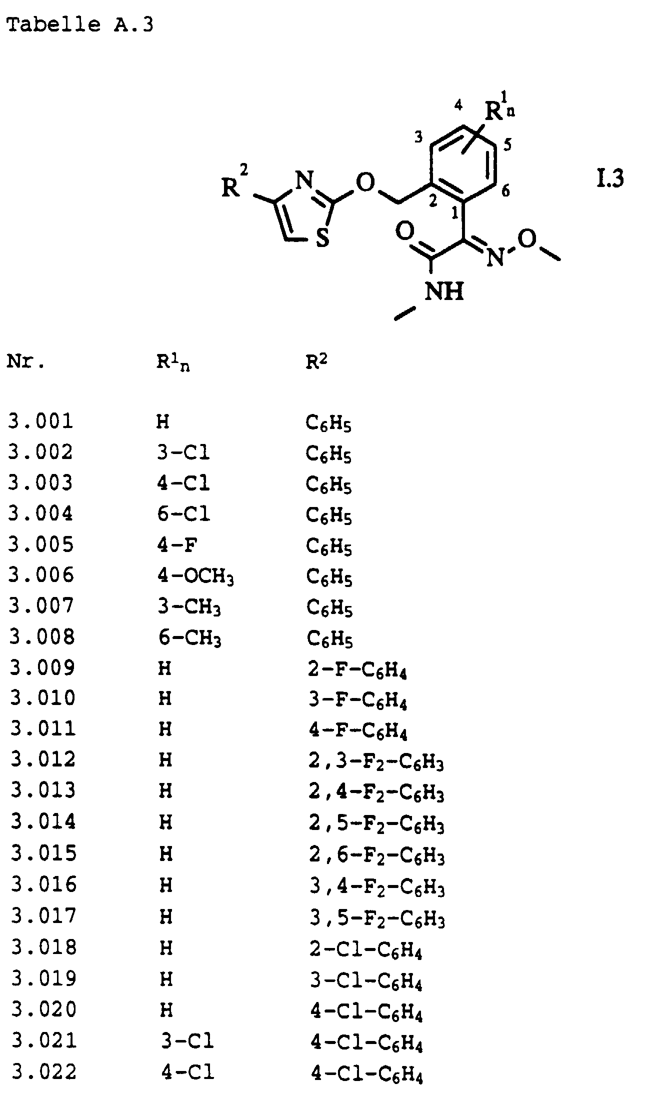

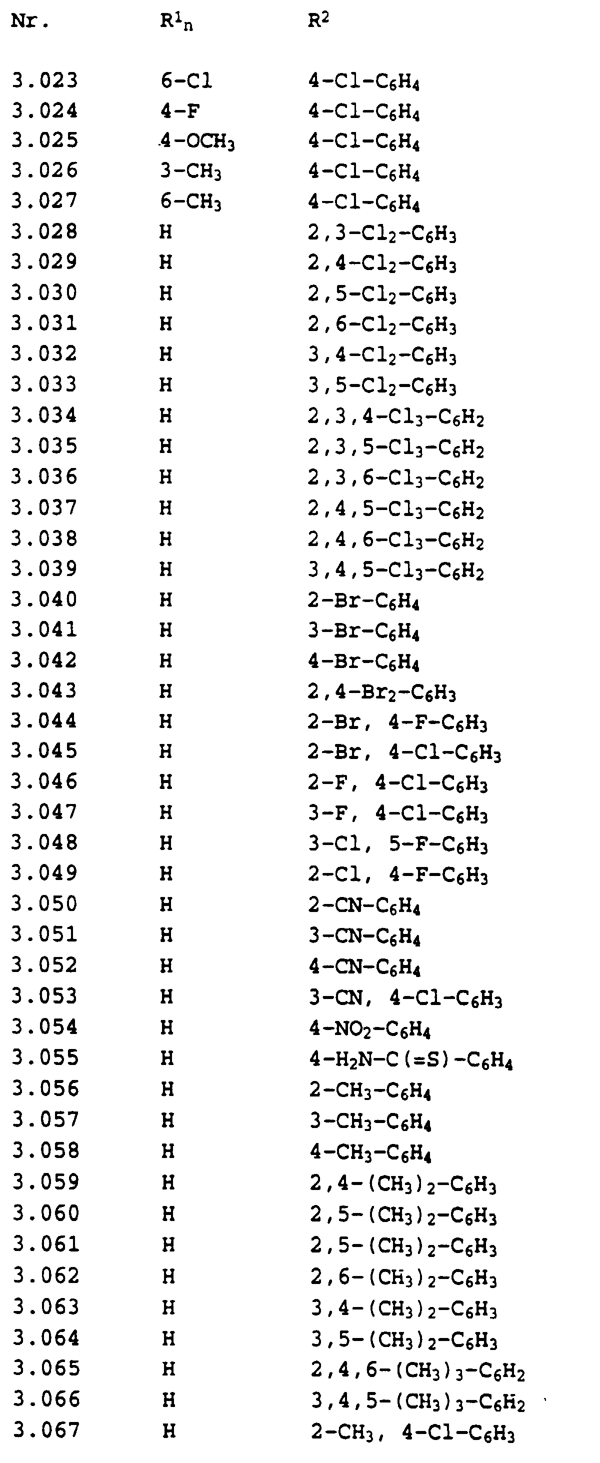

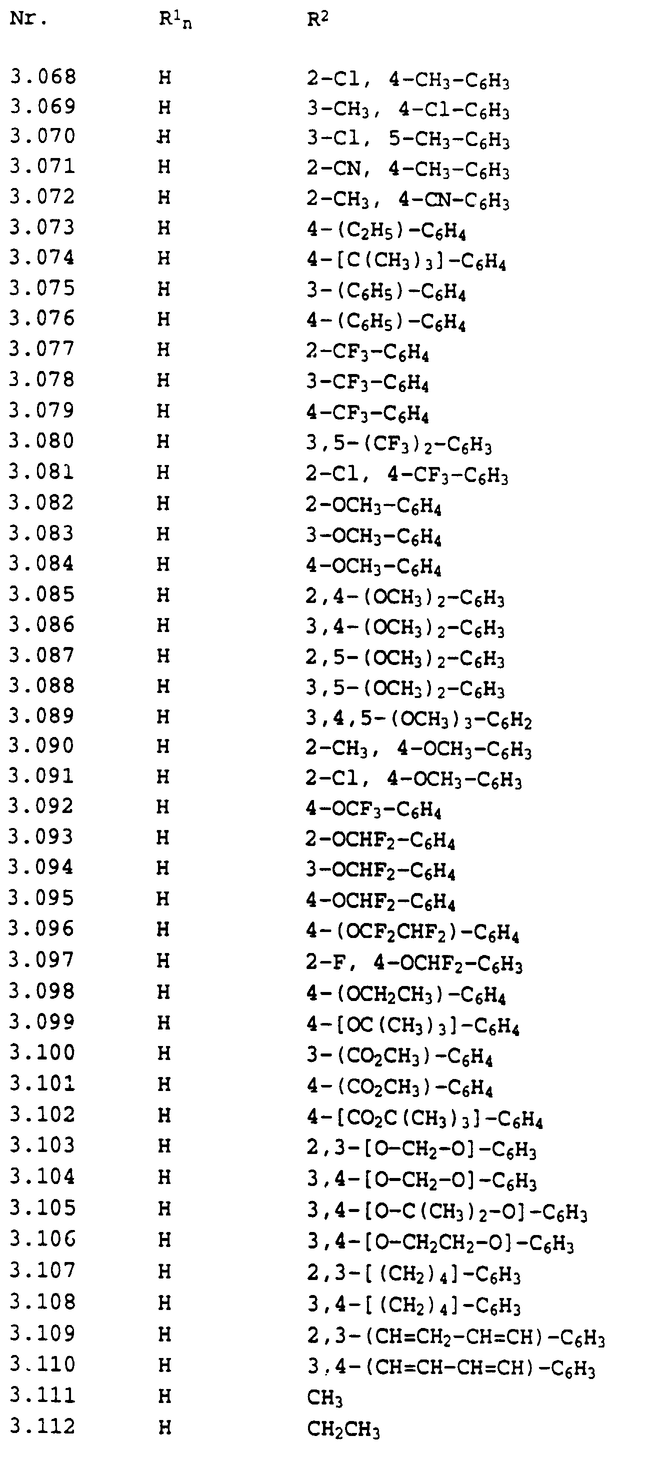

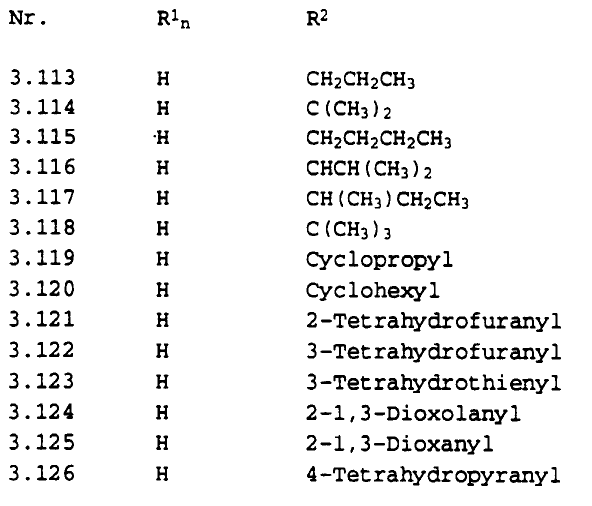

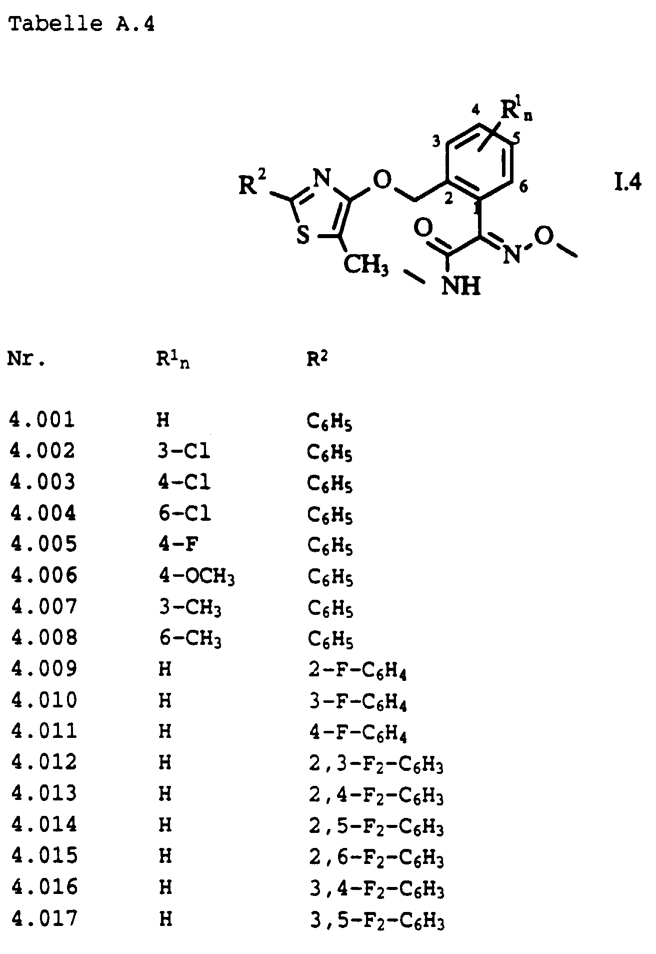

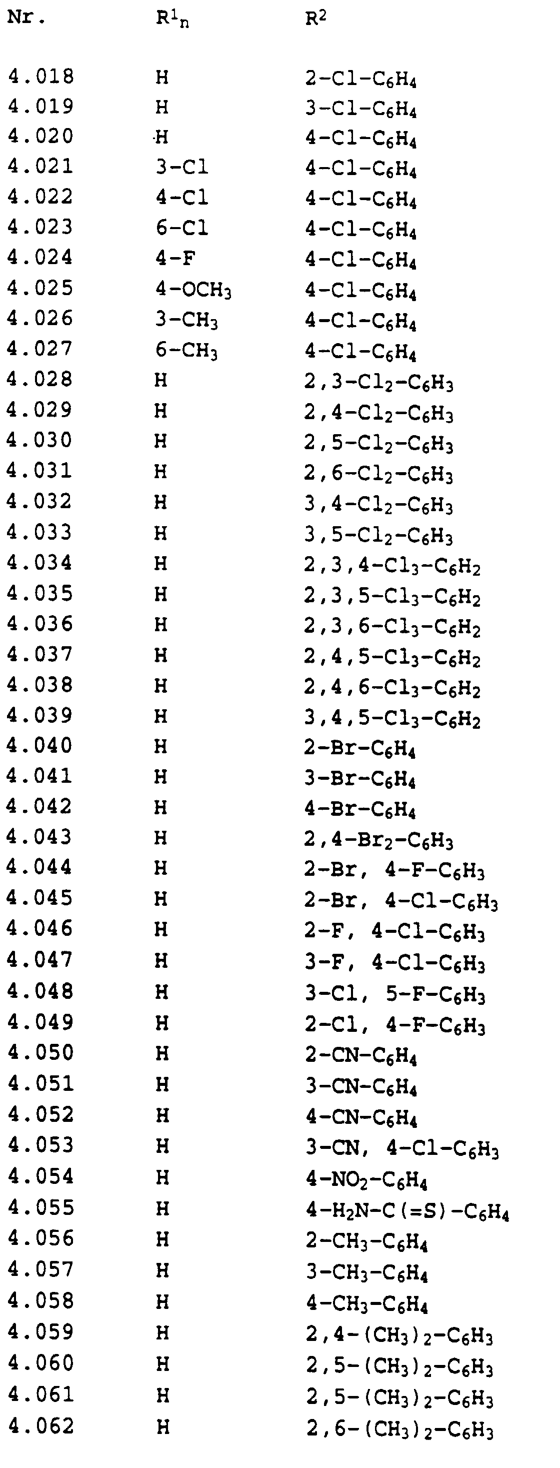

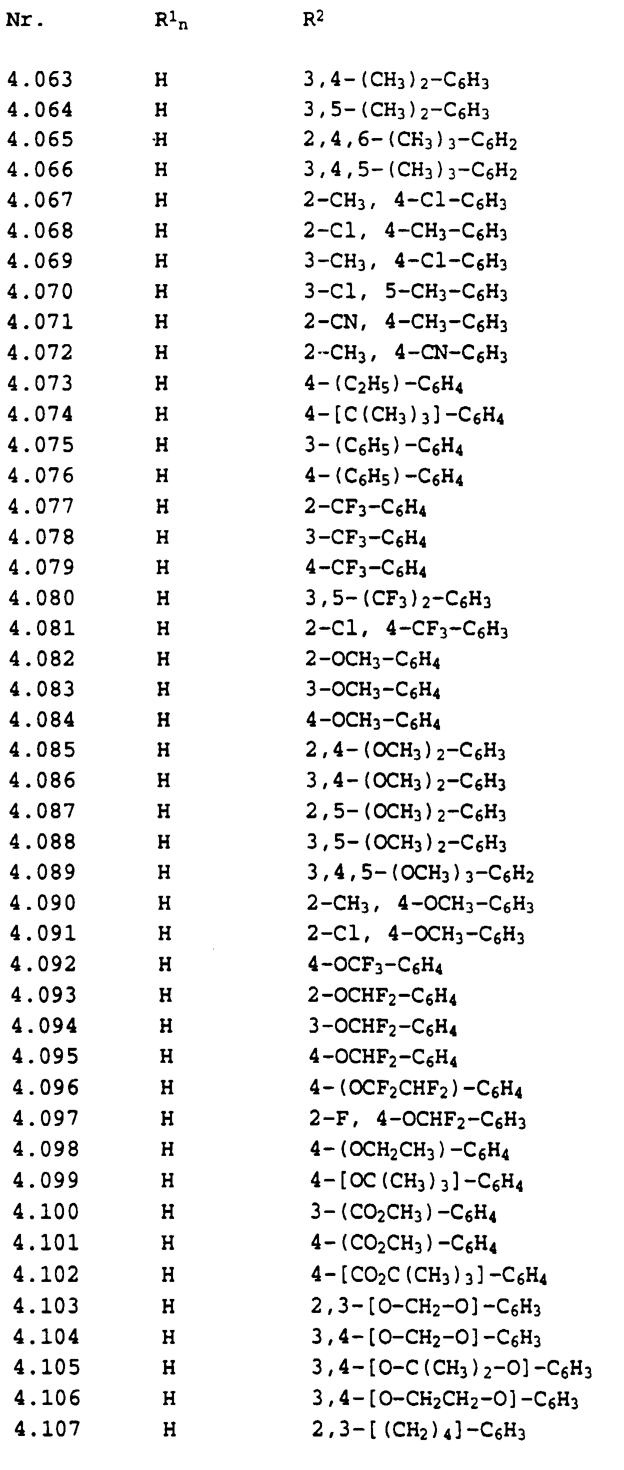

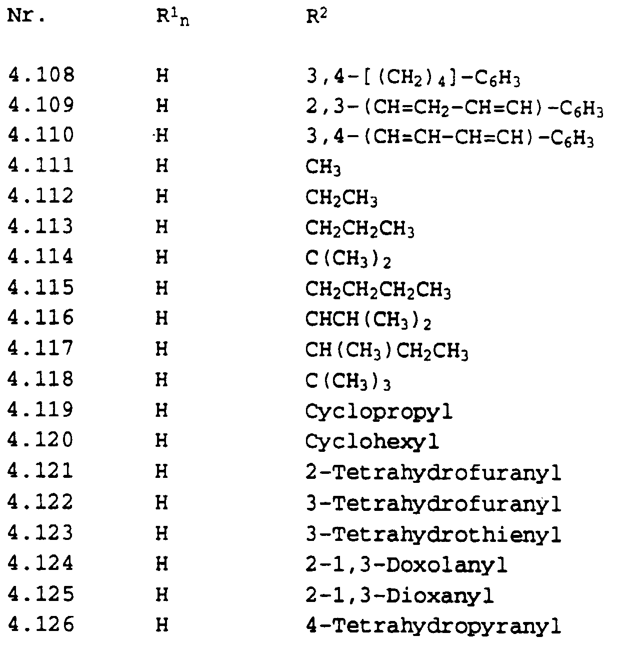

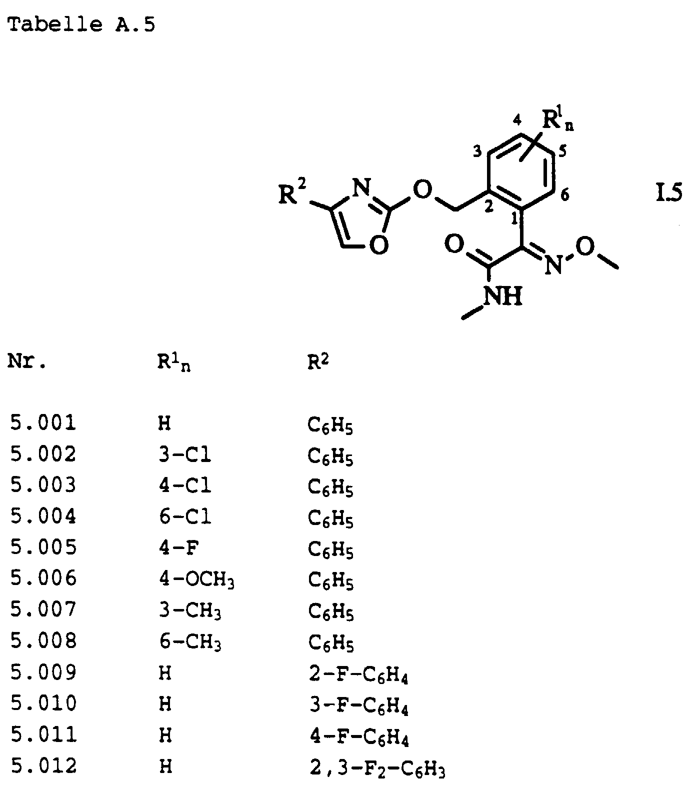

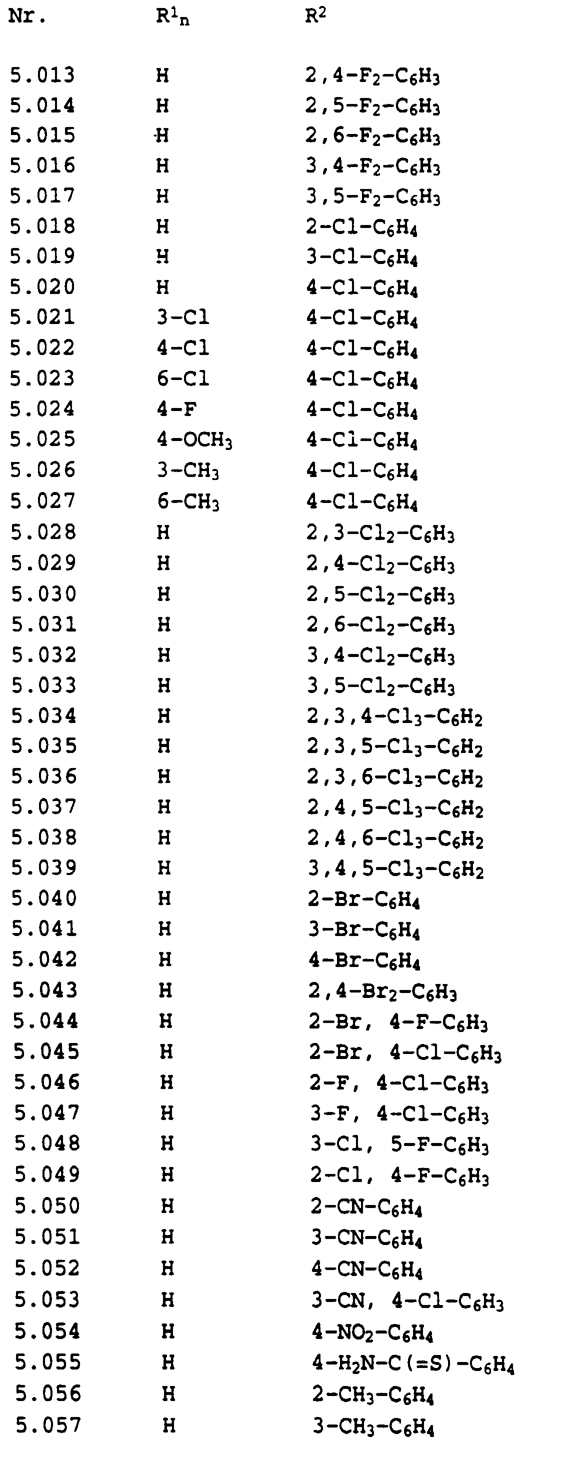

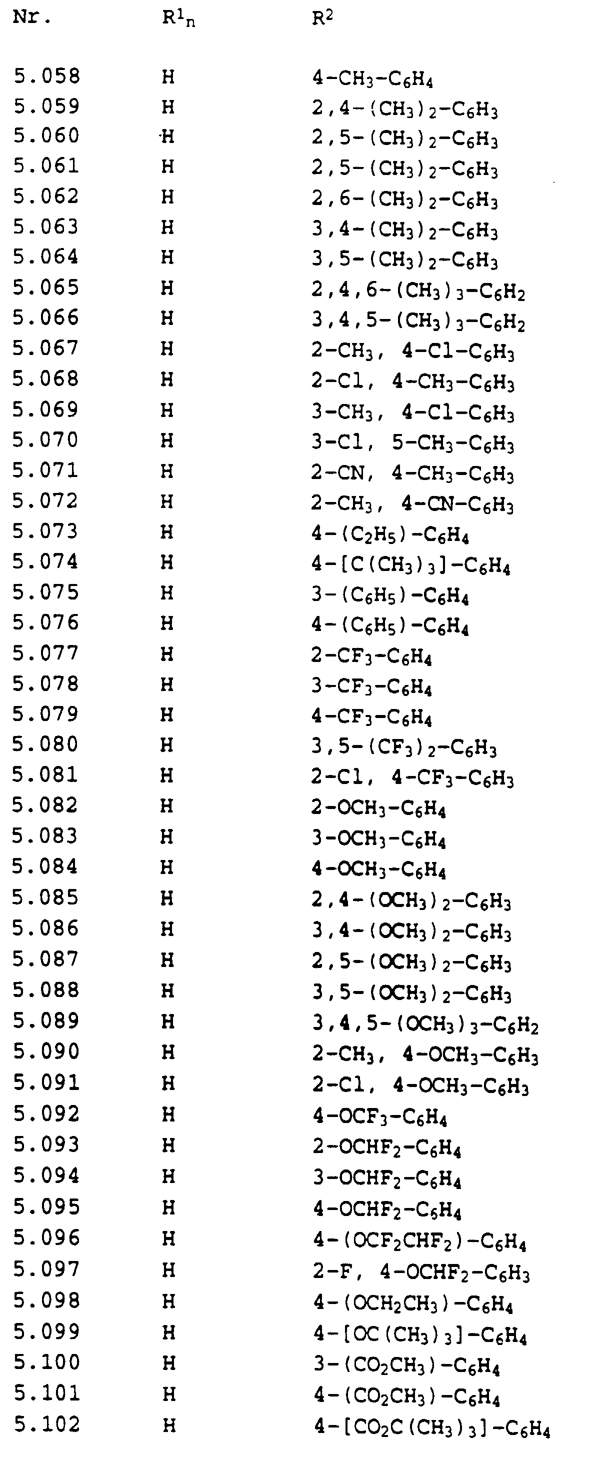

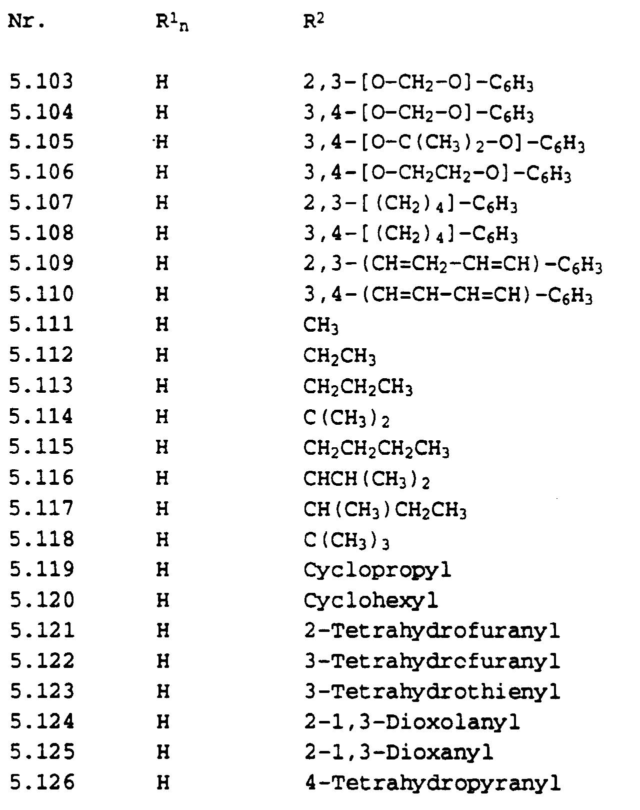

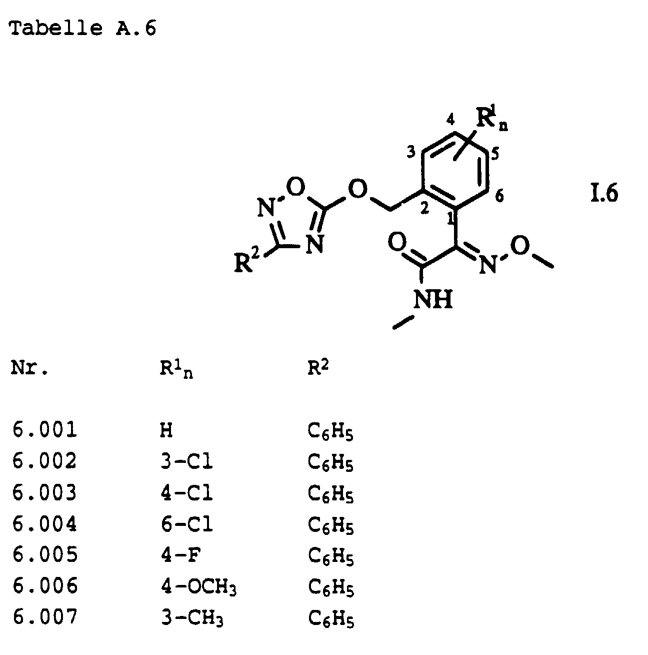

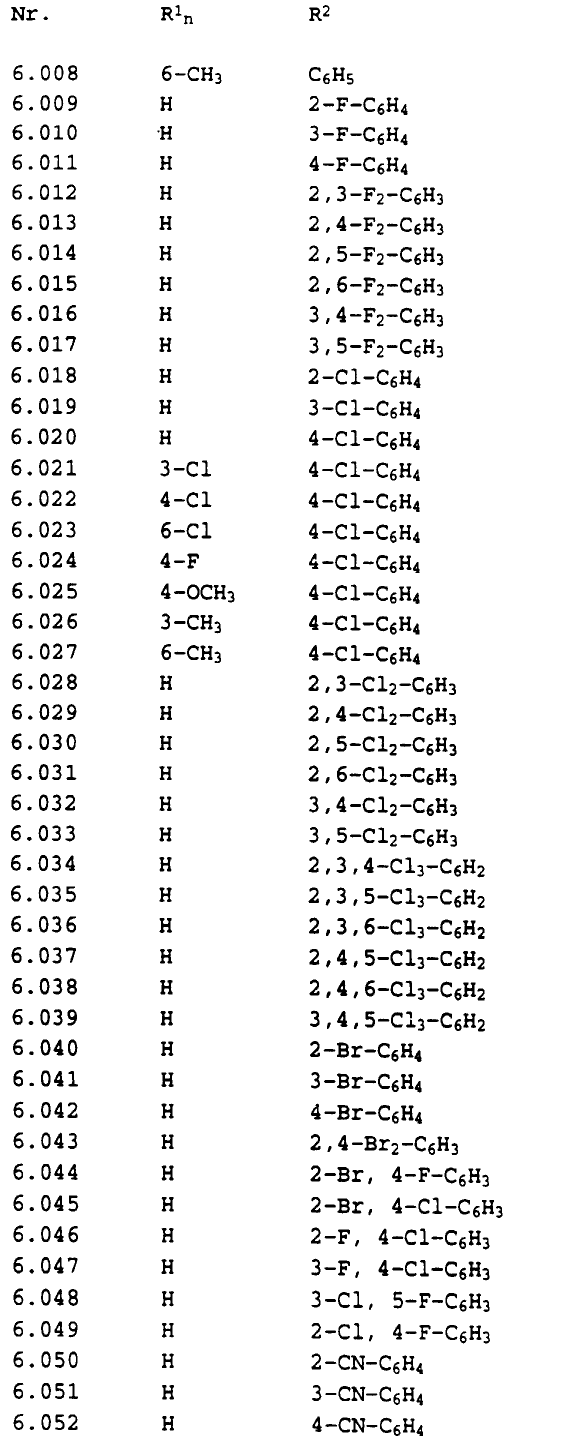

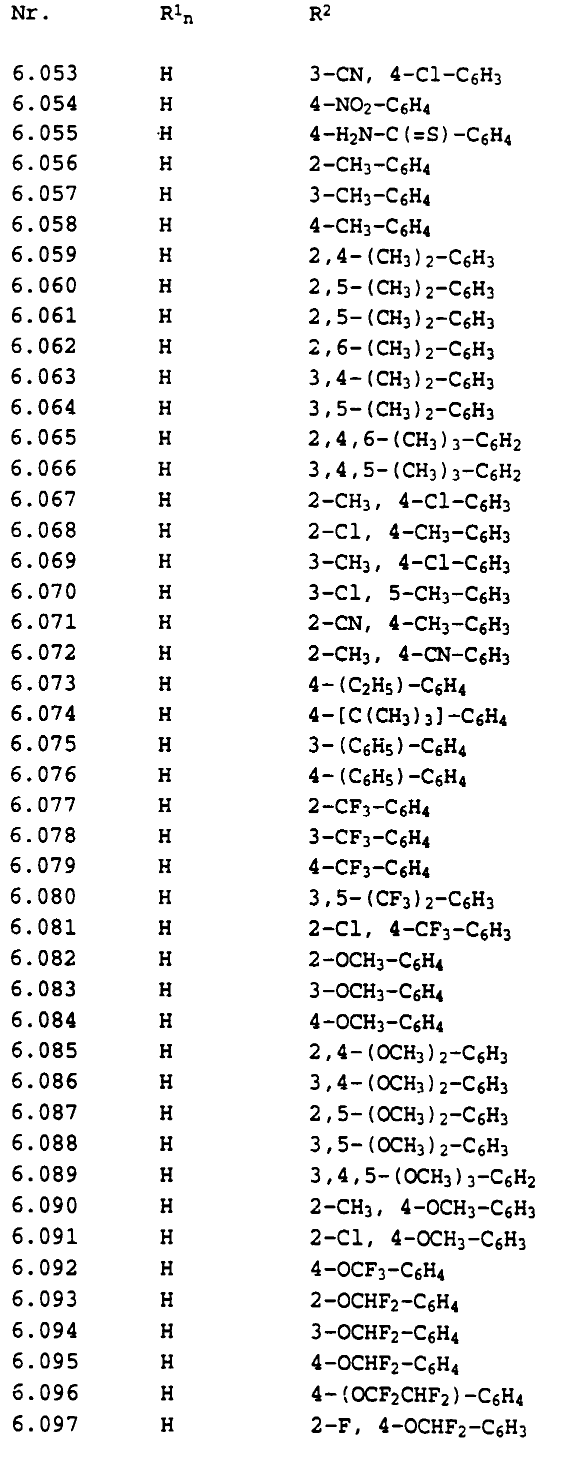

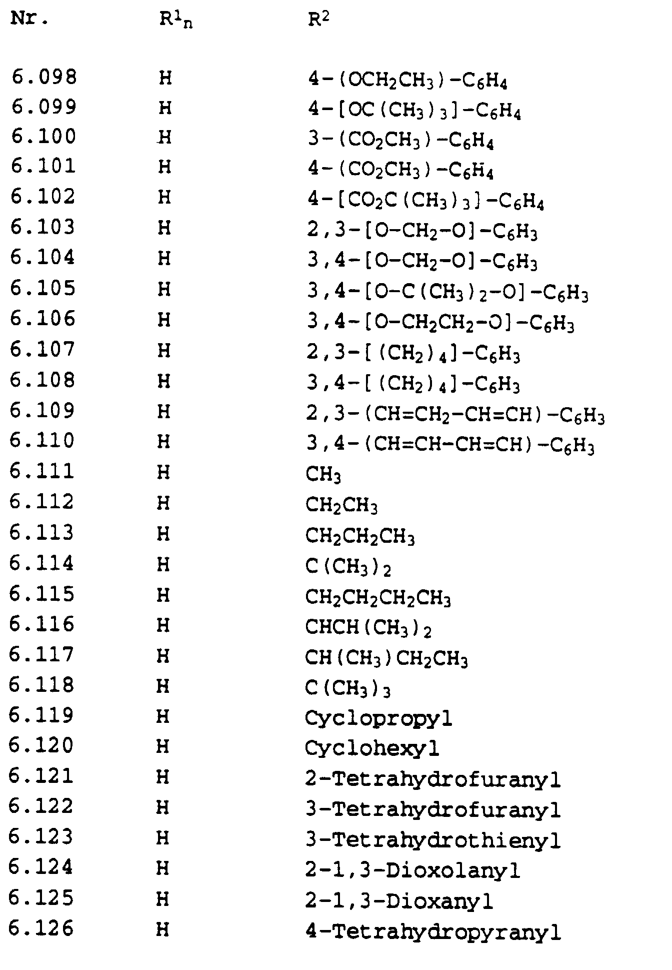

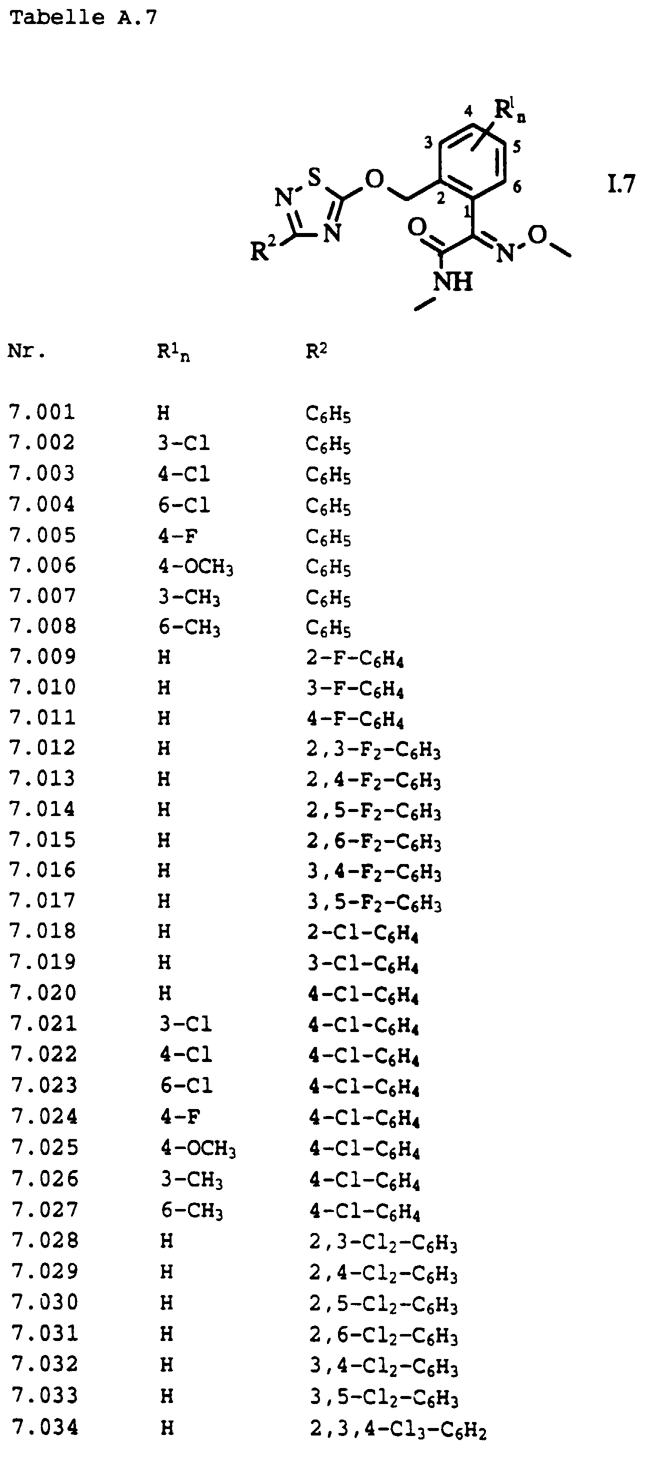

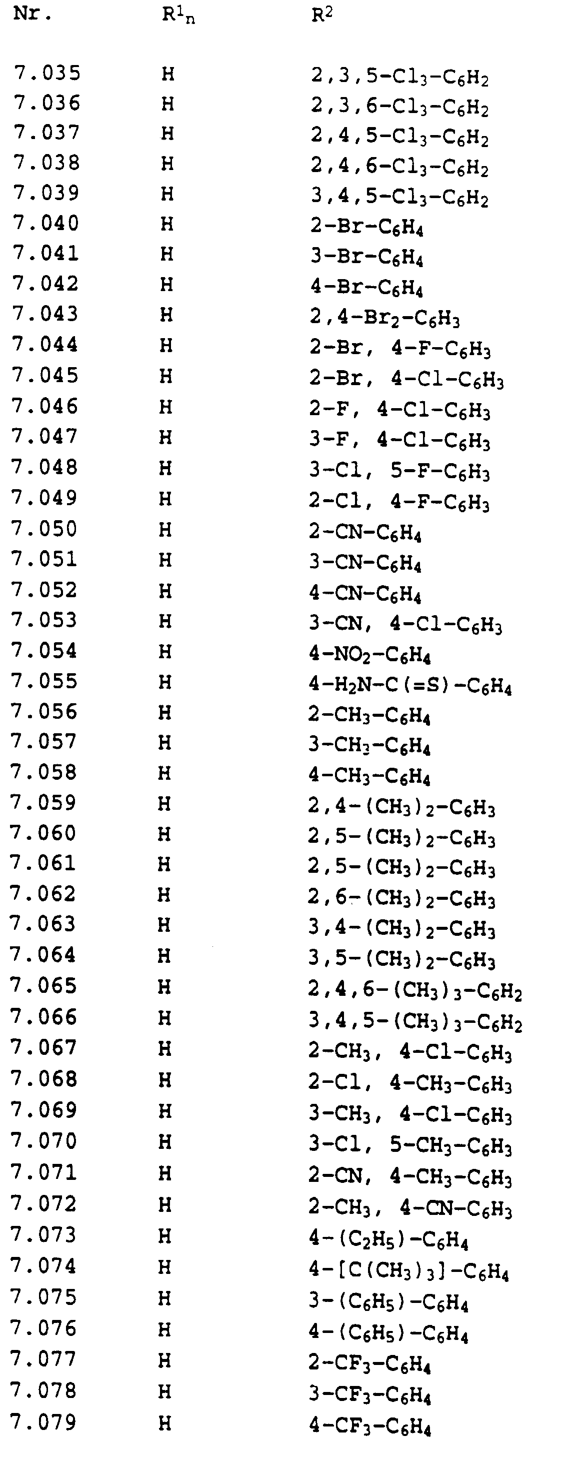

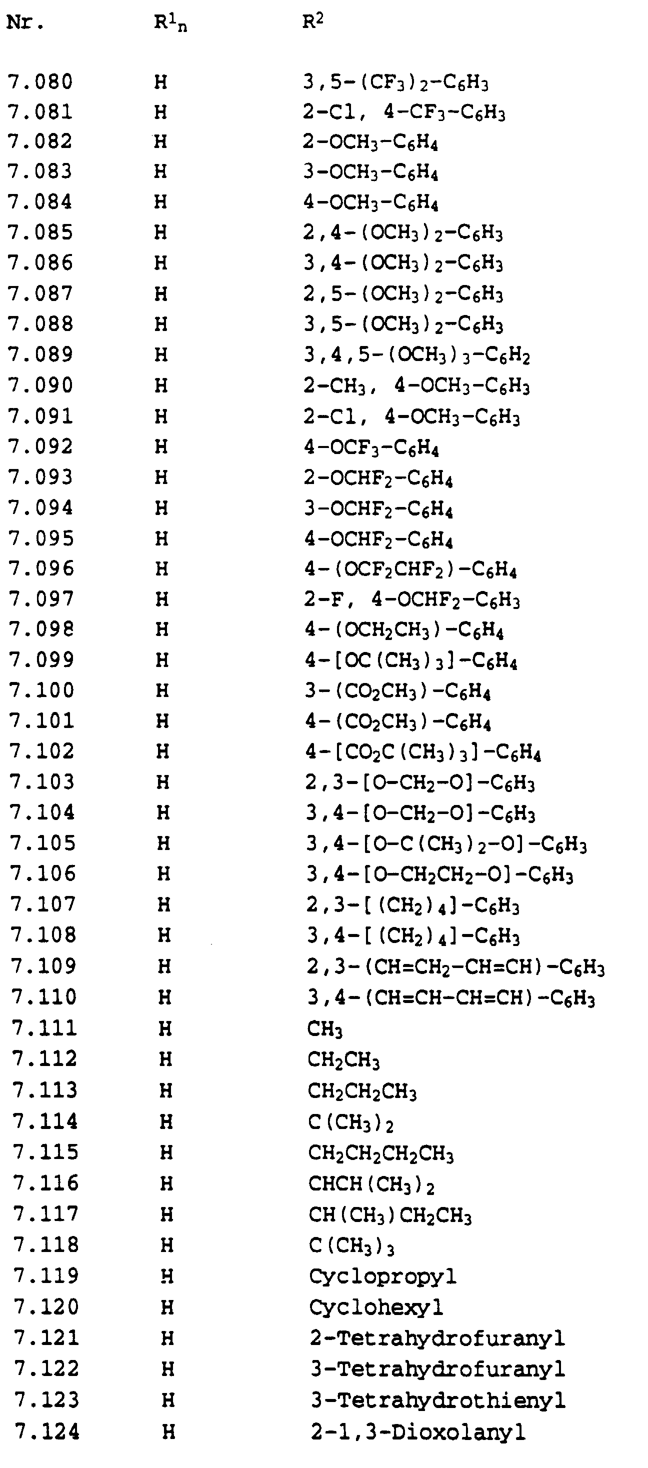

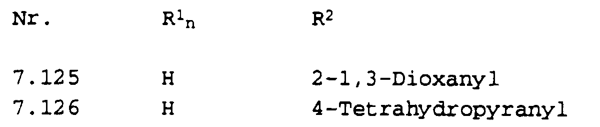

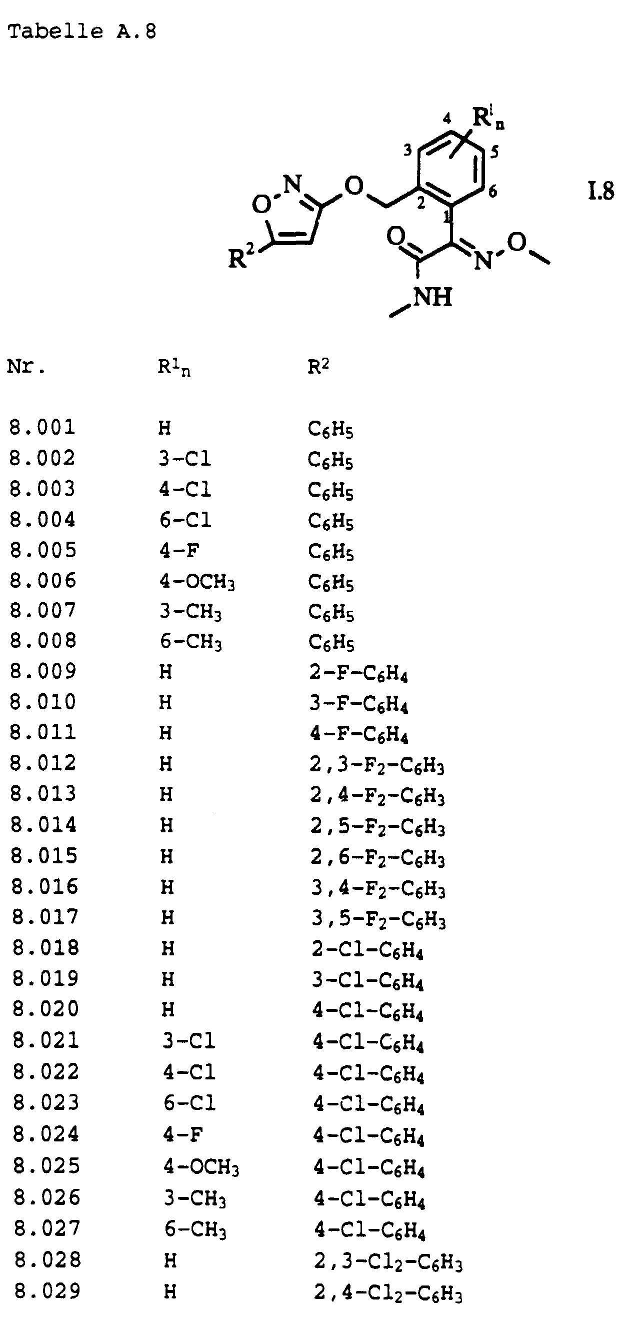

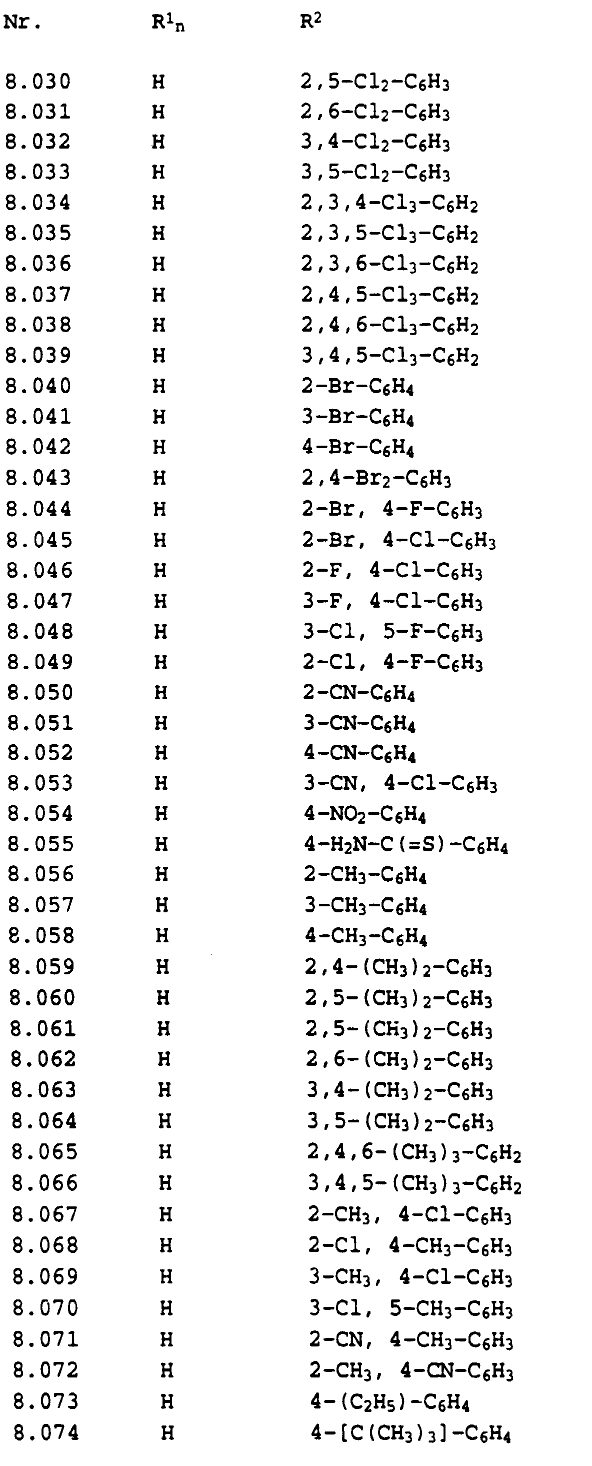

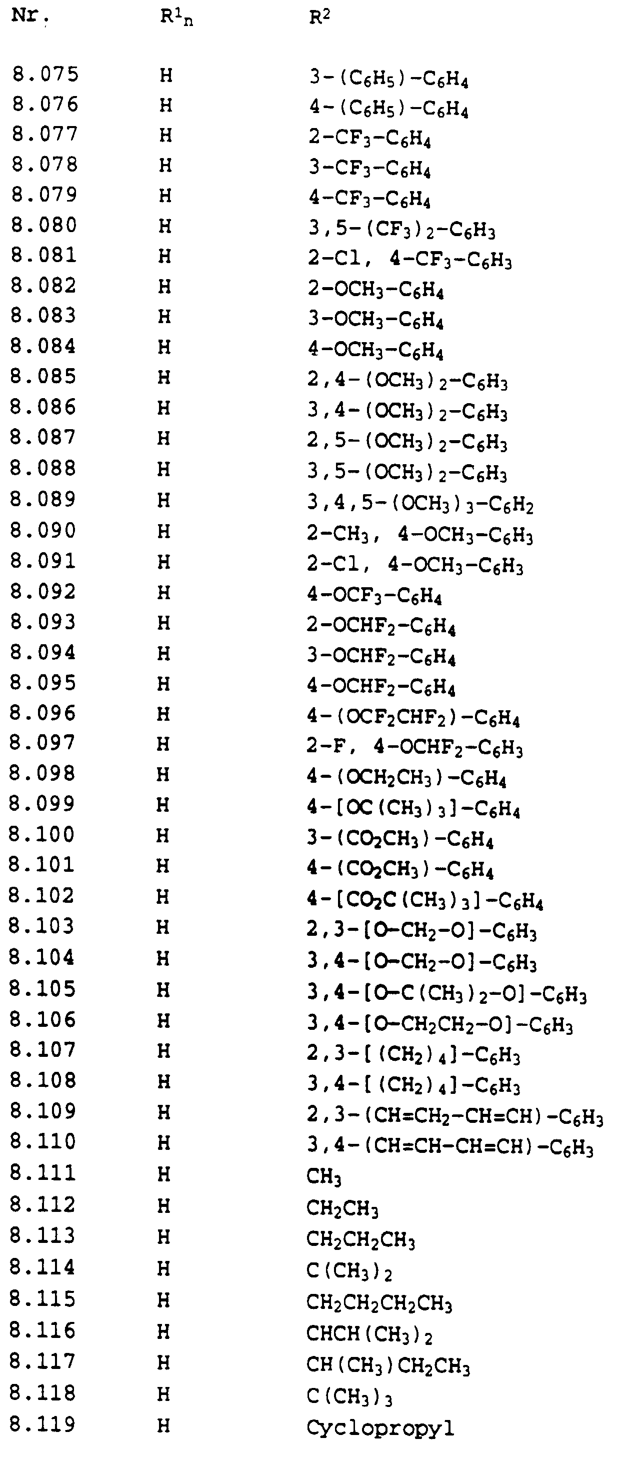

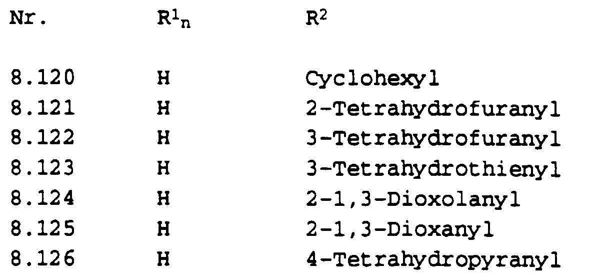

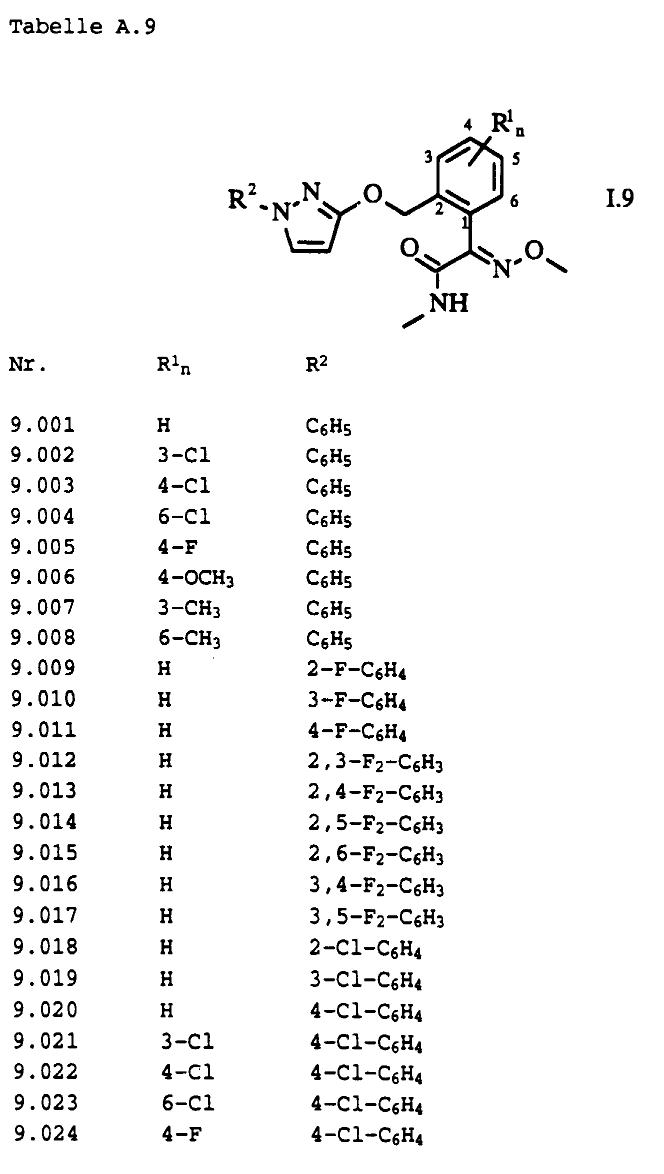

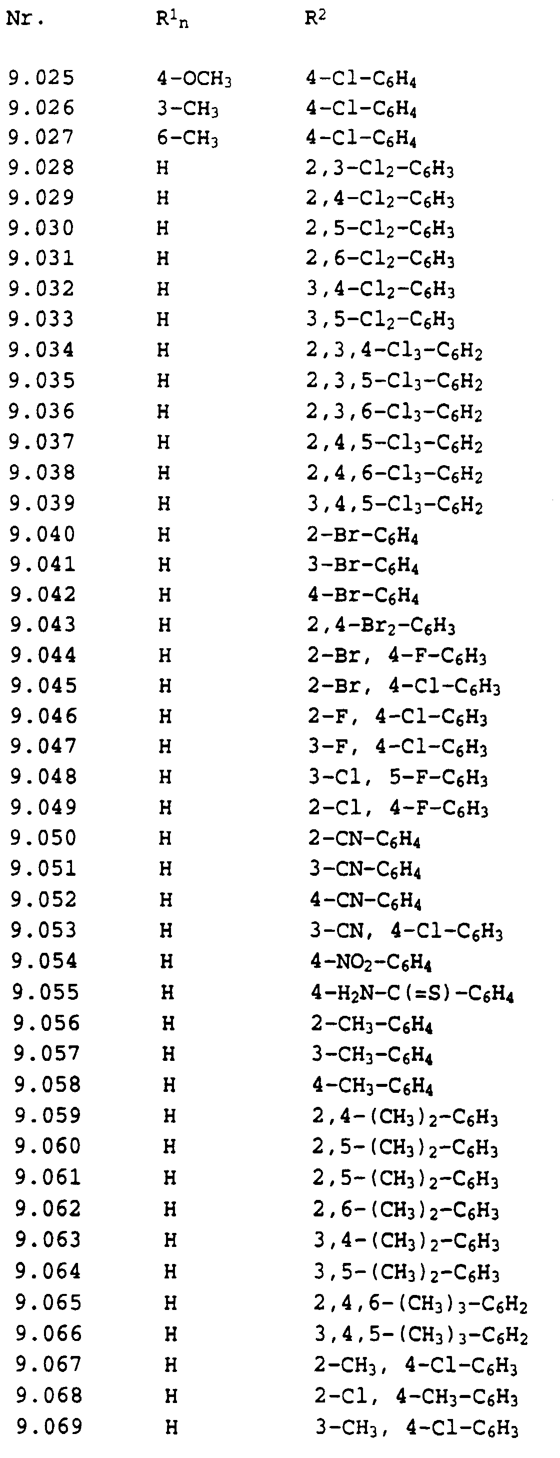

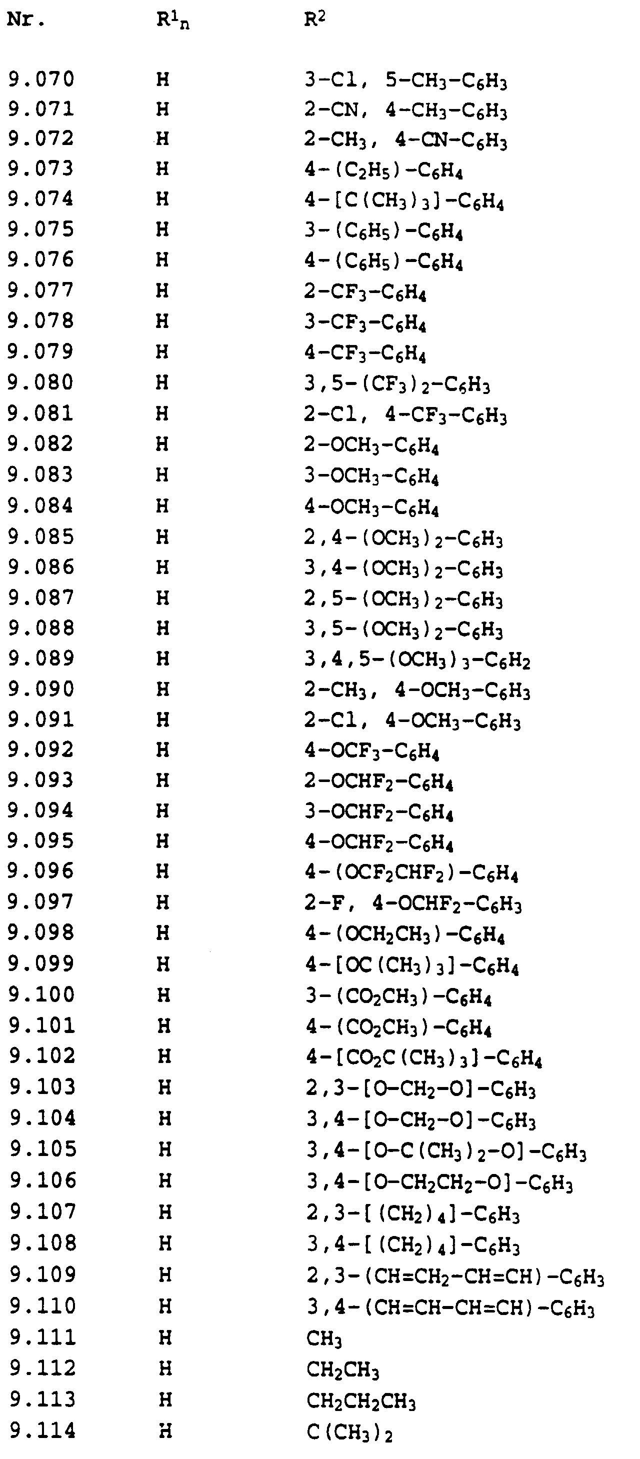

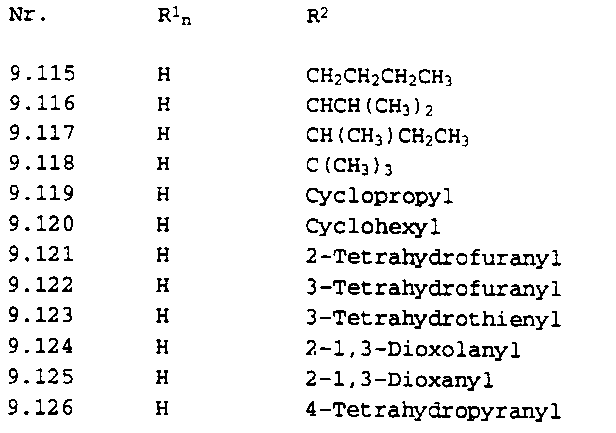

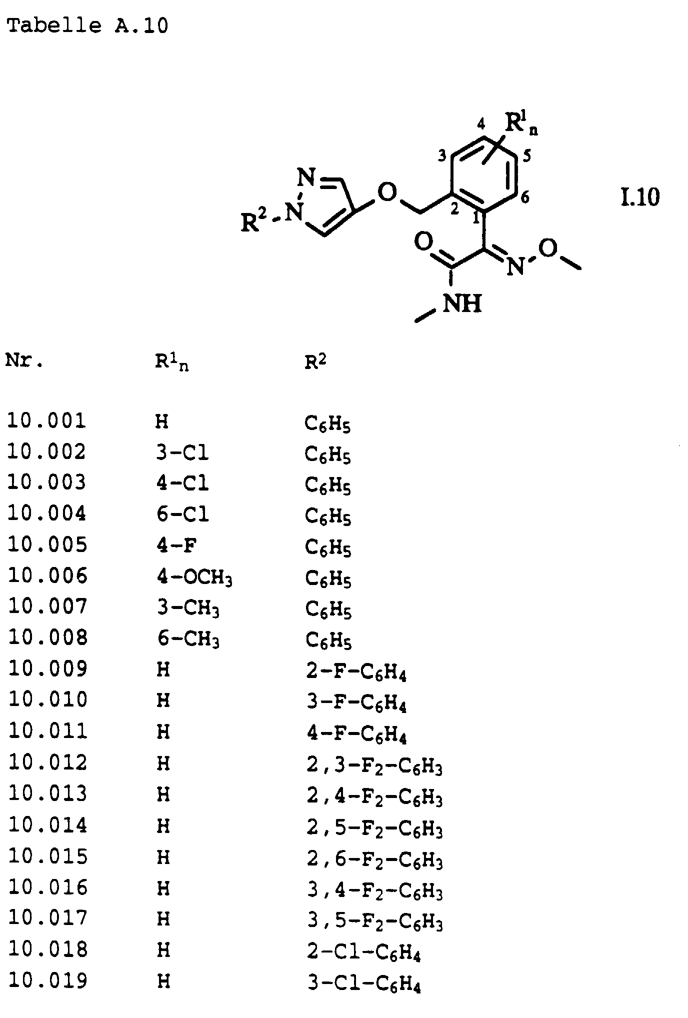

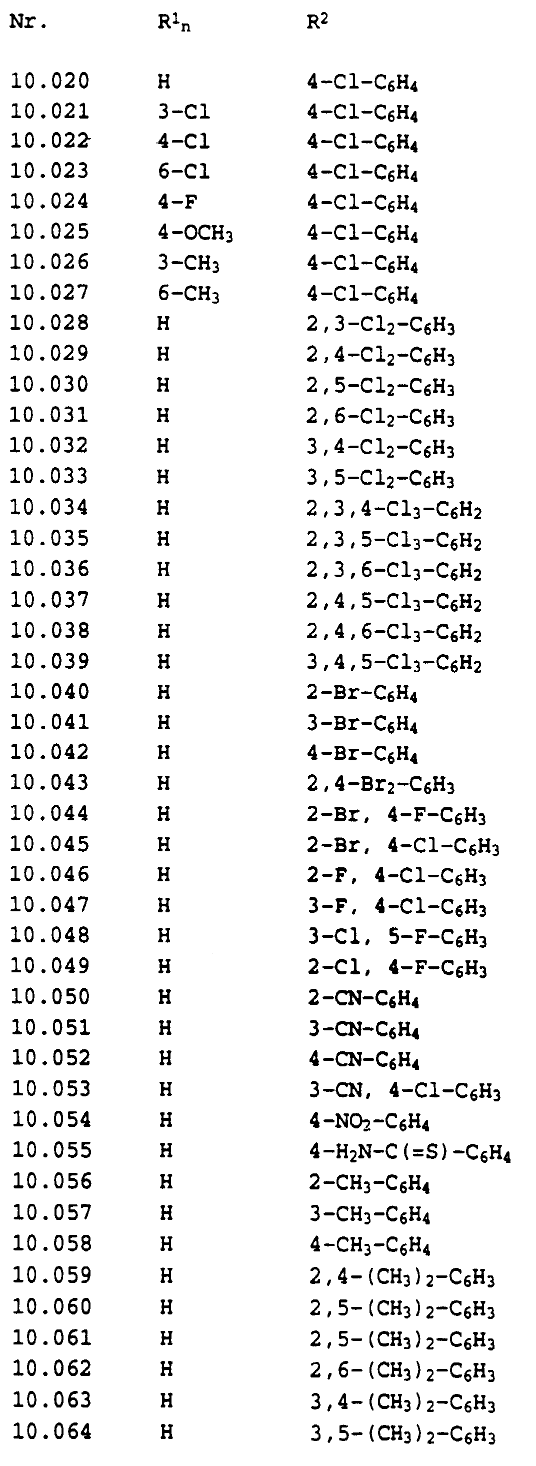

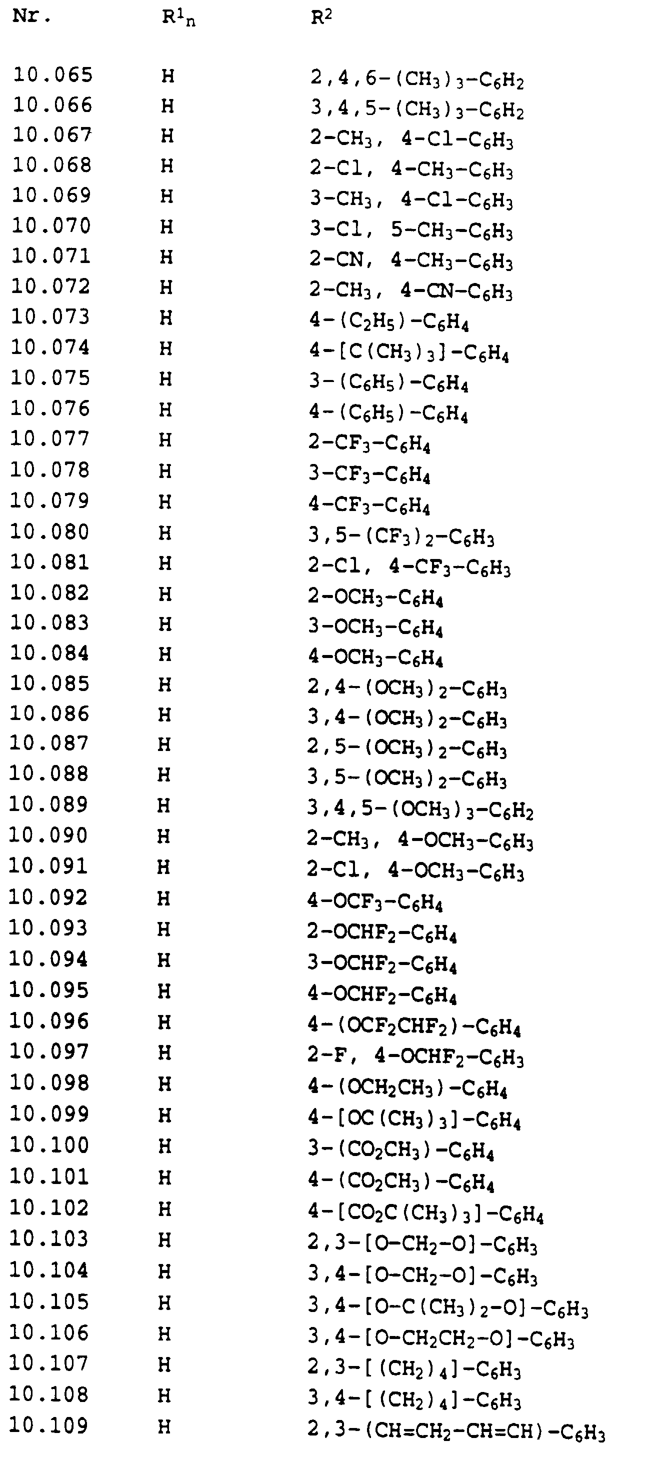

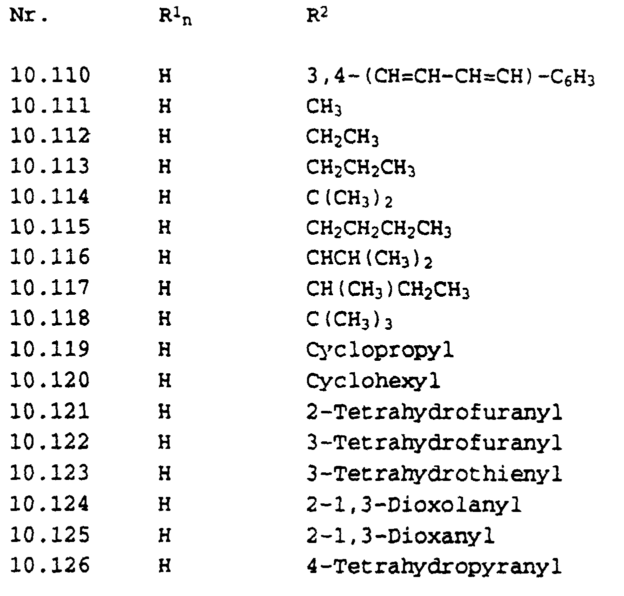

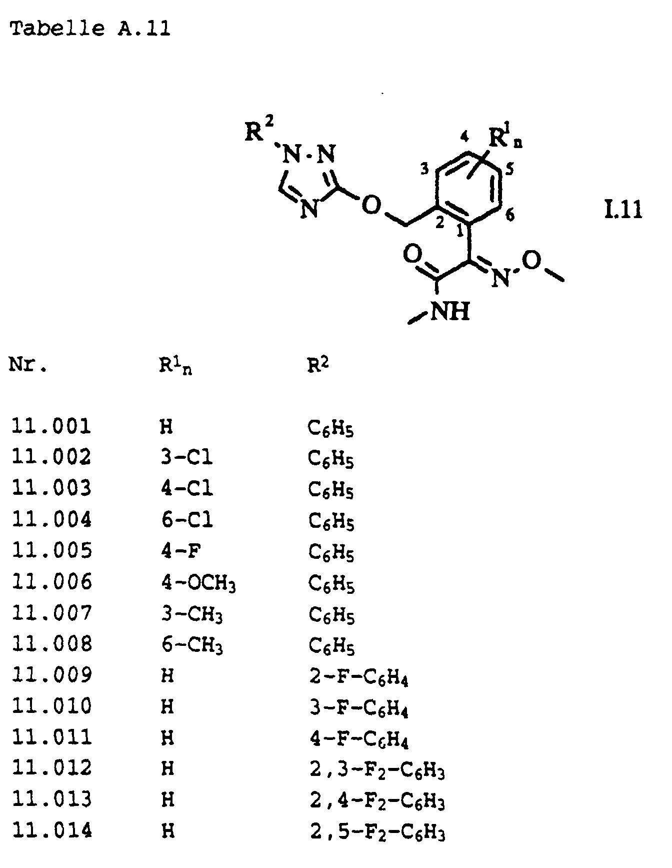

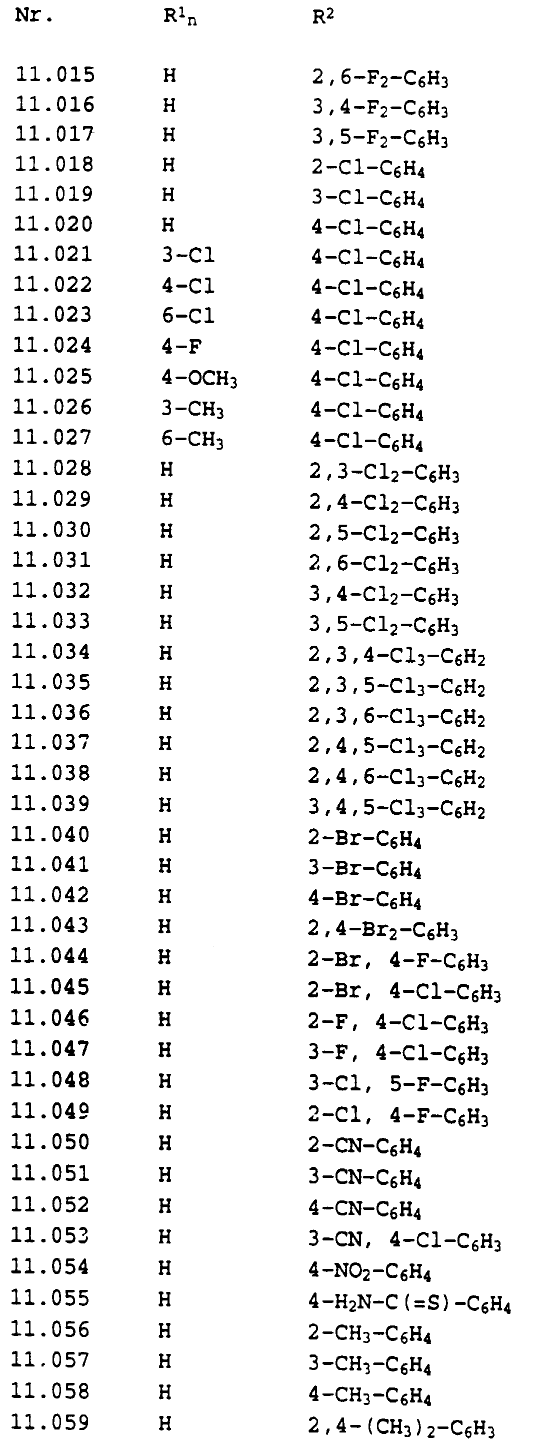

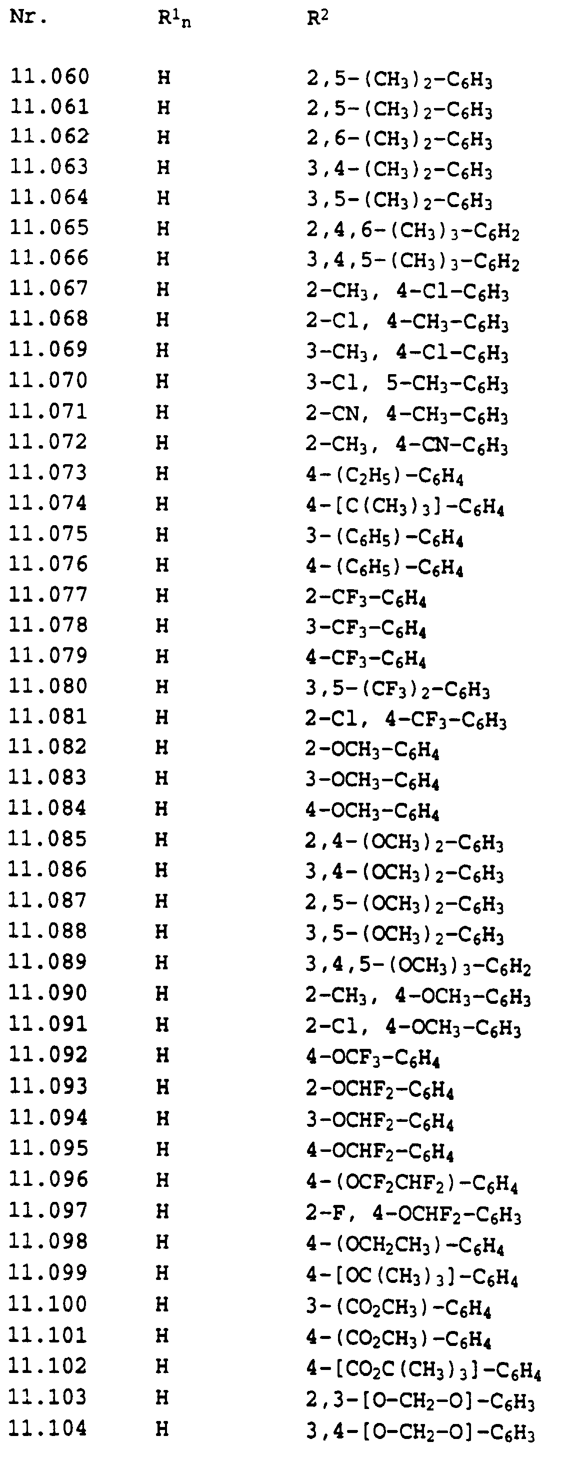

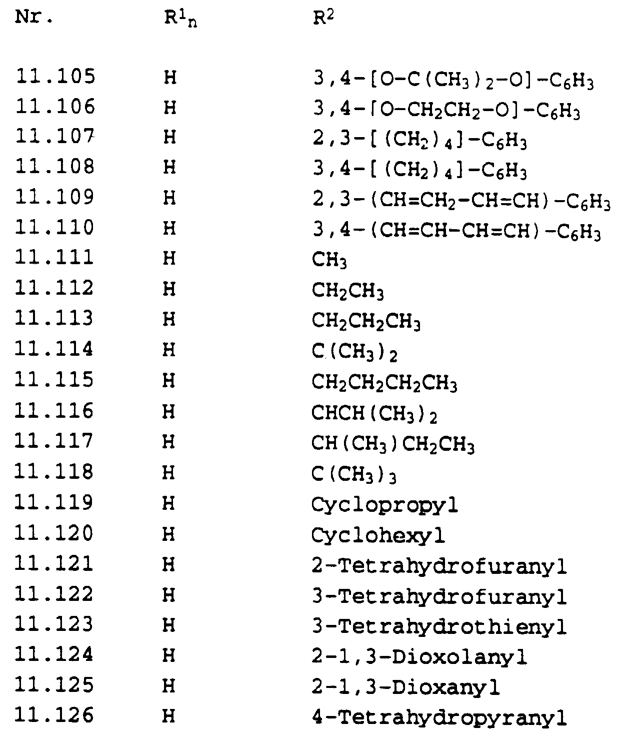

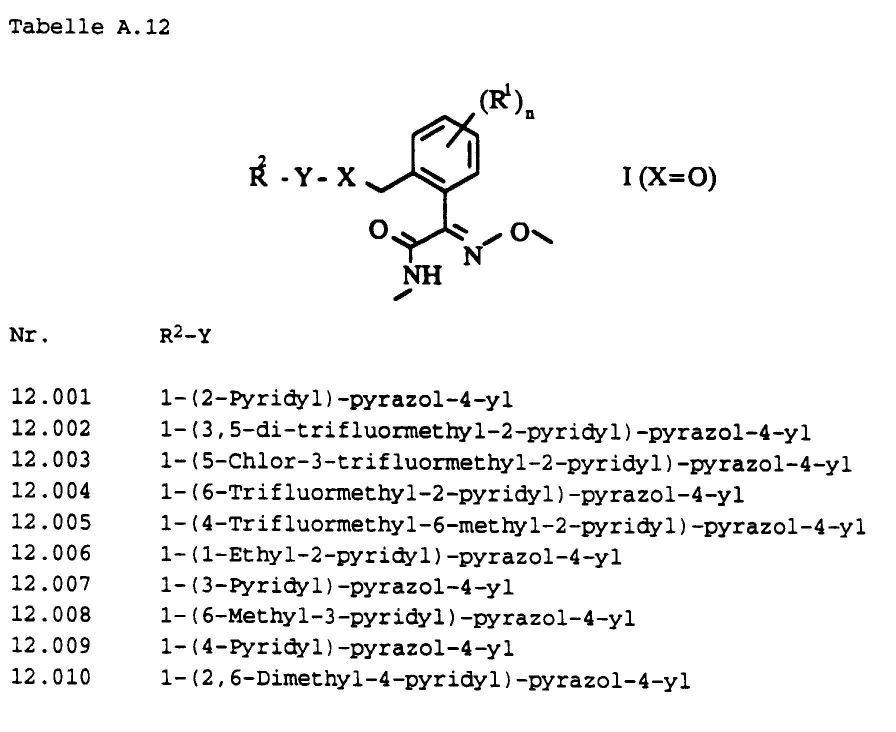

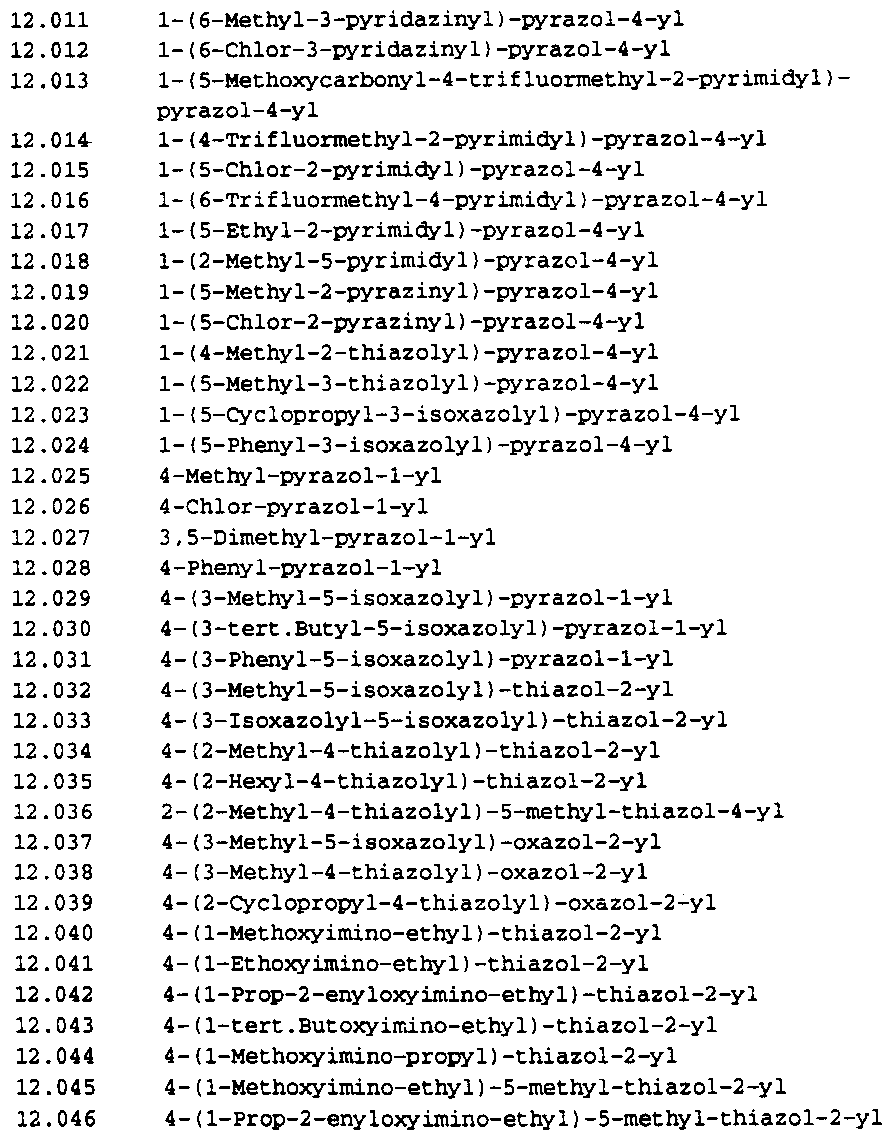

Die Herstellung der Ausgangsverbindungen IA kann beispielsweise wie unten beschrieben (Beispiel 1 bis 3) aus dem gut zugänglichen E-2-Methoxyimino-2-[(2-methylphenyloxymethyl)-phenyl]essigsäuremethylester (vgl. EP 493 711) durch Aminolyse mit Methylamin und anschließender Spaltung mit Bortrichlorid bzw. Bromwasserstoff erfolgen.

Die in den nachstehenden Synthesebeispielen wiedergegebenen Vorschriften wurden unter entsprechender Abwandlung der Ausgangsverbindungen zur Herstellung weiterer Verbindungen I benutzt. Die so erhaltenen Verbindungen sind in den nachstehenden Tabellen mit physikalischen Angaben aufgeführt.

### Beispiel 1

### E-2-Methoxyimino-2-[(2-methylphenyloxymethyl)-phenyl]-essigsäuremethylamid

250 g E-2-Methoxyimino-2-[(2-methylphenyloxymethyl)-phenyl]-essig-säuremethylester werden in 1 l 40 %ige wäßrige Methylaminlösung suspendiert und 4 h auf 40°C erwärmt. Nach Abkühlen auf Raumtemperatur (20°C) wird der Feststoff abgesaugt, mehrmals mit Wasser nachgewaschen und bei 50°C getrocknet. Man erhält 229,8 g der Titelverbindung als farblose Kristalle.
Fp.: 109 - 112°C
¹H-NMR (CDCl₃, δ in ppm):
2,20 (s, 3H); 2,85 (d, 3H); 3,95 (s, 3H); 4,90 (s, 2H); 6,7 (NH); 6,8 - 7,6 (m, 8H)

### Beispiel 2

### E-2-Methoxyimino-2-[(2-chlormethyl)-phenyl]-essigsäuremethylamid

2 g E-2-Methoxyimino-2-[(2-methylphenyloxymethyl)-phenyl]-essigsäuremethylamid aus Beispiel 1 werden in 30 ml wasserfreiem Dichlormethan bei 10°C vorgelegt. Hierzu tropft man 9,4 g Bortrichlorid (als 1molare Lösung in n-Hexan), erhitzt 1,5 h auf Rückfluß, kühlt auf 10°C ab, gibt nochmals 9,4 g Bortrichlorid zu und läßt über Nacht bei Raumtemperatur (20°C) rühren. Nach Zutropfen von 8,2 g Methanol wird der Ansatz einrotiert. Der Rückstand wird in 100ml Dichlormethan aufgenommen, mit 5 % Natronlauge und dann mit Wasser gewaschen. Die organ. Phase wird abschließend über Natriumsulfat getrocknet. Nach Abziehen des Lösungsmittels verbleiben 1,2 g der Titelverbindung als Öl.
¹H-NMR (CDCl₃, δ in ppm):
2,95 (d, 3H); 3,90 (s, 3H); 4,45 (s, 2H); 6,8 (NH); 7,1 - 7,6 (m, 4H)

### Beispiel 3

### E-2-Methoxyimino-2-[(2-brommethyl)-phenyl]-essigsäuremethylamid

10 g E-2-Methoxyimino-2-[(2-methylphenyloxymethyl)-phenyl]-essigsäuremethylamid aus Beispiel 1 werden in 50 ml wasserfreiem Dichlormethan vorgelegt. In die Lösung wird Bromwasserstoff bis zur Sättigungskonzentration (ca. 9 g HBr) eingeleitet. Nach 68 h Rühren bei Raumtemperatur ist das Edukt vollständig umgesetzt. Nach Zugabe von weiteren 50 ml Dichlormethan wird wie in Beispiel 2 aufgearbeitet. Es verbleiben 7,0 g der Titelverbindung als Öl, welches beim Stehenlassen durchkristallisiert.
Fp.: 128 - 129°C
¹H-NMR (CDCl₃, δ in ppm):
2,95 (d, 3H); 3,95 (s, 3H); 4,35 (s, 2H); 6,85 (NH); 7,1 - 7,5 (m, 4H)

### Beispiel 4

### E-2-Methoxyimino-2-[(2-[5-(4-chlorphenyl)-isoxazol-3-yl]-oxymethyl)-phenyl]-essigsauremethylester

1,8 g 3-Hydroxy-5-(4-chlorphenyl)-isoxazol werden in 10 ml Dimethylformamid gelöst. Hierzu gibt man 1,6 g fein gepulvertes Kaliumcarbonat und 2,8 g E-2-Methoxyimino-2-[(2-brommethyl)-phenyl]-essigsäuremethylester. Man rührt über Nacht bei Raumtemperatur und gießt dann zur Aufarbeitung den Reaktionsansatz auf 50 ml Wasser. Nach dreimaligem Extrahieren mit Methyltert.butylether werden die vereinigten organischen Phasen über Natriumsulfat getrocknet. Nach Abziehen des Lösungsmittels wird der Rückstand mit Methyl-tert.butylether verrieben. Nach Absaugen verbleiben 1,8 g der Titelverbindung.
Fp.: 155 - 157°C
¹H-NMR (CDCl₃, δ in ppm):
3,85 (s, 3H); 4,06 (s, 3H); 5,2 (s, 2H); 6,1 (s, 1H); 7,2-7,7 (m, 8H)

### Beispiel 5

### E-2-Methoxyimino-2-[(2-[5-(4-chlorphenyl)-isoxazol-3-yl]-oxymethyl)-phenyl]-essigsäuremethylamid

1,0 g des Methylesters aus Beispiel 4 werden in 15 ml Tetrahydrofuran gelost und mit 1 ml 40 %iger wäßriger Methylaminlösung versetzt. Man rührt über Nacht bei Raumtemperatur, engt den Ansatz ein und nimmt den Rückstand in 50ml Methyl-tert.butylether auf. Die organische Phase wird mit Wasser extrahiert, über Natriumsulfat getrocknet und abschließend bis zur Trockne eingedampft. Es verbleiben 0,9 g der Titelverbindung als farbloser Feststoff.
Fp.: 195 - 198°C
¹H-NMR (CDCl₃, δ in ppm):
2,95 (d, 3H); 3,95 (s, 3H); 5,2 (s, 2H); 6,15 (s, 1H); 6,85 (NH); 7,2-7,8 (m, 8H)

### Beispiel 6

### E-2-Methoxyimino-2-[(2-[5-(4-Trifluormethylphenyl)-1,3,4-oxadiazol-2- yl]-thiomethyl)phenyl]-essigsäuremethylamid

1,35 g 5-(4-Trifluormethylphenyl)-2-mercapto-1,3,4-oxadiazol und 1,43 g der Brommethylverbindung aus Beispiel 3 werden in 30 ml Aceton gelöst. Nach Zugabe von 0,8 g fein gepulvertem Kaliumcarbonat wird der Ansatz bei RT über Nacht gerührt. Man saugt vom Feststoff ab und dampft das Filtrat zur Trockne ein. Der verbliebene Rückstand wird mit n-Pentan verrieben und abgesaugt. Es verbleiben 2,2 g der Titelverbindung als farbloser Feststoff.
Fp.: 128-129°C
¹H-NMR (CDCl₃, δ in ppm):

### Beispiel 7

### E-2-Methoxyimino-2-[(2-[1-(4-chlor-2-methylphenyl)-pyrazol-4-yl]-oxymethyl)-phenyl]-essigsäuremethylamid

1,05 g 1-(4-Chlor-2-methylphenyl)-4-hydroxypyrazol werden in 20 ml Dichlormethan vorgelegt. Nach Zugabe von 50 mg Tetra-n-butylammoniumiodid und einer Lösung von 1,43 g Brommethylverbindung aus Beispiel 3 in 10 ml Dichlormethan, wird die Reaktion mit 20 ml 10%iger Natronlauge gestartet. Das Zweiphasensystem wird bis zum vollständigen Umsatz der Brommethylverbindung heftig gerührt. Man trennt die organische Phase ab, extrahiert noch zweimal mit Dichlormethan und trocknet die vereinigten organischen Phasen über Natriumsulfat. Das nach Abziehen des Lösungsmittels verbleibende Rohprodukt wird in 70 ml Isopropylether/Aceton (5:2, v/v) aufgenommen. Man dekantiert von unlöslichen Bestandteilen ab, engt die Flüssigkeit ein und chromatographiert über Kieselgel (Laufmittel Toluol/Ethylacetat, 9:1).

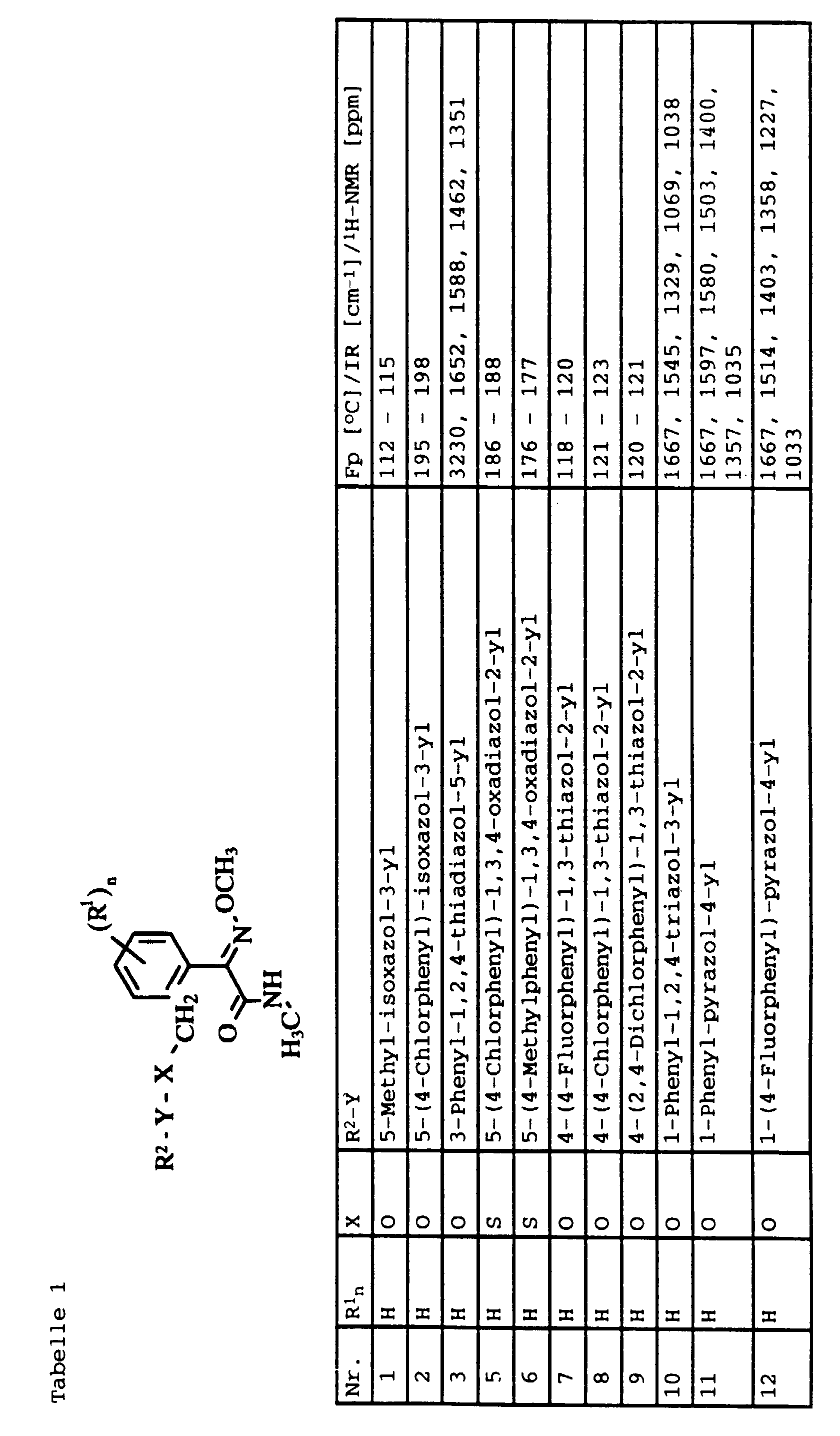

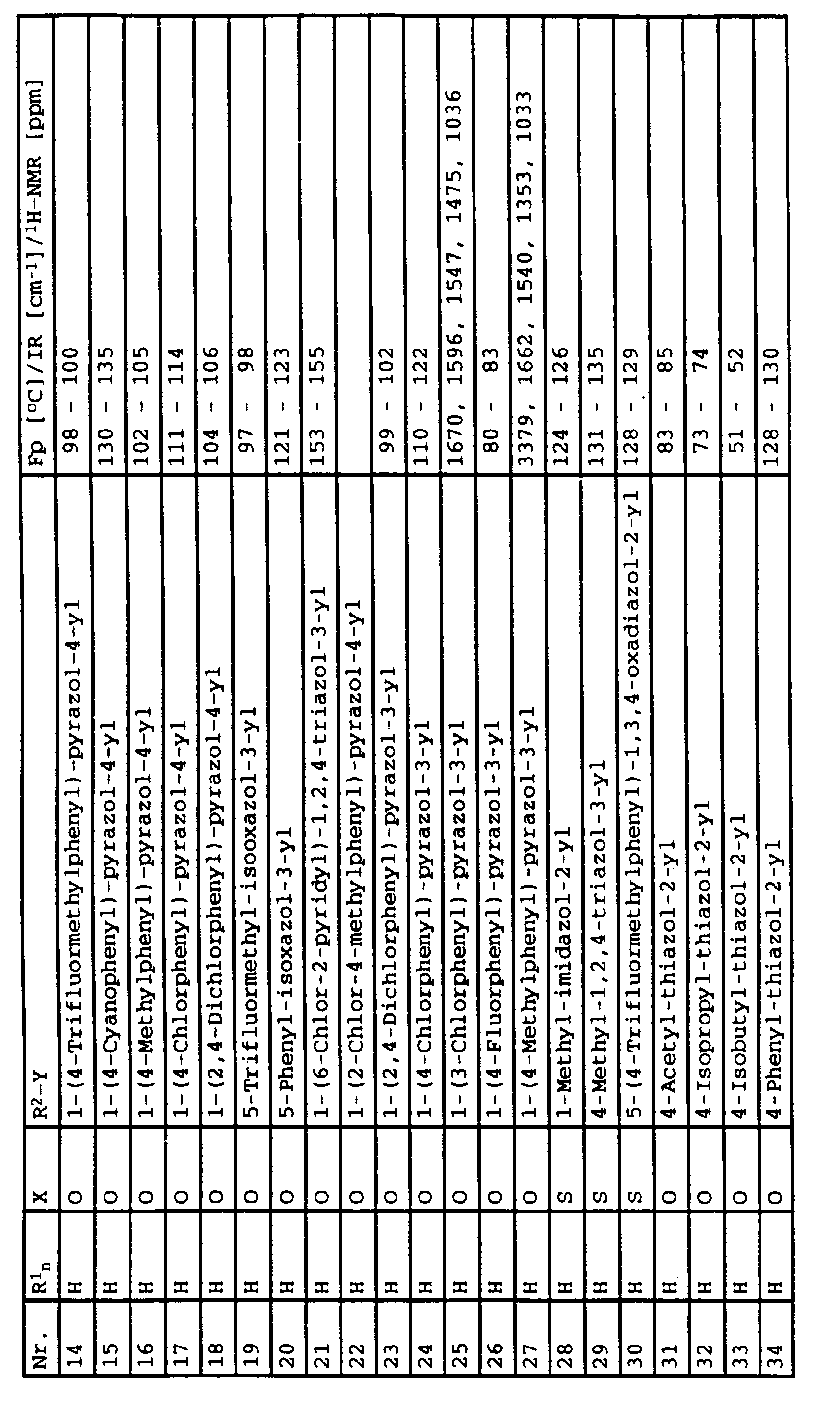

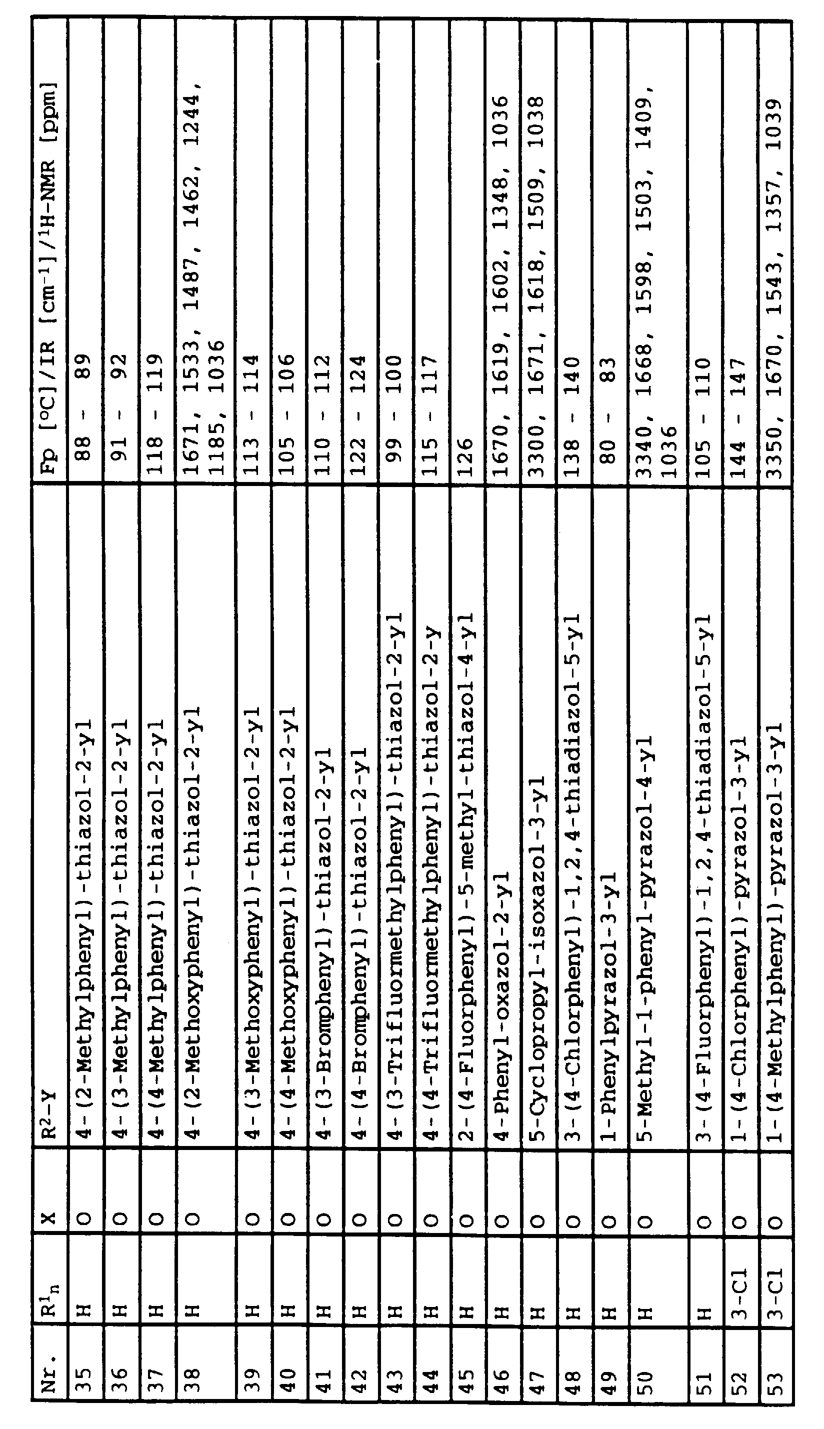

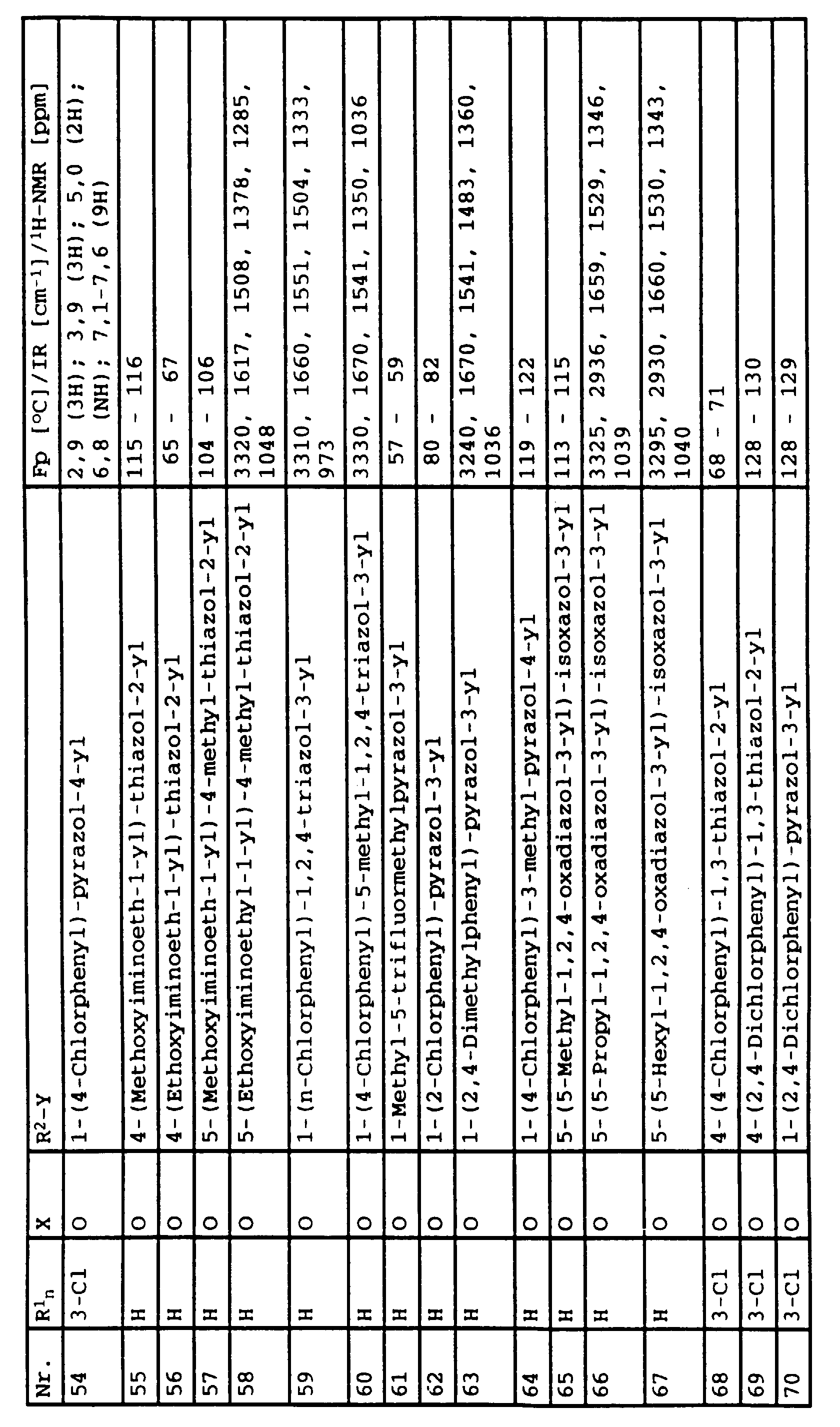

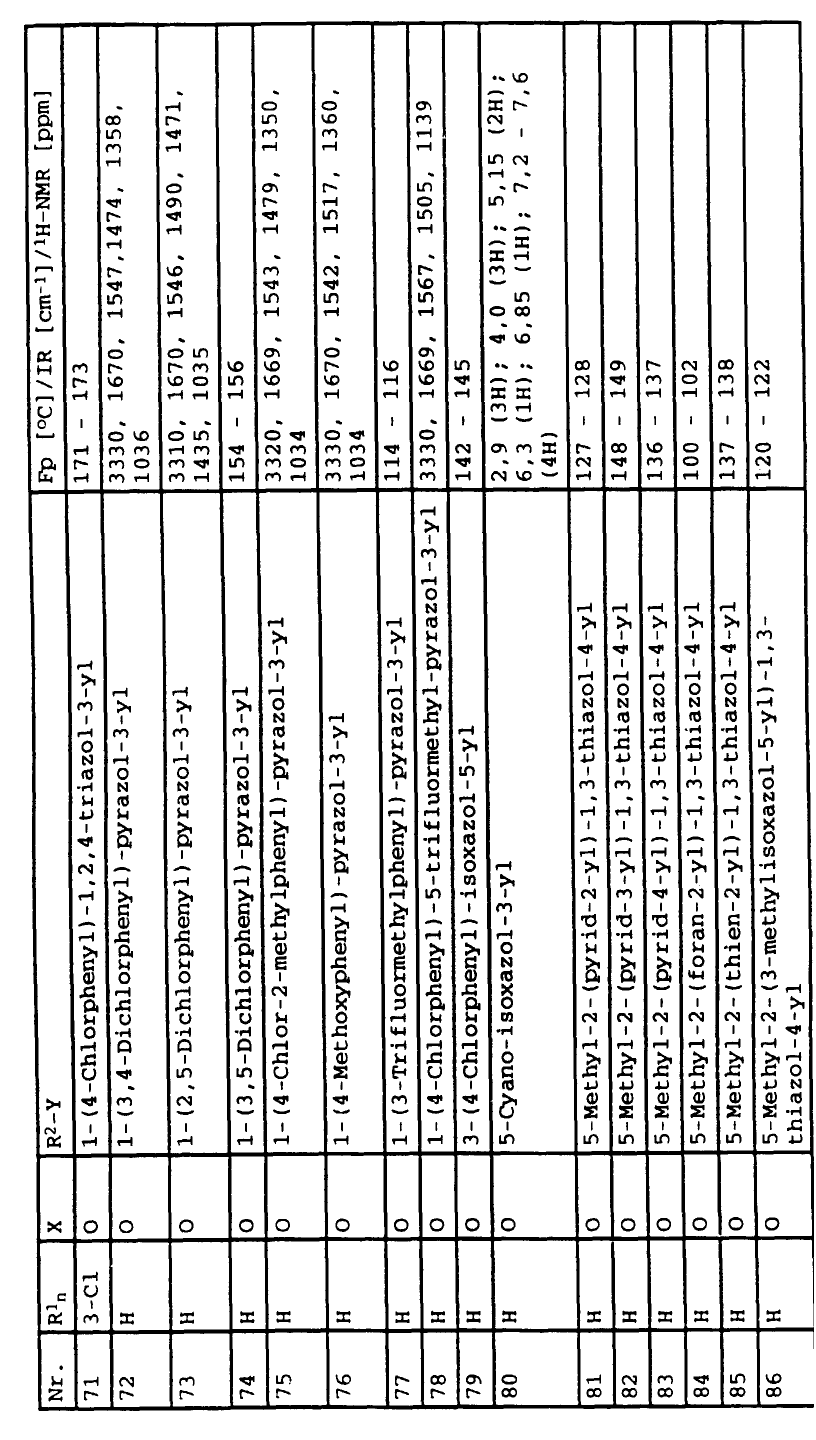

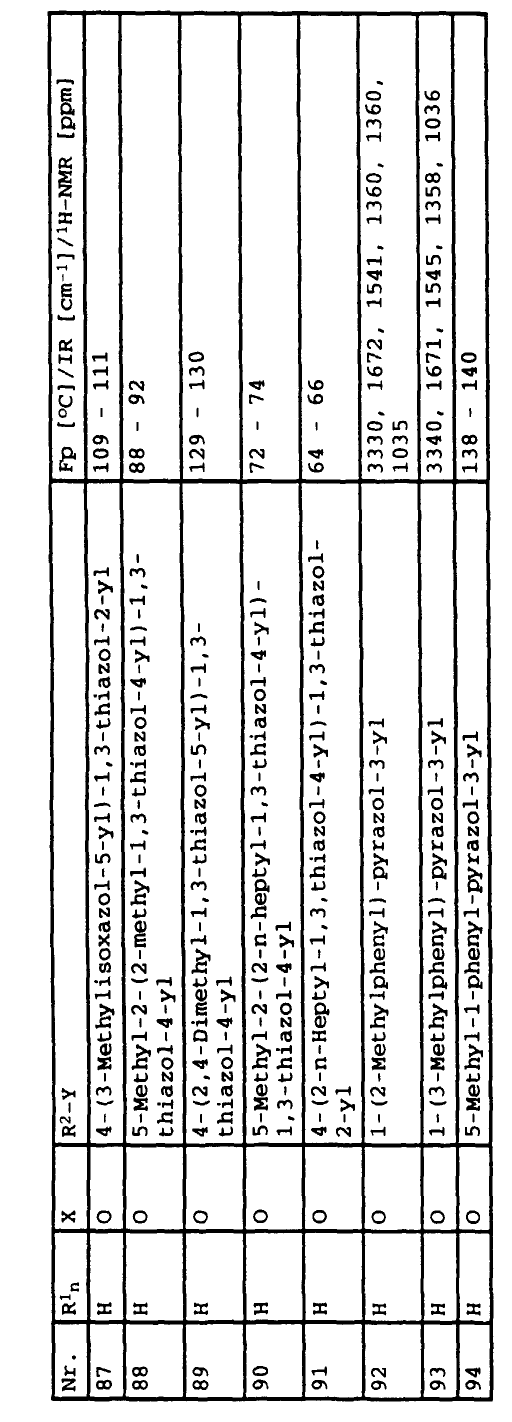

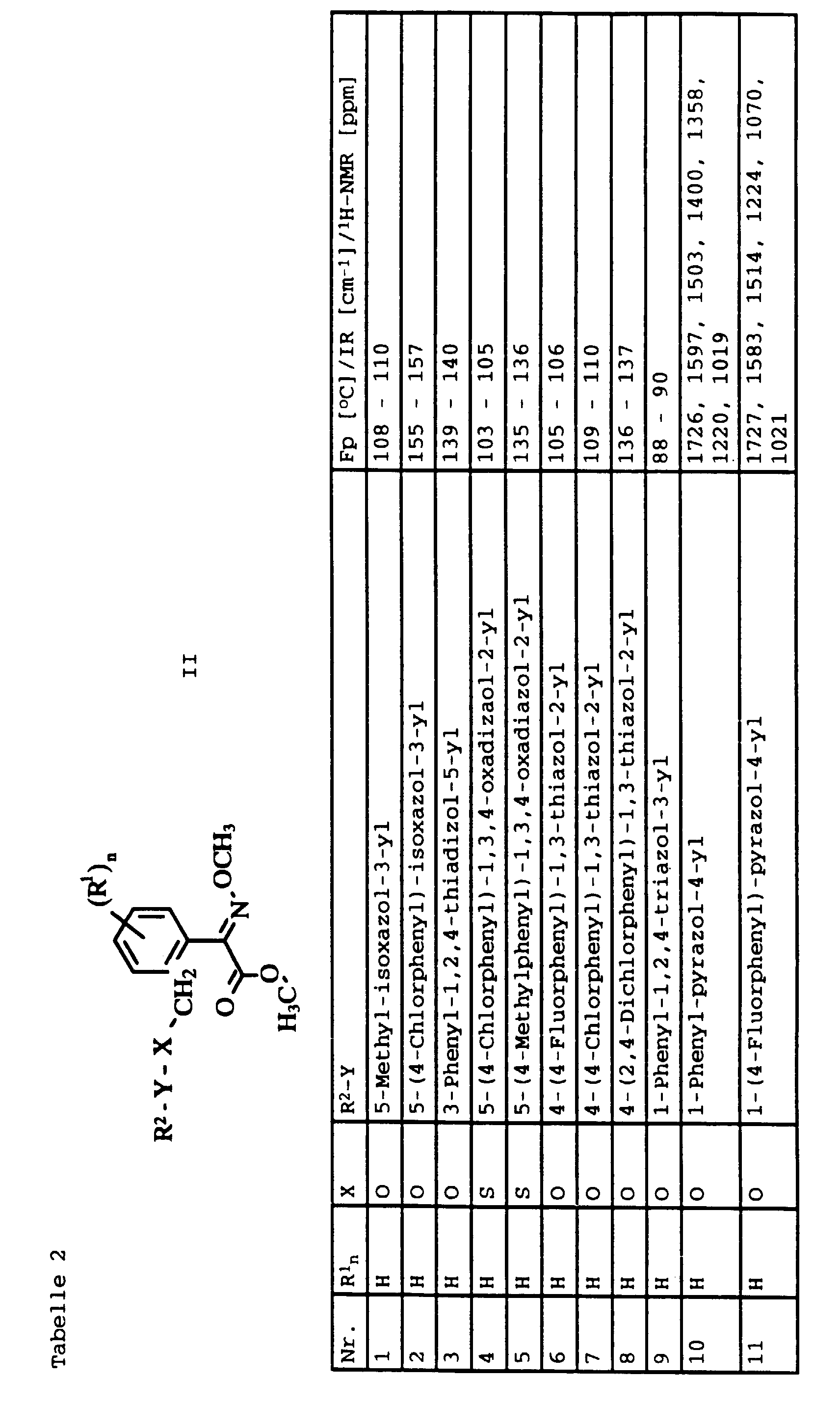

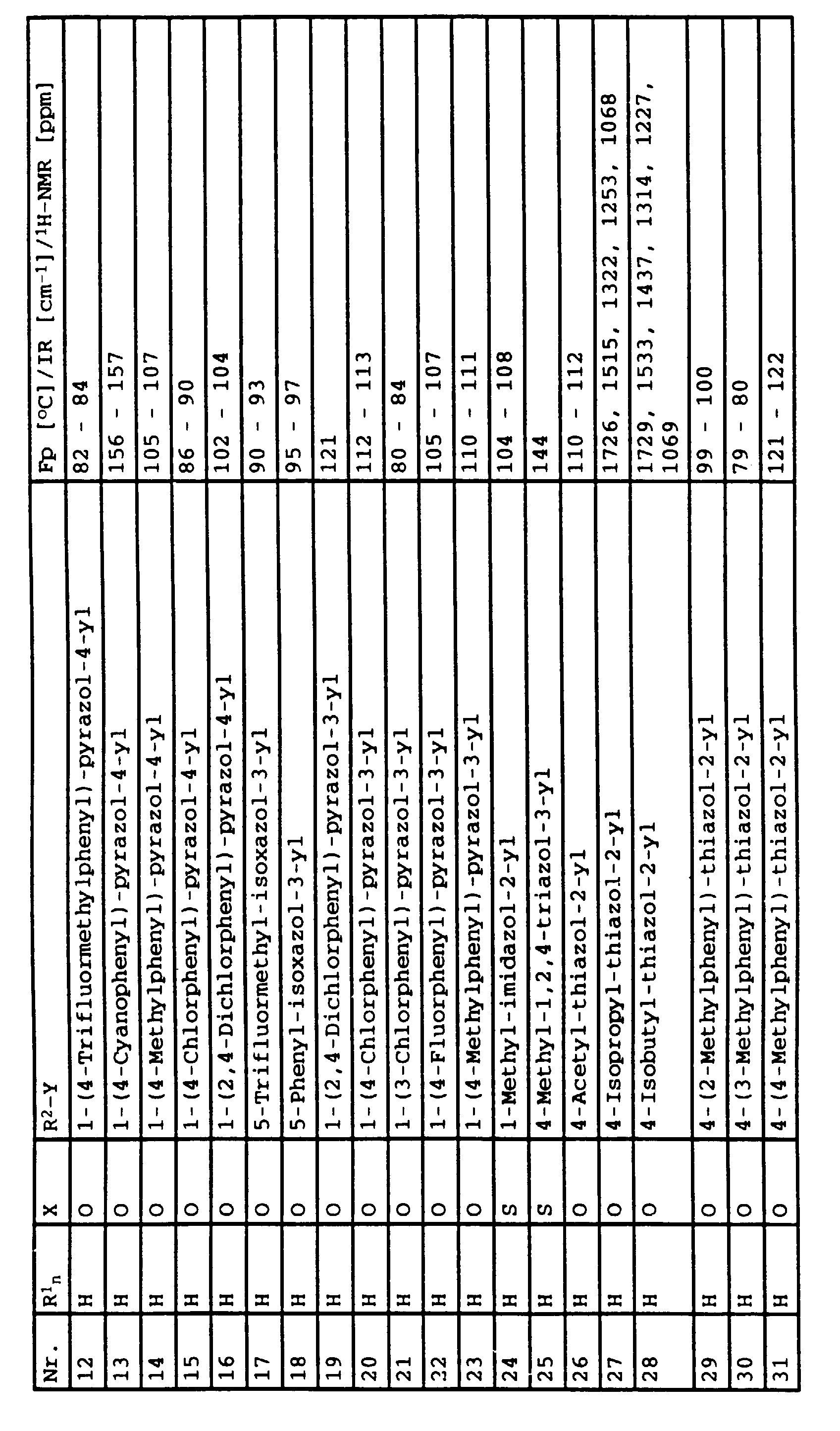

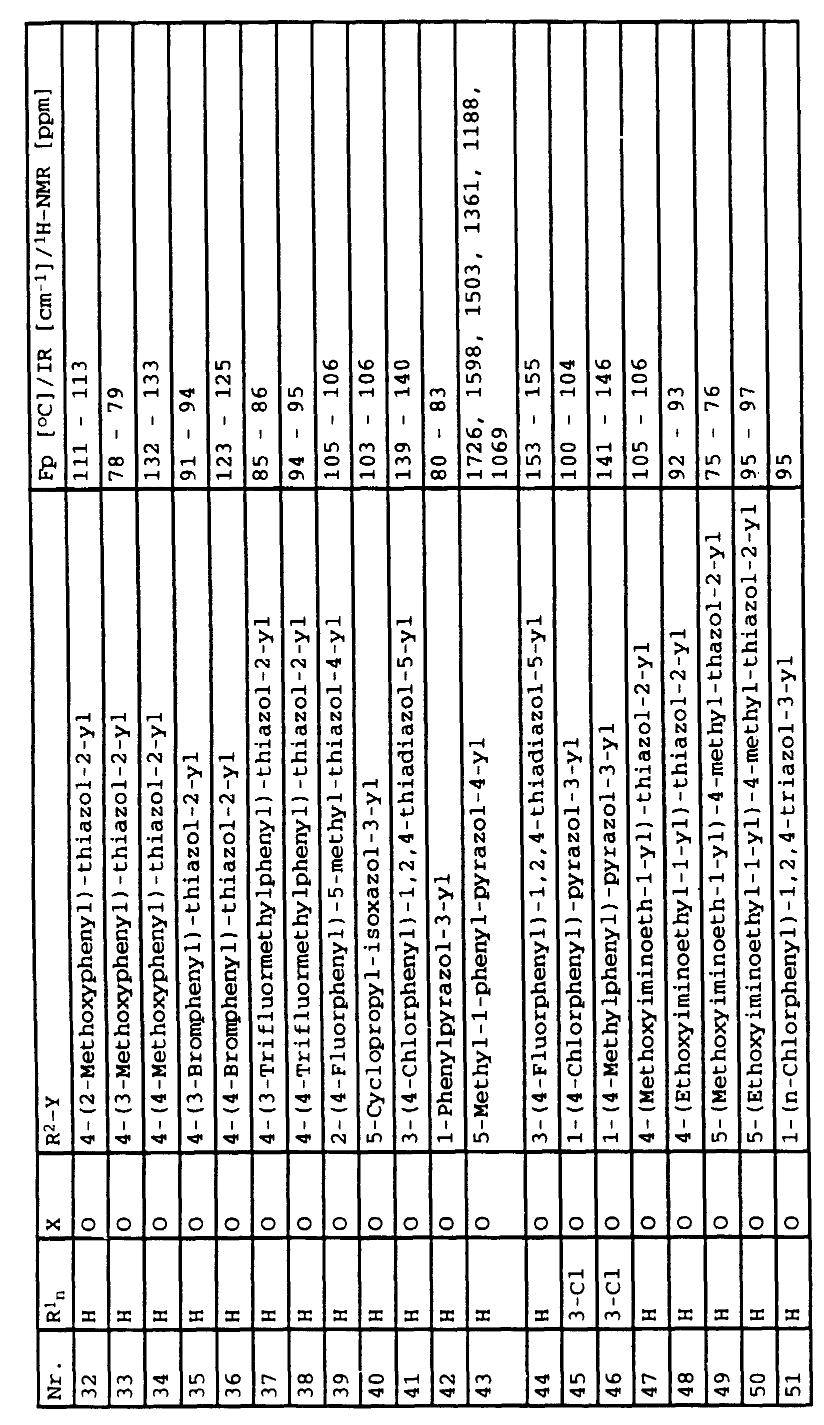

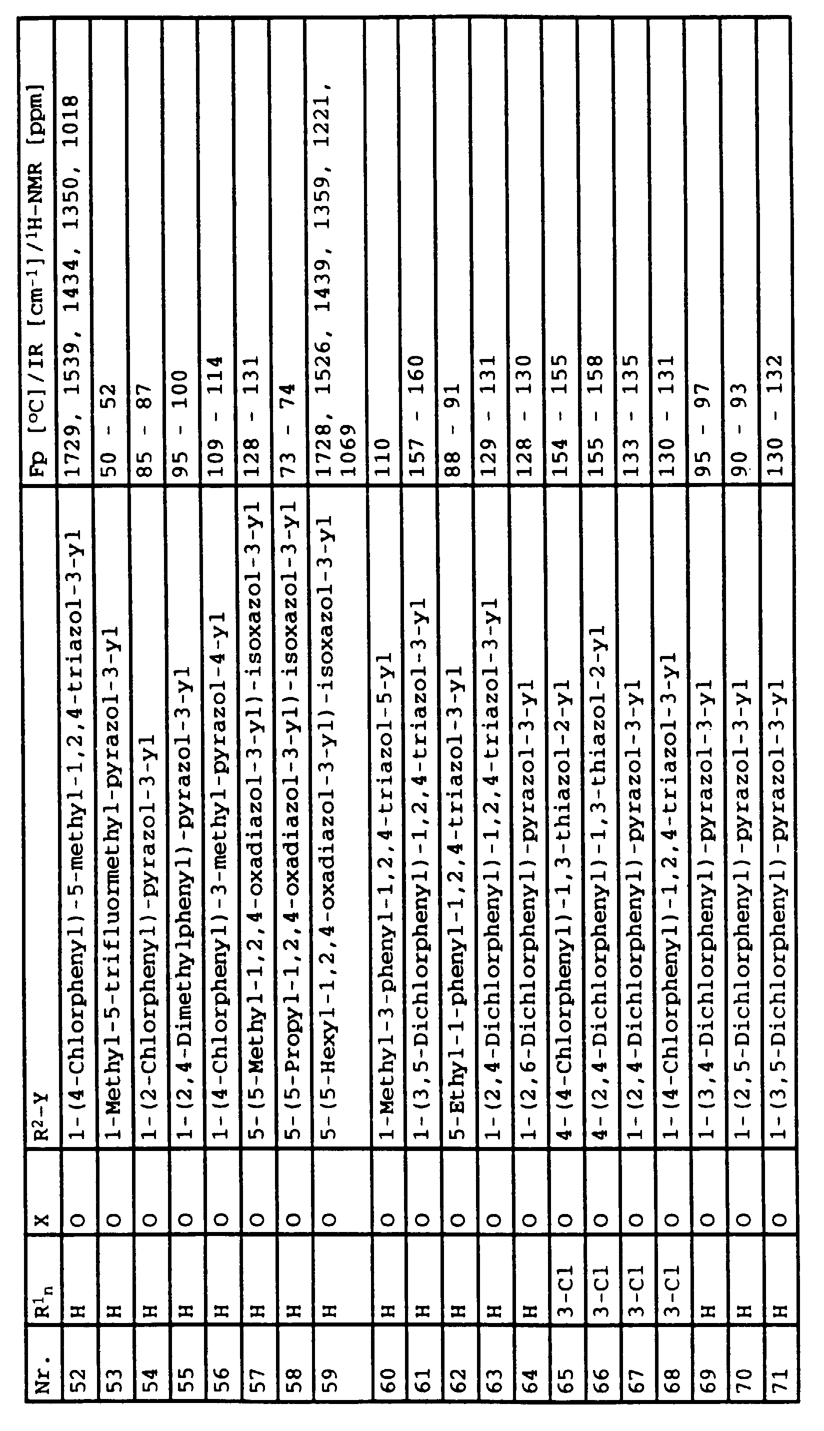

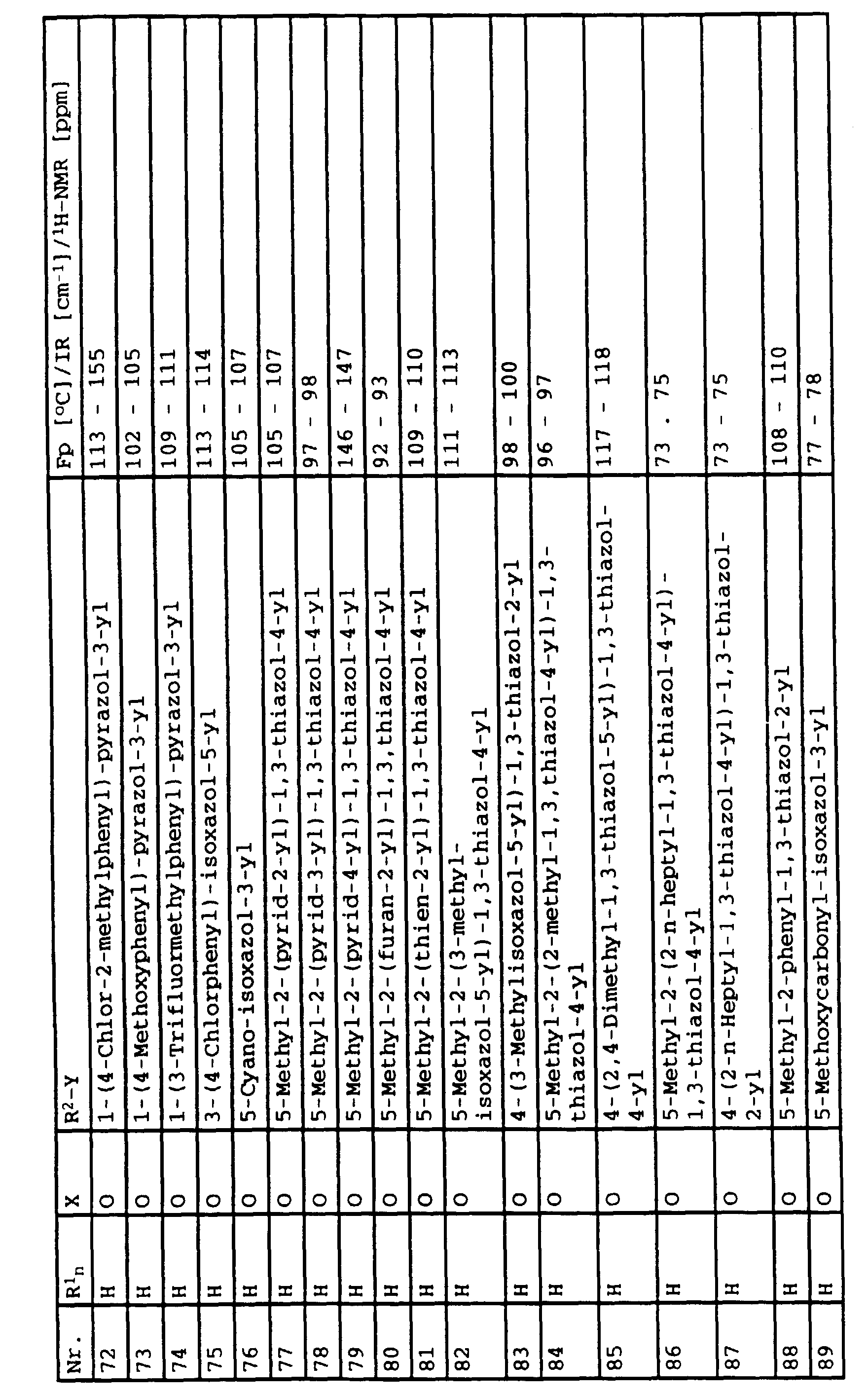

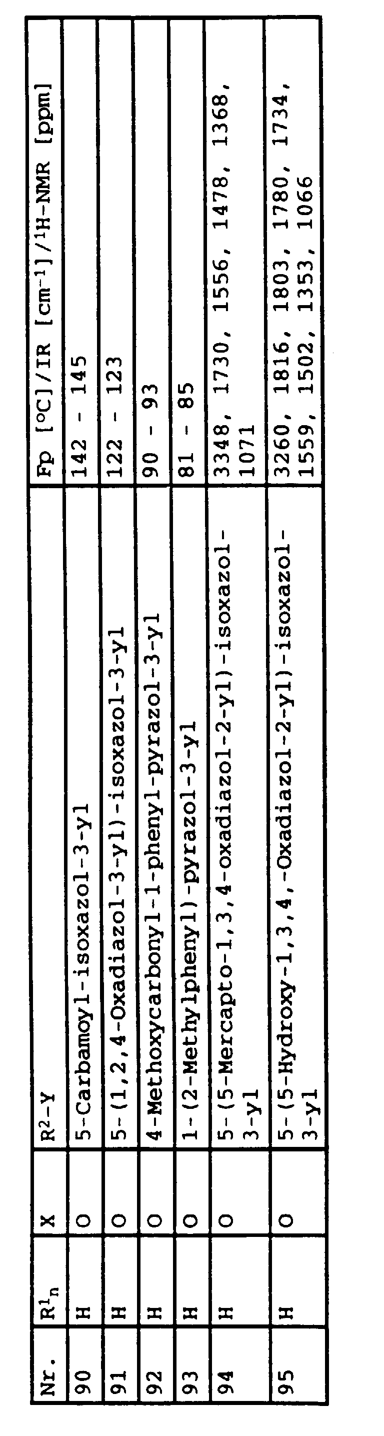

Die Verbindungen I eignen sich als Fungizide.

Die Verbindungen I zeichnen sich durch eine hervorragende Wirksamkeit gegen ein breites Spektrum von pflanzenpatnogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt·werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecylmorpholin bzw. dessen Salze, N-[3-(p-tert.-Butylphenyl)-2-methylpropyl]-cis-2,6-dimethyl- morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H- 1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl- harnstoff, 1-(4-Chlorphenoxy)-3,3-dimethyl-1-(1H-1,2,4-triazol-1-yl)-2- butanon, 1-(4-Chlorphenoxy)-3,3-dimethyl-1-(1H-1,2,4-triazol-1-yl)-2- butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäure- imid, 2-Cyano-[N-(ethylaminocarbonyl)-2-methoximino]-acetamid, l-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Besonders bevorzugt sind als Mischungspartner die folgenden Wirkstoffe:
1-(2-Cyano-2-methoxyiminoacetyl)-3-ethylharnstoff (common name: Cymoxanil, US-A 3,957,847); Mangan-ethylenbis(dithiocarbamat-Zinkkomplex) (common name: Mancozeb, US-A 3,379,610); Manganethylenbis(dithiocarbamat) (common name: Maneb, US-A 2,504,404); Zinkammoniat-ethylenbis(dithiocarbamat) (ehem. common name: Metiram, US-A 3,248,400); Zink-ethylenbis(dithiocarbamat) (common name: Zineb, US-A 2,457,674), (#)-cis-4-[3-(4-tert.-butylphenyl)-2-methylpropyl]-2,6-dimethylmorpholin (common name:
Fenpropimorph, US-A 4,202,894); (RS)-1-[3-(4-tert.-butylphenyl)-2-methylpropyl]piperidin (common name: Fenpropidin, US-A 4,202,894); 2,6- Dimethyl-4-[C₁₁-C₁₄-alkyl]-morpholin (common name: Tridemorph, DE-A 11 64 152);
1-[(2RS,4RS;2RS,4SR)-4-brom-2-(2,4-dichlorphenyl)tetrahydrofuryl]-1H-1,2,4-triazol [common name: Bromuconazol, Proc. Br. Crop Prot. Conf.-Pests Dis., 5-6, 439 (1990)]; 2-(4- Chlorphenyl)-3-cyclopropyl-1-(1H-1,2,4-triazol-1-yl)-butan-2-ol (common name: Cyproconazol, US-A 4,664,696);
(#)-4-Chlor-4-[4-methyl-2-(1H-1,2,4-triazol-1-yl-methyl)-1,3-dioxolan-2-yl]-phenyl-4- chlorphenylether (common name: Difenoconazol, GB-A 2,098,607); (E)-(R,S)-1-(2,4-dichlorphenyl)-4,4-dimethyl-2-(1H-1,2,4-triazol-1-yl)pent-1-en-3-ol (common name: Diniconazol, CAS RN [83657-24-3]);
(Z)-2-(1H-1,2,4-Triazol-1-ylmethyl)-2-(4-fluorphenyl)-3-(2-chlorphenyl)-oxiran (common name: Epoxyconazol, EP-A 196 038); 4-(4-Chlorphenyl)-2-phenyl-2-(1H-1,2,4-triazolylmethyl)-butyronitril (common name: Fenbuconazol (vorgeschlagen), EP-A 251 775);
3-(2,4-dichlorphenyl)-6-fluor-2-(1H-1,2,4-triazol-1-yl)chinazolin-4(3H)-on [common name: Fluquinconazol, Proc. Br. Crop Prot. Conf.-Pests Dis., 5-3, 411 (1992)]; Bis(4-fluorphenyl) (methyl) (1H-1,2,4-triazol-1-ylmethyl)silan [common name: Flusilazol, Proc. Br. Crop Prot. Conf.-Pests Dis., 1, 413 (1984)]; (R,S)-2-(2,4-Dichlorphenyl)-1-(1H-1,2,4-triazol-1-yl)-hexan-2-ol (common name: Hexaconazol, CAS RN [79983-71-4]); (1RS,5RS;1RS,5SR)-5-(4-chlorbenzyl)-2,2-dimenthyl-1-(1H-1,2,4-triazol-1-ylmethyl)cyclopentanol [common name: Metconazol, Proc. Br. Crop Prot. Conf.-Pests Dis., 5-4, 419 (1992)]; N-Propyl-N-[2-(2,4,6-trichlorphenoxy)ethyl]imidazol-1-carboxamid (common name: Prochloraz, US-A 3,991,071); (#)-1-[2-(2,4-dichlorphenyl)-4-propyl-1,3-dioxolan-2-ylmethyl]-1H-1,2,4-triazol (common name: Propiconazol, GB-A 1,522,657); (R,S)-1-(4-chlorphenyl)-4,4-dimethyl-3-(1H-1,2,4-triazol-1-ylmethyl)-pentan-3-ol (common name: Tebuconazol, US-A 4,723,984); (#)-2-(2,4-Dichlorphenyl)-3-(1H- 1,2,4-triazol-yl)-propyl-1,1,2,2-tetrafluorethylether [common name: Tetraconazol, Proc. Br. Crop Prot. Conf.-Pests Dis., 1, 49 (1988)]; (E)-1-[1-[[4-Chlor-2-(trifluormethyl)-phenyl]imino]-2- propoxyethyl]-1H-imidazol (common name: Triflumizol, JP-A 79/119,462); (RS)-2,4'-Difluoralpha-(1H-1,2,4-triazol-1-ylmethyl)benzhydroyl-alkohol (common name: Flutriafol, CAS RN [76674-21-0]; 1-[2-(2,5-dimethylphenoxymethyl phenyl)-1-methoxyimino-essigsäure-N-methylamid (EP-A 477 631); 1-[2-(2-methylphenoxymethyl)phenyl]-1-methoxyimino-essigsäuremethylester (EP-A 253 213); 1-{2-[6-(3-cyanophenoxy)-pyrimidin-4-yloxy]phenyl]-1-methoxyimino-essigsäuremethylester (EP-A 382 375); N-(Trichlormethylthio)cyclohex-4-en-1,2-di-carbimid (common name: Captan, US-A 2,553,770); N-(Trichlormethylthio)phthalimid (common name: Folpet, US-A 2,553,770);
4,6-Dimethyl-2-phenylamino-pyrimidin (common name: Pyrimethanil, DD-A 151 404); 4-Methyl-2-phenylamino-6-propinyl-pyrimidin (common name: Mepanipyrim, EP-A 224 339);
4-Cyclopropyl-6-methyl-2-phenylamino-pyrimidin (EP-A 310 550).

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattalchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstauben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile der Verbindung Nr. 1 aus Tabelle B werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile der Verbindung Nr. 2 aus Tabelle B werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile der Verbindung Nr. 3 aus Tabelle B werden in einer Mischung gelost, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile der Verbindung Nr. 4 aus Tabelle B werden in einer Mischung gelost, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.-%).
V. 80 Gew.-Teile der Verbindung Nr. 5 aus Tabelle B werden mit 3 Gew.-Teilen des Natriumsalzes der Diisobutylnaphthalin-alpha-sulfonsaure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile der Verbindung Nr. 6 aus Tabelle B mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile der Verbindung Nr. 7 aus Tabelle B werden in einer Mischung gelost, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile des Wirkstoffs Nr. 8 aus Tabelle B werden mit 3 Gew.-Teilen des Natriumsalzes der Diisobutylnaphthalin-α-sulfonsäure, 17 Gew.-Teilen des Natriumsalzes einer Ligninsulfonsaure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.

Granulate, z.B. Umhüllungs-, Impragnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Anwendungsbeispiele

### 1. Fungizide Wirksamkeit

Die fungizide Wirkung der Verbindungen der allgemeinen Formel I ließ sich durch folgende Versuche zeigen:

Die Wirkstoffe wurden als 20 %-ige Emulsion in einem Gemisch aus 70 Gew.-% Cyclohexanol, 20 Gew.-% Nekanil^{®} LN (Lutensol^{®} AP6, Netzmittel mit Emulgier- und Dispergierwirkung auf der Basis ethoxylierter Alkylphenole) und 10 Gew.-% Emulphor^{®} EL (Emulan^{®} EL, Emulgator auf der Basis ethoxylierter Fettalkohole) aufbereitet und entsprechend der gewünschten Konzentration mit Wasser verdünnt.

### 1.A Erysiphe graminis var. tritici (Weizenmehltau)

Weizenkeimlinge der Sorte "Frühgold" wurden mit einer wäßrigen Wirkstoffsuspension tropfnaß gespritzt. Nach 24 h wurden die Pflanzen mit Oiden (Sporen) des Pilzes Erysiphe graminis var. tritici (Weizenmehltau) bestäubt. Nach 7 Tagen bei 22°C - 24°C und 75-80% Luftfeuchtigkeit wurde der Pilz-Befall der Blätter beurteilt.

In diesem Test zeigten die mit 63 ppm der Verbindungen 3, 7, 8, 9, 11, 12, 14, 18, 19, 23, 27, 32, 33, 35, 49, 50, 51, 52, 53, 54, 55, 56, 57, 58 und 59 behandelten Pflanzen einen Befall von 15% und weniger, während bei den unbehandelten Pflanzen 65% der Blattflächen befallen waren.

### 1.B Plasmopara viticola (Rebensperonospora)

Topfreben der Sorte "Müller-Thurgau" wurden mit einer wäßrigen Wirkstoffsuspension tropfnaß gespritzt. Nach 8 Tagen im Gewächshaus wurden die Blätter mit einer Zoosporenaufschwemmung von Plasmopara viticola (Rebensperonospora) infiziert. Die Pflanzen wurden zunächst 48 Stunden bei 24°C in einer wasserdampfgesattigten Kammer, dann 5 Tage im Gewächshaus bei 20°C-30°C und anschließend nochmals 16 Stunden bei 24°C in einer wasserdampfgesattigten Kammer aufgestellt, bevor der Befall beurteilt wurde.

In diesem Test zeigten die mit 63 ppm der Verbindungen 3, 7, 8, 9, 10, 11, 12, 14, 15, 16, 17, 18, 21, 22, 23, 24, 25, 26, 27, 33, 34, 35, 36, 37, 38, 39, 40, 41, 42, 23, 44 und 48 behandelten Pflanzen einen Befall von 15% und weniger, während bei den unbehandelten Pflanzen 80% der Blattunterseiten befallen waren.

### 1.C Pyricularia oryzae (Protektive Wirkung)

Blätter von Reiskeimlingen der Sorte "Tai-Nong 67" wurden mit wäßrigen Emulsionen der Wirkstoffe (Gehalt der Trockensubstanz: 80% Wirkstoff und 20% Emulgator) tropfnaß gesprüht und 24 Stunden später mit einer Sporensuspension von Pyricularia oryzae inokuliert. Anschließend wurden die Versuchspflanzen in Klimakammern bei 22-24°C und 95-99% relativer Luftfeuchtigkeit aufbewahrt. Nach 6 Tagen wurde das Ausmaß des Befalls ermittelt.

In diesem Test zeigten die mit 250 ppm der Verbindungen 3, 7, 8, 10, 11, 12, 20, 21, 22, 23, 24, 25, 26, 27, 32, 33, 38, 48, 49, 50, 51, 52, 53, 54 und 55 behandelten Pflanzen einen Befall von 15% und weniger, während bei den unbehandelten Pflanzen 70% der Blattflächen befallen waren.

### 2. Wirkung gegen tierische Schädlinge

Die insektizide Wirkung der Verbindungen der allgemeinen Formel I ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil^{®} LN (Lutensol^{®} AP6, Netzmittel mit Emulgier- und Dispergierwirkung auf der Basis ethoxylierter Alkylphenole) und 10 Gew.-% Emulphor^{®} EL (Emulan^{®} EL, Emulgator auf der Basis ethoxylierter Fettalkohole)

aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

### 2.A Aphis fabae (Schwarze Laus), Kontaktwirkung

Stark befallene Buschbohnen (Vicia faba) wurden mit der waßrigen Wirkstoffaufbereitung behandelt.

Nach 24 h wurde die Mortalitätsrate bestimmt.

In diesem Test zeigten die Verbindungen 9, 18, 24, 37, 42, 43 und 44 Wirkschwellen von mindestens 400 ppm.

### 2.B Nephotettix cincticeps (Grüne Reiszikade), Kontaktwirkung

Rundfilter wurden mit der waßrigen Wirkstoffaufbereitung behandelt und anschließend mit 5 adulten Zikaden belegt.

Nach 24 h wurde die Mortalität beurteilt.

In diesem Test zeigten die Verbindungen 3, 16, 24, 32, 33, 36, 40, 42, 46, 50, 51, 55, 56, 58, 61 und 63 Wirkschwellen von mindestens 0,4 mg.

### 2.C Prodenia litura (Ägypt. Baumwollwurm), Zuchtversuch

Fünf Raupen des Entwicklungsstadiums L3 (10 - 12 mm), die keine feststellbare Schädigung im Kontaktversuch erlitten hatten, wurden auf Standardnährboden (3,1 l Wasser, 80 g Agar, 137 g Bierhefe, 515 g Maismehl, 130 g Weizenkeime sowie übliche Zusatzstoffe und Vitamine (20 g Wessonsalz, 5 g Nipagin, 5 g Sorbin, 10 g Zellulose, 18 g Ascorbinsäure, 1 g Lutavit^{®} blend (Vitamin), 5 ml alkoholische Biotin-Lösung)) aufgebracht, der zuvor mit der wäßrigen Wirkstoffaufbereitung benetzt worden war. Die Beobachtung erstreckte sich bis zum Schlüpfen der Falter in einem Kontrollversuch ohne Wirkstoff.

In diesem Test zeigten die Verbindungen 9, 24, 26, 40, 42, 43, 44, 51, 52 und 63 Wirkschwellen von mindestens 0,4 mg.

### 2.D Tetranychus telarius (Rote Spinne), Kontaktwirkung

Stark befallene getopfte Buschbohnen, die das zweite Folgeblattpaar zeigten, wurden mit wäßrigen Wirkstoffaufbereitung behandelt.

Nach 5 Tagen im Gewächshaus wurde der Bekämpfungserfolg mittels Binokular bestimmt.

In diesem Test zeigten die Verbindungen 7, 8, 9, 18, 23, 24, 37, 40, 42, 43, 44, 49, 50, 51, 52, 61 und 63 Wirkschwellen von mindestens 400 ppm.

## Patentansprüche

1. Verbindungen der allgemeinen Formel I, 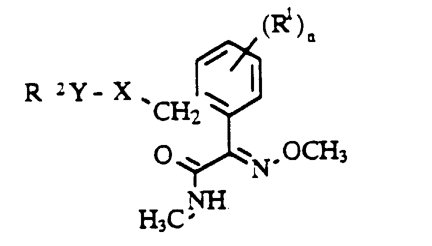 in der der Index und die Substituenten die folgende Bedeutung haben:
n 0, 1, 2, 3 oder 4, wobei die Reste R¹ verschieden sein können, wenn n größer als 1 ist;
X O oder S;
Y 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,2,4-Triazol-5-yl, 1, 3, 4-oxadiazol-2-yl, 1,2,3-oxadiazol-4-yl, 1, 2,3-oxadiazol-5-yl, 1,3,4-Thiadiazol-2-yl, 1,2,3-Thiadiazol-5-yl und 1,2,3-Triazol-4-yl, welche neben R² noch einen oder zwei Reste aus der Gruppe Cl, CH₃, CF₃ oder OCH₃ tragen können;
R¹ Nitro, Cyano, Halogen, C₁-C₄-Alkyl, C₁-C₄-Halogenalkyl, C₁-C₄-Alkoxy, C₁-C₄-Halogenalkoxy, C₁-C₄-Alkylthio;
Phenyl oder Phenoxy, wobei die aromatischen Ringe ein bis fünf Halogenatome oder ein bis drei der folgenden Reste tragen können: Halogen, C₁-C₄-Alkyl, C₁-C₄-Halogenalkyl, C₁-C₄-Alkoxy, C₁-C₄-Halogenalkoxy und C₁-C₄-Alkylthio;
oder, sofern n größer als 1 ist, eine an zwei benachbarte C-Atome des Phenylrests gebundene 1,3-Butadien-1,4-diylgruppe, welche ihrerseits ein bis vier Halogenatome oder ein oder zwei der folgenden Reste tragen kann: Halogen, C₁-C₄-Alkyl, C₁-C₄-Halogenalkyl, C₁-C₄-Alkoxy, C₁-C₄-Halogenalkoxy und C₁-C₄-Alkylthio;
R² Wasserstoff,
C₁-C₆-Alkyl, C₂-C₆-Alkenyl oder C₃-C₆-Alkinyl, wobei diese Gruppen partiell oder vollständig halogeniert sein können und/oder ein bis drei der folgenden Substituenten tragen können:
- Nitro, Cyano, Thiocyanato, C₁-C₄-Alkoxy, C₁-C₄-Halogenalkoxy, C₁-C₄-Alkylthio, C₁-C₄-Alkylamino, Di-C₁-C₄-Alkylamino, C₃-C₆-Alkenyloxy, C₃-C₆-Alkinyloxy, C₁-C₆-Alkylcarbonyl, C₁-C₆-Alkoxyimino, C₃-C₆-Alkenyloxyimino, C₃-C₆-Alkinyloxyimino, C₁-C₆-Alkoxycarbonyl, C₁-C₆-Alkylthiocarbonyl, C₁-C₆-Alkylaminocarbonyl, Di-C₁-C₆-alkylaminocarbonyl, C₁-C₆-Alkylcarbonyloxy, C₁-C₆-Alkylcarbonylamino, C₃-C₇-Cycloalkyl, C₃-C₇-Cycloalkoxy, C₃-C₇-Cycloalkylthio, C₃-C₇-Cycloalkylamino, C₅-C₇-Cycloalkenyl,
- 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2, 3-Dihydropyrazol-4-yl, 2, 3-Dihydropyrazol-5-yl, 3,4-Dihydropyrazol-1-yl, 3,4-Dihydropyrazol-3-yl, 3, 4-Dihydropyrazol-4-yl, 3, 4-Dihydropyrazol-5-yl, 4, 5-Dihydropyrazol-1-yl, 4,5-Dihydropyrazol-3-yl, 4,5-Dihydropyrazol-4-yl, 4, 5-Dihydropyrazol-5-yl, 2,3-Dihydrooxazol-2-yl, 2, 3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 2-Piperidinyl, 3-Piperidinyl, 4-Piperidinyl, 1,3-Dioxan-5-yl, 2-Tetrahydropyranyl, 4-Tetrahydropyranyl, 3-Tetrahydropyridazinyl, 4-Tetrahydropyridazinyl, 2-Terahydropyrimidinyl, 4-Tetrahydropyrimidinyl, 5-Tetrahydropyrimidinyl, 2-Tetrahydropyrazinyl, 1,3,5-Tetrahydrotriazin-2-yl und 1,2,4-Tetrahydrotriazin-3-yl,
- ggf. subst. Phenyl oder Naphtyl, oder
- ein ggf, subst. 5-Ring Heteroaromat, welcher ein bis drei Stickstoffatome und/oder ein Sauerstoff- oder Schwefelatom enthält, und an den ein Benzolring anneliert sein kann, oder
- ein ggf. subst. 6-Ring Heteroaromat, welcher ein bis vier Stickstoffatome enthält, und an den ein Benzolring anneliert sein kann,
ggf. subst. Cyclopropyl, Cyclopentyl, Cyclohexyl, Cyclopent-2-enyl, Cyclohex-2-enyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3, 4-Dihydrooxazol-5-yl, 2-Piperidinyl, 3-Piperidinyl, 4-Piperidinyl, 1,3-Dioxan-5-yl, 2-Tetrahydropyranyl, 4-Tetrahydropyranyl, 3-Tetrahydropyridazinyl, 4-Tetrahydropyridazinyl, 2-Terahydropyrimidinyl, 4-Tetrahydropyrimidinyl, 5-Tetrahydropyrimidinyl, 2-Tetrahydropyrazinyl, 1,3,5-Tetrahydrotriazin-2-yl und 1,2,4-Tetrahydrotriazin-3-yl,
oder
ggf. subst. Phenyl oder Naphtyl, oder
ein ggf. subst. 5-Ring Heteroaromat, welcher ein bis drei Stickstoffatome und/oder ein Sauerstoff- oder Schwefelatom enthält, oder ein ggf. subst. 6-Ring Heteroaromat, welcher ein bis vier Stickstoffatome enthält,
wobei die mit *"ggf. subst."* bezeichneten, bei R² genannten gesättigten oder ein- oder zweifach ungesättigten alicyclischen oder heterocyclischen Systeme ihrerseits partiell oder vollständig halogeniert sein können und/oder ein bis drei der folgenden Substituenten tragen können:Nitro, Cyano, C₁-C₆-Alkyl, C₁-C₄-Halogenalkyl, C₁-C₄-Alkoxy, C₁-C₄-Alkylthio, Di-C₁-C₄-Alkylamino, C₂-C₆-Alkenyl, C₂-C₆-Alkinyl, C₁-C₆-Alkoxycarbonyl, C₁-C₆-Alkylaminocarbonyl, Di-C₁-C₆-alkylaminocarbonyl, C₁-C₆-Alkylcarbonyloxy, C₁-C₆-Alkylcarbonylamino, C₃-C₇-Cycloalkyl und Phenyl,
und wobei die mit *"ggf. subst."* bezeichneten, bei R² genannten ein- oder zweikernigen aromatischen oder heteroaromatischen Systeme ihrerseits partiell oder vollständig halogeniert sein können und/oder ein bis drei der folgenden Substituenten tragen können:
- Nitro, Cyano, Thiocyanato, C₁-C₆-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₂-C₆-Alkenyl, C₃-C₆-Alkenyloxy, C₂-C₆-Alkinyl, C₃-C₆-Alkinyloxy, C₁-C₆-Alkylcarbonyl, C₁-C₆-Alkoxycarbonyl, C₁-C₆-Alkylthiocarbonyl, C₁-C₆-Alkylaminocarbonyl, Di-C₁-C₆-alkylaminocarbonyl, C₁-C₆-Alkylcarbonyloxy, C₁-C₆-Alkylcarbonylamino, C₃-C₇-Cycloalkyl, C₃-C₇-Cycloalkoxy, C₃-C₇-Cycloalkylthio, C₃-C₇-Cycloalkylamino, C₅-C₇-Cycloalkenyl,
- Phenyl, oder
ein ggf. subst. 5-Ring Heteroaromat, welcher ein bis drei Stickstoffatome und/oder ein Sauerstoff- oder Schwefelatom enthält, oder ein ggf. subst. 6-Ring Heteroaromat, welcher ein bis vier Stickstoffatome enthält, wobei diese Aryl- oder Heteroarylringe ein bis drei der folgenden Gruppen tragen können: Fluor, Chlor, Cyano, Methyl, Methoxy, Trifluormethyl oder Trifluormethoxy.

2. Verfahren zur Herstellung von Verbindungen der allgemeinen Formel I gemäß Anspruch 1, dadurch gekennzeichnet, daß man ein 2-Methoxyiminophenylessigsäurederivat der Formel IA 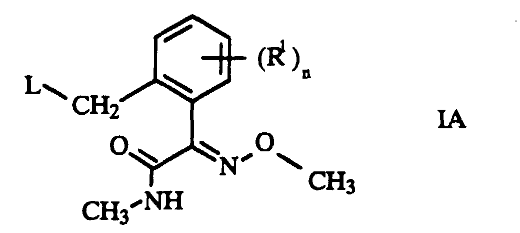 in der R¹ die in Anspruch 1 genannten Bedeutung hat und L eine Abgangsgruppe bedeutet, mit einem Hydroxy- oder Mercapto-Fünfringheterocyclus der allgemeinen Formel III
R² - Y - XH III
in der R², Y und X die im Anspruch 1 genannten Bedeutungen haben, im Gegenwart einer Base umsetzt.

3. Verfahren zur Herstellung von Verbindungen der allgemeinen Formel I gemäß Anspruch 1, dadurch gekennzeichnet, daß man einen 2-Methoxyiminophenylessigsäurederivat der Formel II 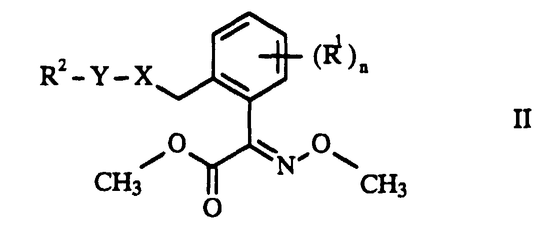 in der R¹, R², Y, X und n die im Anspruch 1 genannten Bedeutungen haben, mit Methylamin umsetzt.

4. Mittel zur Bekämpfung von Schadpilzen, enthaltend eine fungizide Menge einer Verbindung der Formel I gemäß Anspruch 1 und einem inerten Zusatzstoff.

5. Schädlingsbekämpfungsmittel, enthaltend eine wirksame Menge einer Verbindung der Formel I gemäß Anspruch 1 und einem inerten Zusatzstoff.

6. Verfahren zur Bekämpfung von Schadpilzen, dadurch gekennzeichnet, daß man die Schadpilze, ihren Lebensraum oder die von Schadpilzen freizuhaltenden Pflanzen oder Materialien mit einer fungizid wirksamen Menge einer Verbindung der Formel I gemäß Anspruch 1 behandelt.

7. Verfahren zur Bekämpfung von Schädlingen, dadurch gekennzeichnet, daß man die Schädlinge, ihren Lebensraum oder die von Schädlingen freizuhaltenden Pflanzen oder Materialien mit einer wirksamen Menge einer Verbindung der Formel I gemäß Anspruch 1 behandelt.

8. Verwendung der Verbindungen I gemäß Anspruch 1 zur Bekämpfung von Schadpilzen.

9. Verwendung der Verbindungen I gemäß Anspruch 1 zur Bekämpfung von Schädlingen aus den Klassen der Insekten, Spinnentiere und Nematoden.

## Claims

1. A compound of the general formula I 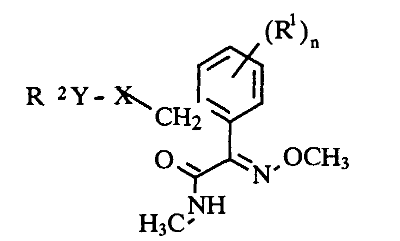 where the index and the substituents have the following meanings:
n is 0, 1, 2, 3 or 4 and the radicals R¹ may be different when n is greater than 1;
X is O or S;
Y is 2-furyl, 3-furyl, 2-thienyl, 3-thienyl, 1-pyrrolyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 1-pyrazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 1-imidazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,2,4-triazol-5-yl, 1,3,4-oxadiazol-2-yl, 1,2,3-oxadiazol-4-yl, 1,2,3-oxadiazol-5-yl, 1,3,4-thiadiazol-2-yl, 1,2,3-thiadiazol-5-yl and 1,2,3-triazol-4-yl, each of which, in addition to R², may furthermore carry one or two radicals selected from the group consisting of Cl, CH₃, CF₃ and OCH₃;
R¹ is nitro, cyano, halogen, C₁-C₄-alkyl, C₁-C₄-haloalkyl, C₁-C₄-alkoxy, C₁-C₄-haloalkoxy or C₁-C₄-alkylthio;
phenyl or phenoxy, where the aromatic rings may carry one to five halogen atoms or one to three of the following radicals: halogen, C₁-C₄-alkyl, C₁-C₄-haloalkyl, C₁-C₄-alkoxy, C₁-C₄-haloalkoxy and C₁-C₄-alkylthio;
or, if n is greater than 1, a 1,3-butadiene-1,4-diyl group which is bonded to two adjacent C atoms of the phenyl radical and which for its part can carry one to four halogen atoms or one or two of the following radicals: halogen, C₁-C₄-alkyl, C₁-C₄-haloalkyl, C₁-C₄-alkoxy, C₁-C₄-haloalkoxy and C₁-C₄-alkylthio;
R² is hydrogen,
C₁-C₆-alkyl, C₂-C₆-alkenyl or C₃-C₆-alkynyl, where these groups may be partially or completely halogenated and/or may carry one to three of the following substituents:
- nitro, cyano, thiocyanato, C₁-C₄-alkoxy, C₁-C₄-haloalkoxy, C₁-C₄-alkylthio, C₁-C₄-alkylamino, di-C₁-C₄-alkylamino, C₃-C₆-alkenyloxy, C₃-C₆-alkynyloxy, C₁-C₆-alkylcarbonyl, C₁-C₆-alkoxyimino, C₃-C₆-alkenyloxyimino, C₃-C₆-alkynyloxyimino, C₁-C₆-alkoxycarbonyl, C₁-C₆-alkylthiocarbonyl, C₁-C₆-alkylaminocarbonyl, di-C₁-C₆-alkylaminocarbonyl, C₁-C₆-alkylcarbonyloxy, C₁-C₆-alkylcarbonylamino, C₃-C₇-cycloalkyl, C₃-C₇-cycloalkoxy, C₃-C₇-cycloalkylthio, C₃-C₇-cycloalkylamino or C₅-C₇-cycloalkenyl,
- 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2,3-pyrrolin-2-yl, 2,3-pyrrolin-3-yl, 2,4-pyrrolin-2-yl, 2,4-pyrrolin-3-yl, 2,3-isoxazolin-3-yl, 3,4-isoxazolin-3-yl, 4,5-isoxazolin-3-yl, 2,3-isoxazolin-4-yl, 3,4-isoxazolin-4-yl, 4,5-isoxazolin-4-yl, 2,3-isoxazolin-5-yl, 3,4-isoxazolin-5-yl, 4,5-isoxazolin-5-yl, 2,3-isothiazolin-3-yl, 3,4-isothiazolin-3-yl, 4,5-isothiazolin-3-yl, 2,3-isothiazolin-4-yl, 3,4-isothiazolin-4-yl, 4,5-isothiazolin-4-yl, 2,3-isothiazolin-5-yl, 3,4-isothiazolin-5-yl, 4,5-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 2-piperidinyl, 3-piperidinyl, 4-piperidinyl, 1,3-dioxan-5-yl, 2-tetrahydropyranyl, 4-tetrahydropyranyl, 3-tetrahydropyridazinyl, 4-tetrahydropyridazinyl, 2-tetrahydropyrimidinyl, 4-tetrahydropyrimidinyl, 5-tetrahydropyrimidinyl, 2-tetrahydropyrazinyl, 1,3,5-tetrahydrotriazin-2-yl and 1,2,4-tetrahydrotriazin-3-yl,
- unsubstituted or substituted phenyl or naphthyl or
- an unsubstituted or substituted heteroaromatic which has a 5-membered ring and contains one to three nitrogen atoms and/or one oxygen or sulfur atom and to which a benzene ring may be fused, or
- an unsubstituted or substituted heteroaromatic which has a 6-membered ring and contains one to four nitrogen atoms and to which a benzene ring may be fused,
unsubstituted or substituted cyclopropyl, cyclopentyl, cyclohexyl, cyclopent-2-enyl, cyclohex-2-enyl, 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2,3-pyrrolin-2-yl, 2,3-pyrrolin-3-yl, 2,4-pyrrolin-2-yl, 2,4-pyrrolin-3-yl, 2,3-isoxazolin-3-yl, 3,4-isoxazolin-3-yl, 4,5-isoxazolin-3-yl, 2,3-isoxazolin-4-yl, 3,4-isoxazolin-4-yl, 4,5-isoxazolin-4-yl, 2,3-isoxazolin-5-yl, 3,4-isoxazolin-5-yl, 4,5-isoxazolin-5-yl, 2,3-isothiazolin-3-yl, 3,4-isothiazolin-3-yl, 4,5-isothiazolin-3-yl, 2,3-isothiazolin-4-yl, 3,4-isothiazolin-4-yl, 4,5-isothiazolin-4-yl, 2,3-isothiazolin-5-yl, 3,4-isothiazolin-5-yl, 4,5-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 2-piperidinyl, 3-piperidinyl, 4-piperidinyl, 1,3-dioxan-5-yl, 2-tetrahydropyranyl, 4-tetrahydropyranyl, 3-tetrahydropyridazinyl, 4-tetrahydropyridazinyl, 2-tetrahydropyrimidinyl, 4-tetrahydropyrimidinyl, 5-tetrahydropyrimidinyl, 2-tetrahydropyrazinyl, 1,3,5-tetrahydrotriazin-2-yl and 1,2,4-tetrahydrotriazin-3-yl,
or
unsubstituted or substituted phenyl or naphthyl, or
an unsubstituted or substituted heteroaromatic which has a 5-membered ring and contains one to three nitrogen atoms and/or one oxygen or sulfur atom, or
an unsubstituted or substituted heteroaromatic which has a 6-membered ring and contains one to four nitrogen atoms,
where the saturated or monounsaturated or diunsaturated alicyclic or heterocyclic systems which are defined as unsubstituted or substituted and mentioned in the case of R² may in turn be partially or completely halogenated and/or may carry one to three of the following substituents: nitro, cyano, C₁-C₆-alkyl, C₁-C₄-haloalkyl, C₁-C₄-alkoxy, C₁-C₄-alkylthio, di-C₁-C₄-alkylamino, C₂-C₆-alkenyl, C₂-C₆-alkynyl, C₁-C₆-alkoxycarbonyl, C₁-C₆-alkylaminocarbonyl, di-C₁-C₆-alkylaminocarbonyl, C₁-C₆-alkylcarbonyloxy, C₁-C₆-alkylcarbonylamino, C₃-C₇-cycloalkyl and phenyl,
and where the mononuclear or dinuclear aromatic or heteroaromatic systems which are defined as unsubstituted or substituted and mentioned in the case of R² may in turn be partially or completely halogenated and/or may carry one to three of the following substituents:
- nitro, cyano, thiocyanato, C₁-C₆-alkyl, C₁-C₄-haloalkyl, C₁-C₄-alkoxy, C₁-C₄-haloalkoxy, C₁-C₄-alkylthio, C₁-C₄-alkylamino, di-C₁-C₄-alkylamino, C₂-C₆-alkenyl, C₃-C₆-alkenyloxy, C₂-C₆-alkynyl, C₃-C₆-alkynyloxy, C₁-C₆-alkylcarbonyl, C₁-C₆-alkoxycarbonyl, C₁-C₆-alkylthiocarbonyl, C₁-C₆-alkylaminocarbonyl, di-C₁-C₆-alkylaminocarbonyl, C₁-C₆-alkylcarbonyloxy, C₁-C₆-alkylcarbonylamino, C₃-C₇-cycloalkyl, C₃-C₇-cycloalkoxy, C₃-C₇-cycloalkylthio, C₃-C₇-cycloalkylamino or C₅-C₇-cycloalkenyl,
- phenyl or
an unsubstituted or substituted heteroaromatic which has a 5-membered ring and contains one to three nitrogen atoms and/or one oxygen or sulfur atom, or an unsubstituted or substituted heteroaromatic which has a 6-membered ring and contains one to four nitrogen atoms, where these aryl or hetaryl rings may carry one to three of the following groups: fluorine, chlorine, cyano, methyl, methoxy, trifluoromethyl or trifluoromethoxy.

2. A process for preparing compounds of the general formula I as claimed in claim 1, which comprises reacting a 2-methoxyiminophenylacetic acid derivative of the formula IA 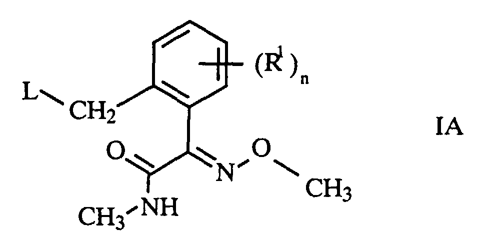 where R¹ has the meaning mentioned in claim 1 and L is a leaving group, with a hydroxy- or mercapto-substituted five-membered ring heterocycle of the general formula III
R² - Y - XH III
where R², Y and X have the meanings mentioned in claim 1, in the presence of a base.

3. A process for preparing compounds of the general formula I as claimed in claim 1, which comprises reacting a 2-methoxyiminophenylacetic acid derivative of the formula II 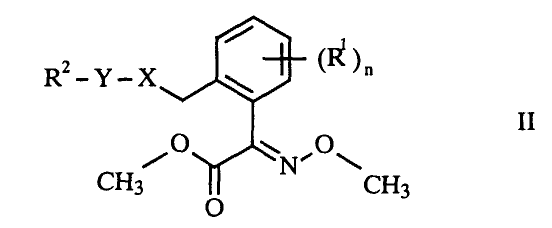 where R¹, R², Y, X and n have the meanings mentioned in claim 1, with methylamine.

4. A composition for controlling harmful fungi, containing a fungicidal amount of a compound of the formula I as claimed in claim 1 and an inert additive.

5. A pesticide containing an effective amount of a compound of the formula I as claimed in claim 1 and an inert additive.

6. A process for controlling harmful fungi, which comprises treating the harmful fungi, their environment or the plants or materials to be kept free of harmful fungi with a fungicidally effective amount of a compound of the formula I as claimed in claim 1.

7. A process for controlling pests, which comprises treating the pests, their environment or the plants or materials to kept free of pests with an effective amount of a compound of the formula I as claimed in claim 1.

8. The use of the compounds I as claimed in claim 1 for controlling harmful fungi.

9. The use of the compounds I as claimed in claim 1 for controlling pests from the classes of insects, arachnida and nematodes.

## Revendications

1. Composés de formule générale I 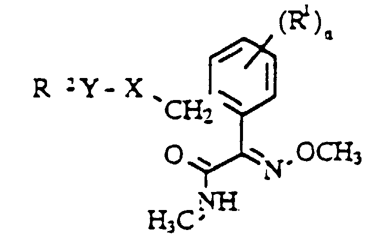 dans laquelle l'indice et les symboles ont les significations suivantes :
n est égal à 0, 1, 2, 3, ou 4, les substituants R¹ pouvant être différents lorsque n est supérieur à 1 ;
X représente O ou S ;
Y représente un groupe 2-furyle, 3-furyle, 2-thiényle, 3-thiényle, 1-pyrrolyle, 2-pyrrolyle, 3-pyrrolyle, 3-isoxazolyle, 4-isoxazolyle, 5-isoxazolyle, 3-isothiazolyle, 4-isothiazolyle, 5-isothiazolyle, 1-pyrazolyle, 3-pyrazolyle, 4-pyrazolyle, 5-pyrazolyle, 2-oxazolyle, 4-oxazolyle, 5-oxazolyle, 2-thiazolyle, 4-thiazolyle, 5-thiazolyle, 1-imidazolyle, 2-imidazolyle, 4-imidazolyle, 1,2,4-oxadiazol-3-yle, 1,2,4-oxadiazol-5-yle, 1,2,4-thiadiazol-3-yle, 1,2,4-thiadiazol-5-yle, 1,2,4-triazol-3-yle, 1,2,4-triazol-5-yle, 1,3,4-oxadiazol-2-yle, 1,2,3-oxadiazol-4-yle, 1,2,3-oxadiazol-5-yle, 1,3,4-thiadiazol-2-yle, 1,2,3-thiadiazol-5-yle et 1,2,3-triazol-4-yle, qui, en plus de R², peut encore porter un ou deux substituants choisis parmi Cl, CH₃, CF₃ ou OCH₃ ;
R¹ représente un groupe nitro, cyano, un halogène, un groupe alkyle en C1-C4, halogénoalkyle en C1-C4, alcoxy en C1-C4, halogénoalcoxy en C1-C4, alkylthio en C1-C4 ;
un groupe phényle ou phénoxy, les noyaux aromatiques pouvant porter un à cinq atomes d'halogènes ou un à trois des substituants suivants :
halogéno, alkyle en C1-C4, halogénoalkyle en C1-C4, alcoxy en C1-C4, halogénoalcoxy en C1-C4 et alkylthio en C1-C4 ;
ou bien, lorsque n est supérieur à 1, un groupe 1,3-butandiène-1,4-diyle relié à deux atomes de carbone voisins du groupe phényle et qui peut lui-même porter un à quatre atomes d'halogènes ou un ou deux des substituants suivants : halogéno, alkyle en C1-C4, halogénoalkyle en C1-C4, alcoxy en C1-C4, halogénoalcoxy en C1-C4 et alkylthio en C1-C4;
R² représente l'hydrogène,
un groupe alkyle en C1-C6, alcényle en C2-C6 ou alcynyle en C3-C6, ces groupes pouvant être partiellement ou totalement halogénés et/ou porter un à trois des substituants suivants :
- nitro, cyano, thiocyanato, alcoxy en C1-C4, halogénoalcoxy en C1-C4, alkylthio en C1-C4, alkylamino en C1-C4, di-(alkyle en C1-C4)amino, alcényloxy en C3-C6, alcynyloxy en C3-C6, (alkyle en C1-C6)carbonyle, alcoxyimino en C1-C6, alcényloxyimino en C3-C6, alcynyloxyimino en C3-C6, (alcoxy en C1-C6)carbonyle, (alkylthio en C1-C6)carbonyle, (alkyle en C1-C6)aminocarbonyle, di-(alkyle en C1-C6)aminocarbonyle, (alkyle en C1-C6)carbonyloxy, (alkyle en C1-C6)carbonylamino, cycloalkyle en C3-C7, cycloalcoxy en C3-C7, cycloalkylthio en C3-C7, cycloalkylamino en C3-C7, cycloalcényle en C5-C7,
- 2-tétrahydrofuranyle, 3-tétrahydrofuranyle, 2-tétrahydrothiényle, 3-tétrahydrothiényle, 2-pyrrolidinyle, 3-pyrrolidinyle, 3-isoxazolidinyle, 4-isoxazolidinyle, 5-isoxazolidinyle, 3-isothiazolidinyle, 4-isothiaolidinyle, 5-isothiazolidinyle, 3-pyrazolidinyle, 4-pyrazolidinyle, 5-pyrazolidinyle, 2-oxazolidinyle, 4-oxazolidinyle, 5-oxazolidinyle, 2-thiazolidinyle, 4-thiazolidinyle, 5-thiazolidinyle, 2-imidazolidinyle, 4-imidazolidinyle, 1,2,4-oxadiazolidin-3-yle, 1,2,4-oxadiazolidin-5-yle, 1,2,4-thiadiazolidin-3-yle, 1,2,4-thiadiazolidin-5-yle, 1,2,4-triazolidin-3-yle, 1,3,4-oxadiazolidin-2-yle, 1,3,4-thiadiazolidin-2-yle, 1,3,4-triazolidin-2-yle, 2,3-dihydrofur-2-yle, 2,3-dihydrofur-3-yle, 2,4-dihydrofur-2-yle, 2,4-dihydrofur-3-yle, 2,3-dihydrothién-2-yle, 2,3-dihydrothién-3-yle, 2,4-dihydrothién-2-yle, 2,4-dihydrothién-3-yle, 2,3-pyrrolin-2-yle, 2,3-pyrrolin-3-yle, 2,4-pyrrolin-2-yle, 2,4-pyrrolin-3-yle, 2,3-isoxazolin-3-yle, 3,4-isoxazolin-3-yle, 4,5-isoxazolin-3-yle, 2,3-isoxazolin-4-yle, 3,4-isoxazolin-4-yle, 4,5-isoxazolin-4-yle, 2,3-isoxazolin-5-yle, 3,4-isoxazolin-5-yle, 4,5-isoxazolin-5-yle, 2,3-isothiazolin-3-yle, 3,4-isothiazolin-3-yle, 4,5-isothiazolin-3-yle, 2,3-isothiazolin-4-yle, 3,4-isothiazolin-4-yle, 4,5-isothiazolin-4-yle, 2,3-isothiazolin-5-yle, 3,4-isothiazolin-5-yle, 4,5-isothiazolin-5-yle, 2,3-dihydropyrazol-1-yle, 2,3-dihydropyrazol-2-yle, 2,3-dihydropyrazol-3-yle, 2,3-dihydropyrazol-4-yle, 2,3-dihydropyrazol-5-yle, 3,4-dihydropyrazol-1-yle, 3,4-dihydropyrazol-3-yle, 3,4-dihydropyrazol-4-yle, 3 ,4-dihydropyrazol-5-yle, 4,5-dihydropyrazol-1-yle, 4,5-dihydropyrazol-3-yle, 4,5-dihydropyrazol-4-yle, 4,5-dihydropyrazol-5-yle, 2,3-dihydrooxazol-2-yle, 2,3-dihydrooxazol-3-yle, 2,3-dihydrooxazol-4-yle, 2,3-dihydrooxazol-5-yle, 3,4-dihydrooxazol-2-yle, 3,4-dihydrooxazol-3-yle, 3,4-dihydrooxazol-4-yle, 3,4-dihydrooxazol-5-yle, 2-pipéridinyle, 3-pipéridinyle, 4-pipéridinyle, 1,3-dioxan-5-yle, 2-tétrahydropyranyl, 4-tétrahydropyranyle, 3-tétrahydropyridazinyle, 4-tétrahydropyridazinyle, 2-tétrahydroyrimidinyle, 4-tétrahydropyrimidinyle, 5-tétrahydropyrimidinyle, 2-tétrahydropyrazinyle, 1,3,5-tétrahydrotriazin-2-yle, et 1,2,4-tétrahydrotriazin-3-yle,
- un groupe phényle ou naphtyle éventuellement substitué, ou bien
- un groupe hétéroaromatique à cinq chaînons, éventuellement substitué, qui contient un à trois atomes d'azote et/ou un atome d'oxygène ou de soufre, et qui peut être condensé sur un noyau benzénique, ou bien
- un groupe hétéroaromatique à six chaînons éventuellement substitué qui contient un à quatre atomes d'azote et qui peut être condensé sur un noyau benzénique,
un groupe cyclopropyle, cyclopentyle, cyclohexyle, cyclopent-2-ényle, cyclohex-2-ényle, 2-tétrahydrofuranyle, 3-tétrahydrofuranyle, 2-tétrahydrothiényle, 3-tétrahydrothiényle, 2-pyrrolidinyle, 3-pyrrolidinyle, 3-isoxazolidinyle, 4-isoxazolidinyle, 5-isoxazolidinyle, 3-isothiazolidinyle, 4-isothiazolidinyle, 5-isothiazolidinyle, 3-pyrazolidinyle, 4-pyrazolidinyle, 5-pyrazolidinyle, 2-oxazolidinyle, 4-oxazolidinyle, 5-oxazolidinyle, 2-thiazolidinyle, 4-thiazolidinyle, 5-thiazolidinyle, 2-imidazolidinyle, 4-imidazolidinyle, 1,2,4-oxadiazolidin-3-yle, 1,2,4-oxadiazolidin-5-yle, 1,2,4-thiadiazolidin-3-yle, 1,2,4-thiadiazolidin-5-yle, 1,2,4-triazolidin-3-yle, 1,3,4-oxadiazolidin-2-yle, 1,3,4-thiadiazolidin-2-yle, 1,3,4-triazolidin-2-yle, 2,3-dihydrofur-2-yle, 2,3-dihydrofur-3-yle, 2,4-dihydrofur-2-yle, 2,4-dihydrofur-3-yle, 2,3-dihydrothién-2-yle, 2,3-dihydrothién-3-yle, 2,4-dihydrothién-2-yle, 2,4-dihydrothién-3-yle, 2,3-pyrrolin-2-yle, 2,3-pyrrolin-3-yle, 2,4-pyrrolin-2-yle, 2,4-pyrrolin-3-yle, 2,3-isoxazolin-3-yle, 3,4-isoxazolin-3-yle, 4,5-isoxazolin-3-yle, 2,3-isoxazolin-4-yle, 3,4-isoxazolin-4-yle, 4,5-isoxazolin-4-yle, 2,3-isoxazolin-5-yle, 3,4-isoxazolin-5-yle, 4,5-isoxazolin-5-yle, 2,3-isothiazolin-3-yle, 3,4-isothiazolin-3-yle, 4,5-isothiazolin-3-yle, 2,3-isothiazolin-4-yle, 3,4-isothiazolin-4-yle, 4,5-isothiazolin-4-yle, 2,3-isothiazolin-5-yle, 3,4-isothiazolin-5-yle, 4,5-isothiazolin-5-yle, 2,3-dihydropyrazol-1-yle, 2,3-dihydropyrazol-2-yle, 2,3-dihydropyrazol-3-yle, 2,3-dihydroyrazol-4-yle, 2,3-dihydropyrazol-5-yle, 3,4-dihydropyrazol-1-yle, 3,4-dihydropyrazol-3-yle, 3,4-dihydropyrazol-4-yle, 3,4-dihydropyrazol-5-yle, 4,5-dihydropyrazol-1-yle, 4,5-dihydropyrazol-3-yle, 4,5-dihydropyrazol-4-yle, 4,5-dihydropyrazol-5-yle, 2,3-dihydrooxazol-2-yle, 2,3-dihydrooxazol-3-yle, 2,3-dihydrooxazol-4-yle, 2,3-dihydrooxazol-5-yle, 3,4-dihydrooxazol-2-yle, 3,4-dihydrooxazol-3-yle, 3,4-dihydrooxazol-4-yle, 3,4-dihydrooxazol-5-yle, 2-pipéridinyle, 3-pipéridinyle, 4-pipéridinyle, 1,3-dioxan-5-yle, 2-tétrahydropyranyle, 4-tétrahydropyranyle, 3-tétrahydropyridazinyle, 4-tétrahydroyridazinyle, 2-tétrahydropyrimidinyle, 4-tétrahydropyrimidinyle, 5-tétrahydropyrimidinyle, 2-tétrahydropyrazinyle, 1,3,5-tétrahydroriazin-2-yle, ou 1,2,4-tétrahydrotriazin-3-yle,
ou bien
un groupe phényle ou naphtyle éventuellement substitué, ou bien
un groupe hétéroaromatique à cinq chaînons éventuellement substitué, contenant un à trois atomes d'azote et/ou un atome d'oxygène ou de soufre, ou bien
un groupe hétéroaromatique à six chaînons éventuellement substitué, qui contient un à quatre atomes d'azote,
l'expression "éventuellement substitué" appliquée aux systèmes alicycliques ou hétérocycliques saturés, mono- ou di-insaturés mentionnés en référence à R², indiquant qu'ils peuvent eux-mêmes être partiellement ou totalement halogénés et/ou qu'ils peuvent porter un à trois des substituants suivants : nitro, cyano, alkyle en C1-C6, halogénoalkyle en C1-C4, alcoxy en C1-C4, alkylthio en C1-C4, di-(alkyle en C1-C4)amino, alcényle en C2-C6, alcynyle en C2-C6, (alcoxy en C12-C6)carbonyle, (alkyle en C1-C6)aminocarbonyle, di-(alkyle en C1-C6)aminocarbonyle, (alkyle en C1-C6)carbonyloxy, (alkyle en C1-C6)carbonylamino, cycloalkyle en C3-C7 et phényle,
et l'expression "éventuellement substitué" appliquée aux systèmes aromatiques ou hétéroaromatiques mono- ou bi-cycliques en référence à R² indiquant qu'ils peuvent eux-mêmes être partiellement ou totalement halogénés et/ou qu'ils peuvent porter un à trois des substituants suivants :
- nitro, cyano, thiocyanato, alkyle en C1-C6, halogénoalkyle en C1-C6, alcoxy en C1-C4, halogénoalcoxy en C1-C4, alkylthio en C1-C4, alkylamino en C1-C4, di-(alkyle C1-C4)amine, alcényle en C2-C6, alcényloxy en C3-C6, alcynyle en C2-C6, alcynyloxy en C3-C6, (alkyle en Cl-C6)carbonyle, (alcoxy en Cl-C6)carbonyle, (alkyle en Cl-C6)thiocarbonyle, (alkyle en Cl-C6)aminocarbonyle, di-(alkyle en Cl-C6)aminocarbonyle, (alkyle en C1-C6)carbonyloxy, (alkyle en C1-C6)carbonylamino, cycloalkyle en C3-C7, cycloalcoxy en C3-C7, cycloalkylthio en C3-C7, cycloalkylamino en C3-C7, cycloalcényle en C5-C7,
- phényle, ou bien
un groupe hétéroaromatique à cinq chaînons éventuellement substitué qui contient un à trois atomes d'azote et/ou un atome d'oxygène ou de soufre, ou bien un groupe hétéroaromatique à six chaînons éventuellement substitué, qui contient un à quatre atomes d'azote, ces cycles aryliques ou hétéroaryliques pouvant porter un à trois des substituants suivants : fluoro, chloro, cyano, méthyle, méthoxy, trifluorométhyle ou trifluorométhoxy.

2. Procédé de préparation des composés de formule générale I selon la revendication 1, caractérisé par le fait que l'on fait réagir, en présence d'une base, un dérivé d'acide 2-méthoxyiminophénylacétique de formule IA 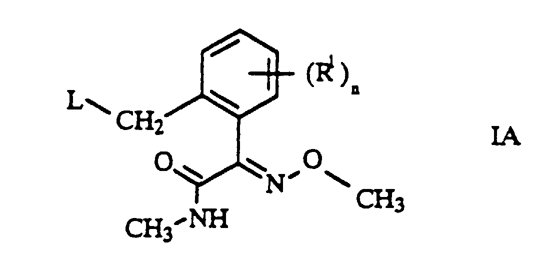 dans laquelle R¹ a les significations indiquées dans la revendication 1, et L représente un groupe éliminable, avec un hydroxy- ou mercaptohétérocycle à cinq chaînons de formule générale III
R² - Y - XH III
dans laquelle R², Y et X ont les significations indiquées dans la revendication 1.

3. Procédé de préparation des composés de formule générale I de la revendication 1, caractérisé par le fait que l'on fait réagir un dérivé d'acide 2-méthoxyiminophénylacétique de formule II 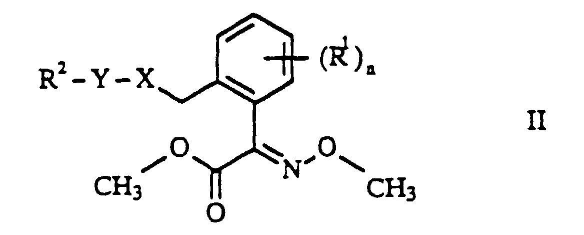 dans laquelle R¹, R², Y, X et n ont les significations indiquées dans la revendication 1, avec la méthylamine.

4. Produit pour lutter contre les mycètes nuisibles, contenant une quantité fongicide d'un composé de formule I selon la revendication 1 et un additif inerte.

5. Produit parasiticide contenant une quantité efficace d'un composé de formule I selon la revendication 1 et un additif inerte.

6. Procédé pour combattre les mycètes nuisibles, caractérisé par le fait que l'on traite les mycètes nuisibles, leur habitat ou les végétaux ou matières à protéger contre les mycètes nuisibles par une quantité fongicide efficace d'un composé de formule I selon la revendication 1.

7. Procédé pour combattre les parasites, caractérisé par le fait que l'on traite les parasites, leur habitat ou les végétaux ou matériaux à protéger contre les parasits, par une quantité efficace d'un composé de formule I selon la revendication 1.

8. Utilisation des composés I selon la revendication 1 pour la lutte contre les mycètes nuisibles.

9. Utilisation des composés I selon la revendication 1 pour la lutte contre les parasites des classes des insectes, des arachnides et des nématodes.
